# EUROPEAN PATENT SPECIFICATION

(11) **EP 4 382 516 B1**
(45) Date of publication and mention of the grant of the patent: **29.10.2025**
(21) Application number: 22867763.9
(22) Date of filing: 13.09.2022
(51) Int. Cl.: C07D 307/91, C07D 407/14, C09D 4/00, C09D 201/00, H10K 99/00, H10K 50/00, C07C 217/76, C09K 11/06, C07C 211/61, C07C 217/94, C07D 407/12

(54) **COMPOUND, COATING COMPOSITION COMPRISING SAME, ORGANIC LIGHT-EMITTING DEVICE USING SAME, AND MANUFACTURING METHOD THEREFOR**
VERBINDUNG, BESCHICHTUNGSZUSAMMENSETZUNG DAMIT, ORGANISCHE LICHTEMITTIERENDE VORRICHTUNG DAMIT UND HERSTELLUNGSVERFAHREN DAFÜR
COMPOSÉ, COMPOSITION DE REVÊTEMENT LE COMPRENANT, DISPOSITIF ÉLECTROLUMINESCENT ORGANIQUE L'UTILISANT ET PROCÉDÉ DE FABRICATION ASSOCIÉ

(30) Priority: 13.09.2021 KR 20210121821; 02.08.2022 KR 20220096200; 02.08.2022 KR 20220096204
(43) Date of publication of application: 12.06.2024
(73) Proprietor: LG Chem, Ltd., Yeongdeungpo-gu Seoul 07336 (KR)
(72) Inventor: SEO, Minseob, Daejeon 34122 (KR); SHIN, Hyeonah, Daejeon 34122 (KR); LEE, Jiyoung, Daejeon 34122 (KR); JUNG, Sejin, Daejeon 34122 (KR); JANG, Songrim, Daejeon 34122 (KR); CHOI, Doowhan, Daejeon 34122 (KR); LEE, Jaechol, Daejeon 34122 (KR); LIM, Dowon, Daejeon 34122 (KR)
(74) Representative: Hoffmann Eitle
(86) International application number: PCT/KR2022/013628
(87) International publication number: WO 2023/038495

(56) References cited:
- WO-A1-2020/241730
- KR-A- 20070 068 419
- KR-A- 20120 052 063
- KR-A- 20180 092 893
- KR-A- 20200 090 177

## Description

### [Technical Field]

This application claims priority to and the benefit of Korean Patent Application Nos. 10-2021-0121821, 10-2022-0096200, and 10-2022-0096204 filed in the Korean Intellectual Property Office on September 13, 2021, August 2, 2022, and August 2, 2022, respectively

The present specification relates to a compound, a coating composition including the compound, an organic light emitting device formed by using the coating composition, and a manufacturing method thereof.

### [Background Art]

An organic light emission phenomenon is one of the examples of converting an electric current into visible rays through an internal process of a specific organic molecule. The principle of the organic light emission phenomenon is as follows. When an organic material layer is disposed between an anode and a cathode and an electric current is applied between the two electrodes, electrons and holes are injected into the organic material layer from the cathode and the anode, respectively. The electrons and the holes which are injected into the organic material layer are recombined to form an exciton, and the exciton falls down again to the ground state to emit light. An organic light emitting device using this principle may be generally composed of a cathode, an anode, and an organic material layer disposed therebetween, for example, an organic material layer including a hole injection layer, a hole transport layer, a light emitting layer, and an electron transport layer. Such organic light emitting devices are known from KR 2018 0092893 A, for instance.

In order to manufacture an organic light emitting device in the related art, a deposition process has been usually used. However, when an organic light emitting device is manufactured by a deposition process, there is a problem in that the loss of materials frequently occurs, so that in order to solve the problem, a technology of manufacturing a device by a solution process capable of increasing the production efficiency due to the low loss of materials has been developed, and there is a need for developing a material that may be used during the solution process.

A material used in an organic light emitting device for a solution process needs to have properties described below.

First, the material used in the organic light emitting device needs to be able to form a storable homogenous solution. Since a commercialized material for a deposition process has good crystallinity so that the material is not dissolved well in a solution or the crystals thereof are easily formed even though the material forms a solution, it is highly likely that according to the storage period, the concentration gradient of the solution varies or a defective device is formed.

Second, a material used for a solution process needs to be excellent in coatability such that during the formation of a thin film, a thin film having a uniform thickness may be formed without the occurrence of holes or an aggregation phenomenon.

Third, layers in which the solution process is performed need to be resistant to the solvents and materials used in a process of forming other layers, and are required to have excellent current efficiency and excellent service life characteristics when an organic light emitting device is manufactured.

Therefore, there is a need for developing a new organic material in the art.

### [Detailed Description of the Invention]

### [Technical Problem]

The present specification provides a compound, a coating composition including the compound, an organic light emitting device formed by using the coating composition, and a manufacturing method thereof.

### [Technical Solution]

An exemplary embodiment of the present specification provides a compound of the following Chemical Formula 1.

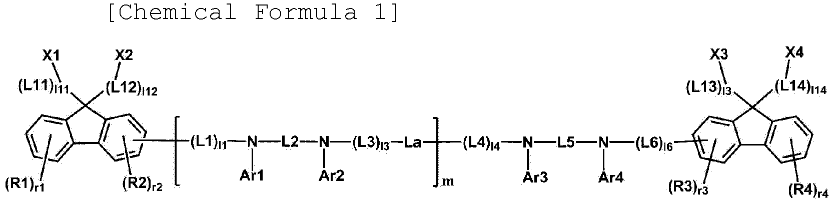

In Chemical Formula 1,
La is -Lx-A-Ly-; a substituted or unsubstituted divalent dihydroanthracene group; a substituted or unsubstituted divalent heterocyclic group; a substituted or unsubstituted divalent fluorenyl group; or a substituted or unsubstituted divalent spirobifluorene group,
Lx and Ly are the same as or different from each other, and are each independently a substituted or unsubstituted arylene group,
A is a substituted or unsubstituted divalent dihydroanthracene group; a substituted or unsubstituted divalent heterocyclic group; a substituted or unsubstituted divalent fluorenyl group; or a substituted or unsubstituted divalent spirobifluorene group,
L1 to L6 are the same as or different from each other, and are each independently a substituted or unsubstituted arylene group; or a substituted or unsubstituted divalent heterocyclic group,
L11 to L14 are the same as or different from each other, and are each independently a direct bond; -O-; a substituted or unsubstituted alkylene group; a substituted or unsubstituted arylene group; or a substituted or unsubstituted divalent heterocyclic group,
Ar1 to Ar4 are the same as or different from each other, and are each independently a substituted or unsubstituted alkyl group; a substituted or unsubstituted aryl group; or a substituted or unsubstituted heterocyclic group,
X1 to X4 are the same as or different from each other, and are each independently hydrogen; deuterium; a halogen group; a substituted or unsubstituted alkyl group; a substituted or unsubstituted alkoxy group; a substituted or unsubstituted aryl group; a substituted or unsubstituted heterocyclic group; a photocurable group or a thermosetting group, and two or more of X1 to X4 are a photocurable group or a thermosetting group,
R1 to R4 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group,
l1, 13, 14 and 16 are each an integer from 0 to 3, and when 11, 13, 14 and 16 are each 2 or higher, structures in the parenthesis are the same as or different from each other,
111 to 114 are each an integer from 1 to 3, and when 111 to 114 are each 2 or higher, structures in the parenthesis are the same as or different from each other,
r1 and r4 are each an integer from 1 to 4, r2 and r3 are each an integer from 1 to 3, and when r1 to r4 are each 2 or higher, substituents in the parenthesis are the same as or different from each other, and
m is an integer from 1 to 10, and when m is 2 or higher, structures in the parenthesis are the same as or different from each other.

Another exemplary embodiment of the present specification provides an organic light emitting device including: a first electrode; a second electrode; and an organic material layer having one or more layers, which includes a light emitting layer provided between the first electrode and the second electrode, in which one or more layers of the organic material layer include the above-described coating composition or a cured product thereof.

Still another exemplary embodiment of the present specification provides a method for manufacturing an organic light emitting device, the method including: preparing a substrate; forming a first electrode on the substrate; forming an organic material layer having one or more layers on the first electrode; and forming a second electrode on the organic material layer, in which the forming of the organic material layer includes forming the organic material layer having one or more layers by using the coating composition.

### [Advantageous Effects]

Since the compound according to an exemplary embodiment of the present specification has a suitable viscosity for solvents, a solution process or an inkjet process is easily performed.

Further, since the compound according to an exemplary embodiment of the present specification has excellent solubility, there is an advantage in that various solvents can be selected when a coating composition is prepared.

In addition, the compound according to an exemplary embodiment of the present specification has an advantage of forming a stable thin film undamaged from subsequent solution processes by forming fully cured thin films from a heat treatment or a light treatment.

Furthermore, since the compound according to an exemplary embodiment of the present specification shows resistance to a specific solvent after curing, a solution process can be performed when a device is manufactured, and accordingly, the device can be made to have a large area.

Further, the compound according to an exemplary embodiment of the present specification can be used as a material for an organic material layer of an organic light emitting device, and can provide low driving voltage, high light emitting efficiency, and/or high service life characteristics.

### [Brief Description of Drawings]

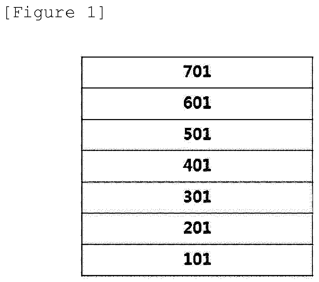

FIG. 1 is a view illustrating the structure of an organic light emitting device according to an exemplary embodiment of the present specification.
101: Substrate
201: Anode
301: Hole injection layer
401: Hole transport layer
501: Light emitting layer
601: Electron transport and injection layer
701: Cathode

### [Best Mode]

Hereinafter, the present specification will be described in detail.

An exemplary embodiment of the present specification provides a compound of the following Chemical Formula 1.

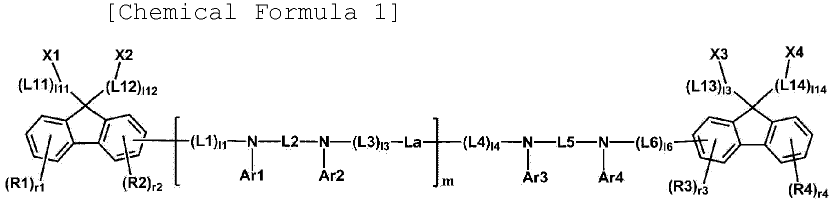

In Chemical Formula 1,
La is -Lx-A-Ly-; a substituted or unsubstituted divalent dihydroanthracene group; a substituted or unsubstituted divalent heterocyclic group; a substituted or unsubstituted divalent fluorenyl group; or a substituted or unsubstituted divalent spirobifluorene group,
Lx and Ly are the same as or different from each other, and are each independently a substituted or unsubstituted arylene group,
A is a substituted or unsubstituted divalent dihydroanthracene group; a substituted or unsubstituted divalent heterocyclic group; a substituted or unsubstituted divalent fluorenyl group; or a substituted or unsubstituted divalent spirobifluorene group,
L1 to L6 are the same as or different from each other, and are each independently a substituted or unsubstituted arylene group; or a substituted or unsubstituted divalent heterocyclic group,
L11 to L14 are the same as or different from each other, and are each independently a direct bond; -O-; a substituted or unsubstituted alkylene group; a substituted or unsubstituted arylene group; or a substituted or unsubstituted divalent heterocyclic group,
Ar1 to Ar4 are the same as or different from each other, and are each independently a substituted or unsubstituted alkyl group; a substituted or unsubstituted aryl group; or a substituted or unsubstituted heterocyclic group,
X1 to X4 are the same as or different from each other, and are each independently hydrogen; deuterium; a halogen group; a substituted or unsubstituted alkyl group; a substituted or unsubstituted alkoxy group; a substituted or unsubstituted aryl group; a substituted or unsubstituted heterocyclic group; a photocurable group or a thermosetting group, and two or more of X1 to X4 are a photocurable group or a thermosetting group,
R1 to R4 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group,
11, 13, 14 and 16 are each an integer from 0 to 3, and when 11, 13, 14 and 16 are each 2 or higher, structures in the parenthesis are the same as or different from each other,
111 to 114 are each an integer from 1 to 3, and when 111 to 114 are each 2 or higher, structures in the parenthesis are the same as or different from each other,
r1 and r4 are each an integer from 1 to 4, r2 and r3 are each an integer from 1 to 3, and when r1 to r4 are each 2 or higher, substituents in the parenthesis are the same as or different from each other, and
m is an integer from 1 to 10, and when m is 2 or higher, structures in the parenthesis are the same as or different from each other.

In general, in the case of a polymer, hole mobility is improved due to a long conjugation length, but there is a problem in that it is difficult to form an organic material layer by a solution process because the polymer is not readily dissolved in a solvent. Conversely, in the case of a monomolecular compound, the compound is readily dissolved in a solvent, and thus is advantageous for a solution process, but since the conjugation length is short, there are few intramolecular hole migrations, and intermolecular hole migration is a main factor, so there is a problem in that the driving voltage is increased and the efficiency is reduced.

A compound according to an exemplary embodiment of the present specification has an effect of exhibiting all the advantages of polymers and single molecules. Specifically, the compound is readily dissolved in a solvent and thus can form an organic material layer through a solution process (or an inkjet process), and exhibits effects of reducing the driving voltage and increasing the efficiency when applied to a device because the compound has excellent hole mobility. In addition, when another layer is stacked on the surface of an organic material layer formed using the compound, still another layer can also be formed through a solution process. These effects are due to the structural characteristics of the compound of Chemical Formula 1.

Regarding the hole mobility, the compound of Chemical Formula 1 includes 4 or more (2m+2) N's, but the number of N's relative to the molecular weight is similar to that of a single molecule. In general, when the number of N's is relatively increased in compounds with the same molecular weight, the HOMO level is decreased (close from the vacuum level), so the difference with the HOMO level of HTL becomes large, which leads to a decrease in hole mobility and an increase in driving voltage.

Conversely, the compound of Chemical Formula 1 exhibits an effect of improving the hole mobility and exhibits good ink properties due to its smaller molecular weight than that of the polymer.

Specifically, when La is a partial bonding position of a divalent heterocyclic group, a divalent fluorenyl group, or a divalent siprobifluorenyl group (particularly, 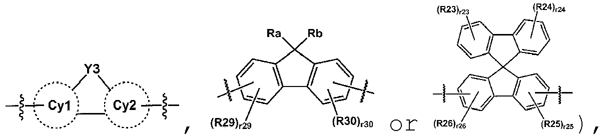 each chain has the same number of N's relative to the molecular weight as that of a single molecule, and this chain is linked through La, and thus, has a longer resonance length than a single molecule. Therefore, compared to the form of a single molecule, the intermolecular hole migration is reduced and the intramolecular hole migration is increased, thereby exhibiting an effect of improving the hole mobility. That is, when La has the above-described structure, the compound of Chemical Formula 1 includes four or more structures in which N and a linker are linked to have an increased resonance length, and thus, exhibits an effect of improving the hole mobility. Therefore, the compound of Chemical Formula 1 exhibits effects of decreasing the driving voltage and increasing the efficiency when applied to a device.

Furthermore, when La is a partial bonding position of -Lx-A-Ly-, a divalent dihydroanthracene group, or a divalent spirobifluorenyl group (particularly, 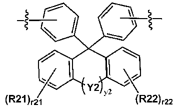 or 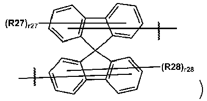 , L1 and L6 are bonded to a specific position (specifically, position 9) of fluorene at both ends to break the resonance between fluorene and chain, thereby maintaining the resonance length and the HOMO level similarly to those of the single molecule. However, since chains are located close to each other in space, the intermolecular hole migration rate is increased compared to the single molecule form, so an effect of improving the hole mobility may be exhibited. That is, when La has the above-described structure, the compound of Chemical Formula 1 includes four or more structures in which N and a linker are linked to decrease the intermolecular distance and constantly maintain the HOMO-LUMO gap, and thus, exhibits an effect of improving the hole mobility.

In the present specification, the chain means a -L1-NAr1-L2-NAr2-L3- unit and a -L4-NAr3-L5-NAr4-L6- unit of Chemical Formula 1.

In the present specification, the linker means one or more of L1 to L6 and La of Chemical Formula 1.

In an exemplary embodiment of the present specification, the N may be N included in an amine group.

In an exemplary embodiment of the present specification, the compound of Chemical Formula 1 has a molecular weight of 1,000 g/mol or more and less than 10,000 g/mol. Specifically, the compound of Chemical Formula 1 has a molecular weight of 1,000 g/mol to 5,000 g/mol. Accordingly, an effect in which the viscosity is similar to that of a single molecule is exhibited.

Regarding the solution process, a compound according to an exemplary embodiment of the present specification has a viscosity similar to that of a single molecule by satisfying the above-described molecular weight range, and thus is readily dissolved in a solvent, so that a solution process (or an inkjet process) can be performed. Further, since the compound of Chemical Formula 1 includes two or more curing groups to form crosslinks by heat treatment or light treatment, it is possible to provide an organic material layer including a thin-filmed structure. The organic material layer thus prepared has an advantage in that a stable thin film, which is not damaged from the subsequent solution process, is formed. That is, the compound can be subjected to a solution process (or an inkjet process) similarly to a single molecule, and has an advantage of forming a thin film which is not damaged similarly to a polymer.

In addition, the organic material layer formed as described above can be subjected to a solution process when another layer is stacked on the surface thereof, and may be prevented from being dissolved, morphologically affected, or decomposed by a solvent. Accordingly, since it is advantageous to maintain a film after the formation of the film during the manufacture of a device, it is possible to provide an organic light emitting device having long service life characteristics.

Furthermore, in the case of a compound according to an exemplary embodiment of the present specification, an organic light emitting device may be manufactured by a solution application method, thereby enabling a large area of the device to be implemented.

In the present specification, the "single molecule" means a material composed of one structure having no repeating unit.

In an exemplary embodiment of the present specification, the single molecule has a molecular weight of less than 10,000 g/mol. Specifically, the molecular weight is 100 g/mol to 5,000g/mol.

In the present specification, the "polymer" means a polymer in which the same structure is repeated. That is, the "polymer" means a polymer in which a repeating unit is present.

In an exemplary embodiment of the present specification, the polymer has a number average molecular weight of 10,000 g/mol to 5,000,000 g/mol. Specifically, the number average molecular weight is 10,000g/mol to 1,000,000 g/mol.

When one member (layer) is disposed "on" another member (layer) in the present specification, this includes not only a case where the one member (layer) is brought into contact with another member, but also a case where still another member (layer) is present between the two members (layers).

When one part "includes" one constituent element in the present specification, unless otherwise specifically described, this does not mean that another constituent element is excluded, but means that another constituent element may be further included.

In the present specification, the "photocurable group or thermosetting group" may mean a reactive substituent which cross-links compounds by exposure to heat and/or light. The photocurable group or thermosetting group may be produced while radicals produced by decomposing carbon-carbon multiple bonds or cyclic structures by means of light irradiation or heat treatment are linked to each other.

In the present specification, the "curing group" is a "photocurable group or thermosetting group".

Unless otherwise defined in the present specification, all technical and scientific terms used in the present specification have the same meaning as commonly understood by one with ordinary skill in the art to which the present invention pertains. Although methods and materials similar to or equivalent to those described in the present invention may be used in the practice or in the test of exemplary embodiments of the present invention, suitable methods and materials will be described below.

In the present specification, "------" and 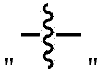 mean a moiety bonded to another substituent or a bonding portion.

In the present specification, the term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more substituents are substituted, the two or more substituents may be the same as or different from each other.

In the present specification, the term "substituted or unsubstituted" means being substituted with one or two or more substituents selected from the group consisting of deuterium; a halogen group; an alkyl group; a cycloalkyl group; an alkoxy group; an aryloxy group; an amine group; an aryl group; a heterocyclic group; and a cross-linkable group, being substituted with a substituent to which two or more substituents among the exemplified substituents are linked, or having no substituent. For example, "the substituent to which two or more substituents are linked" may be a biphenyl group. That is, the biphenyl group may also be an aryl group, and may be interpreted as a substituent to which two phenyl groups are linked.

Examples of the substituents will be described below, but are not limited thereto.

In the present specification, examples of a halogen group include fluorine (F), chlorine (Cl), bromine (Br) or iodine (I).

In the present specification, an alkyl group may be straight-chained or branched, and the number of carbon atoms thereof is not particularly limited, but is preferably 1 to 60. According to an exemplary embodiment, the number of carbon atoms of the alkyl group is 1 to 30. Specific examples of the alkyl group include a methyl group, an ethyl group, a propyl group, an isopropyl group, a butyl group, an isobutyl group, a tert-butyl group, a pentyl group, a hexyl group, a heptyl group, an octyl group, and the like, but are not limited thereto.

In the present specification, an alkylene group means a group having two bonding positions in an alkyl group, that is, a divalent group. The above-described description on the alkyl group may be applied to the alkylene group, except for a divalent alkylene group.

In the present specification, the number of carbon atoms of the cycloalkyl group is not particularly limited, but is preferably 3 to 60. According to an exemplary embodiment, the number of carbon atoms of the cycloalkyl group is 3 to 30. Specific examples of the cycloalkyl group include a cyclopropyl group, a cyclobutyl group, a cyclopentyl group, a cyclohexyl group, a cycloheptyl group, a cyclooctyl group, and the like, but are not limited thereto.

In the present specification, an alkoxy group may be straight-chained, branched, or cyclic. The number of carbon atoms of the alkoxy group is not particularly limited, but is preferably 1 to 30. Specific examples of the alkoxy group may be a methoxy group, an ethoxy group, an n-propoxy group, an isopropoxy group, an n-butoxy group, an isobutoxy group, a tert-butoxy group, a sec-butoxy group, an n-pentyloxy group, a neopentyloxy group, an isopentyloxy group, an n-hexyloxy group, a 3,3-dimethylbutyloxy group, a 2-ethylbutyloxy group, an n-octyloxy group, an n-nonyloxy group, an n-decyloxy group and the like, but are not limited thereto.

In the present specification, an amine group may be selected from the group consisting of -NH₂; an alkylamine group; an arylalkylamine group; an arylamine group; an arylheteroarylamine group; an alkylheteroarylamine group; and a heteroarylamine group, and is not limited thereto. The number of carbon atoms of the amine group is not particularly limited, but is preferably 1 to 60.

In the present specification, the number of carbon atoms of the aryl group is not particularly limited, but is preferably 6 to 60. According to an exemplary embodiment, the number of carbon atoms of the aryl group is 6 to 30. In an exemplary embodiment of the present specification, the aryl group may be a monocyclic aryl group or a polycyclic aryl group. Specific examples of the monocyclic aryl group include a phenyl group, a biphenyl group, a terphenyl group, and the like, but are not limited thereto. Examples of the polycyclic aryl group include a naphthyl group, an anthracenyl group, a phenanthrenyl group, a pyrenyl group, a perylenyl group, a triphenylenyl group, a chrysenyl group, a fluorenyl group, and the like, but are not limited thereto.

In the present specification, an arylene group means a group having two bonding positions in an aryl group, that is, a divalent group. The above-described description on the aryl group may be applied to the arylene group, except that the arylene groups are each a divalent group.

In the present specification, a fluorenyl group may be substituted, and two substituents may be bonded to each other to form a spiro structure.

When the fluorenyl group is substituted, the substituent may be 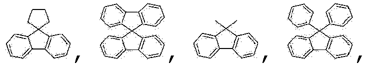 and the like, and the exemplified structure may be substituted with an additional substituent. However, the structure is not limited thereto.

In the present specification, a divalent fluorenyl group means a group having two bonding positions in a fluorenyl group, that is, a divalent group. In this case, the bonding position may be located on the benzene ring of fluorenyl or may be located on the substituent substituted with fluorenyl. For example, the divalent fluorenyl group may be any one of the following structures, but is not limited thereto.

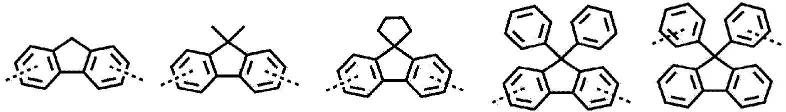

In the present specification, a divalent spirobifluorenyl group means a group having two bonding positions in a spirobifluorenyl group, that is, a divalent group. For example, the divalent spirobifluorenyl group may be any one of the following structures, but is not limited thereto.

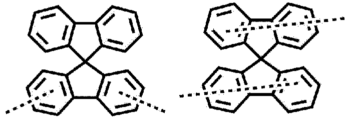

In the present specification, examples of an arylamine group include a substituted or unsubstituted monoarylamine group, a substituted or unsubstituted diarylamine group, or a substituted or unsubstituted triarylamine group. The aryl group in the arylamine group may be a monocyclic aryl group or a polycyclic aryl group. The arylamine group including two or more aryl groups may include a monocyclic aryl group, a polycyclic aryl group, or both a monocyclic aryl group and a polycyclic aryl group. For example, the aryl group in the arylamine group may be selected from the above-described examples of the aryl group. The number of carbon atoms of the arylamine group is not particularly limited, but is preferably 6 to 60.

In the present specification, a heterocyclic group is an aromatic, aliphatic or fused ring group of the aromatic group and the aliphatic group, which includes one or more atoms other than carbon, that is, one or more heteroatoms. Specifically, the heteroatom may include one or more atoms selected from the group consisting of N, O, P, S, Si, Se, and the like. The number of carbon atoms of the heterocyclic group is not particularly limited, but may be 2 to 60. Examples of the heterocyclic group include a thiophene group, a furan group, a pyrrole group, an imidazole group, a thiazole group, an oxazole group, an oxadiazole group, a pyridine group, a bipyridine group, a pyrimidine group, a triazine group, a triazole group, an acridine group, a pyridazine group, a pyrazine group, a quinoline group, a quinazoline group, a quinoxaline group, a phthalazine group, a pyridopyrimidine group, a pyridopyrazine group, a pyrazinopyrazine group, an isoquinoline group, an indole group, a carbazole group, a benzoxazole group, a benzimidazole group, a benzothiazole group, a benzocarbazole group, a benzothiophene group, a dibenzothiophene group, a benzofuran group, a phenanthridine group, a phenanthroline group, an isoxazole group, a thiadiazole group, a phenothiazine group, xanthene group, thioxanthene group, a dibenzofuran group, and the like, but are not limited thereto.

In the present specification, the above-described description on the heterocyclic group may be applied to a heteroaryl group except for an aromatic heteroaryl group.

In the present specification, a divalent heterocyclic group means a group having two bonding positions in a heterocyclic group. The above-described description on the heterocyclic group may be applied to the divalent heterocyclic group, except that the divalent heterocyclic groups are each a divalent group. For example, the divalent heterocyclic group may be the following structure, but is not limited thereto.

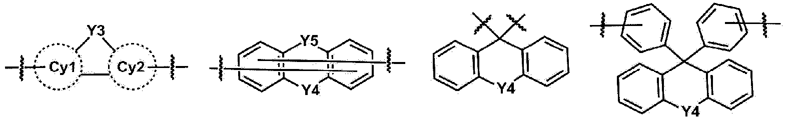

In the structures, Cy1 and Cy2 are the same as or different from each other, and are each independently a substituted or unsubstituted aromatic ring, Y3 and Y4 are the same as or different from each other, and are each independently O; S; or SiRcRd, Y5 is O; S; CRaRb; or SiRcRd, and Ra to Rd are the same as or different from each other, and are each independently hydrogen; deuterium; a substituted or unsubstituted alkyl group; or a substituted or unsubstituted aryl group.

In the present specification, a divalent dihydroanthracene group means a group having two bonding positions in a dihydroanthracene group. For example, the divalent dihydroanthracene group may be the following structure, but is not limited thereto.

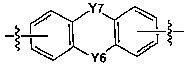

In the structure, Y6 and Y7 are the same as or different from each other, and are each independently CRaRb, Ra and Rb are the same as or different from each other, and are each independently hydrogen; deuterium; a substituted or unsubstituted alkyl group; or a substituted or unsubstituted aryl group.

In the present specification, the "adjacent" group may mean a substituent substituted with an atom directly linked to an atom in which the corresponding substituent is substituted, a substituent disposed to be sterically closest to the corresponding substituent, or another substituent substituted with an atom in which the corresponding substituent is substituted. For example, two substituents substituted at the ortho position in a benzene ring and two substituents substituted with the same carbon in an aliphatic ring may be interpreted as groups which are "adjacent" to each other.

In the present specification, in a ring formed by bonding adjacent groups, the "ring" means a substituted or unsubstituted hydrocarbon ring; or a substituted or unsubstituted hetero ring.

In the present specification, a hydrocarbon ring group may be an aromatic ring, an aliphatic ring, or a ring in which an aromatic ring and an aliphatic ring are fused.

In the present invention, the above-described description on the aryl group may be applied to an aromatic ring.

In the present specification, the above-described description on the cycloalkyl group may be applied to an aliphatic ring.

In an exemplary embodiment of the present specification, Chemical Formula 1 is the following Chemical Formula 1-1.

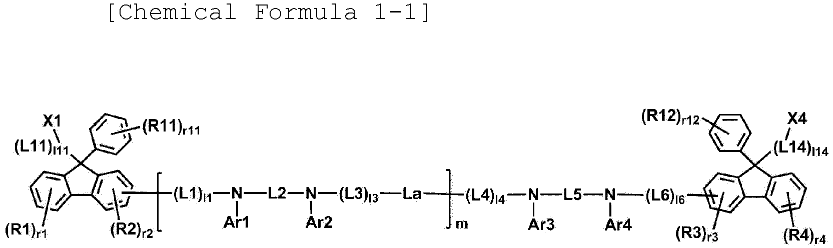

In Chemical Formula 1-1,
La, L1 to L6, L11, L14, Ar1 to Ar4, R1 to R4, 11, 13, 14, 16, 111, 114, r1 to r4 and m are the same as those defined in Chemical Formula 1,
X1 and X4 are the same as or different from each other, and are each independently a photocurable group or a thermosetting group,
R11 and R12 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group, and
r11 and r12 are each an integer from 1 to 5, and when r11 and r12 are each 2 or higher, substituents in the parenthesis are the same as or different from each other.

In an exemplary embodiment of the present specification, Chemical Formula 1 is the following Chemical Formula 1-1-1 or 1-1-2.

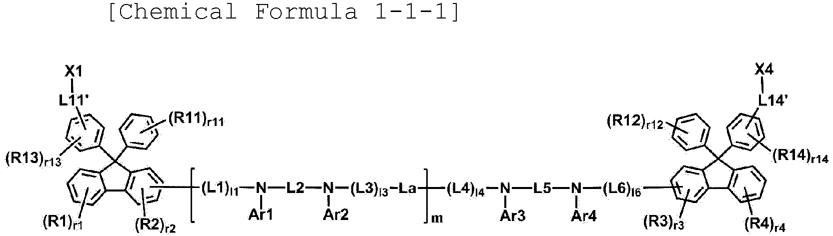

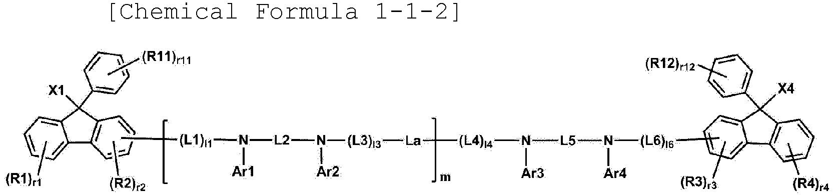

In Chemical Formulae 1-1-1 and 1-1-2,
La, L1 to L6, Ar1 to Ar4, R1 to R4, 11, 13, 14, 16, r1 to r4 and m are the same as those defined in Chemical Formula 1,
X1 and X4 are the same as or different from each other, and are each independently a photocurable group or a thermosetting group,
L11' and L14' are the same as or different from each other, and are each independently a direct bond; or -O-,
R11 to R14 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group, and
r13 and r14 are each an integer from 1 to 4, r11 and r12 are each an integer from 1 to 5, and when r11 to r14 are each 2 or higher, substituents in the parenthesis are the same as or different from each other.

In an exemplary embodiment of the present specification, Chemical 1 is the following Chemical Formula 1-A.

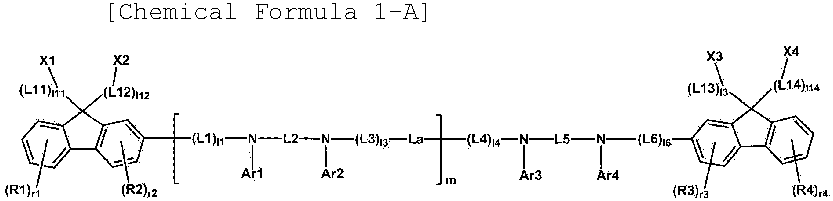

In Chemical Formula 1-A,
La, L1 to L6, L11 to L14, Ar1 to Ar4, X1 to X4, R1 to R4, 11, 13, 14, 16, 111 to 114, r1 to r4 and m are the same as those defined in Chemical Formula 1.

In an exemplary embodiment of the present specification, Chemical Formula 1 is the following Chemical Formula 1-A-1.

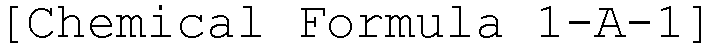

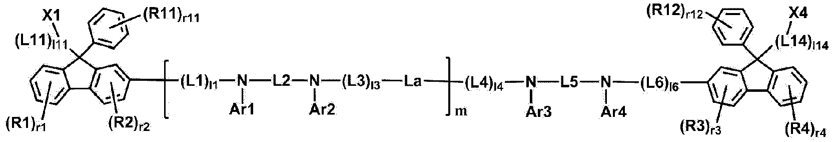

In Chemical Formula 1-A-1,
La, L1 to L6, L11, L14, Ar1 to Ar4, R1 to R4, 11, 13, 14, 16, 111, 114, r1 to r4 and m are the same as those defined in Chemical Formula 1,
X1 and X4 are the same as or different from each other, and are each independently a photocurable group or a thermosetting group,
R11 and R12 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group, and
r11 and r12 are each an integer from 1 to 5, and when r11 and r12 are each 2 or higher, substituents in the parenthesis are the same as or different from each other.

In an exemplary embodiment of the present specification, Chemical Formula 1 is the following Chemical Formula 1-A-11 or 1-A-12.

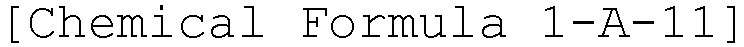

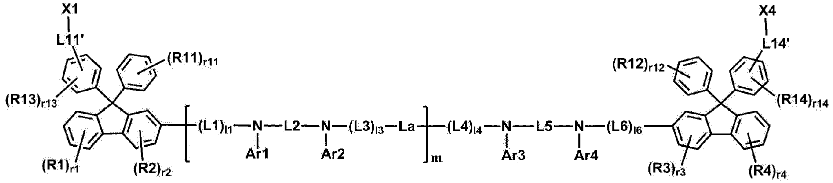

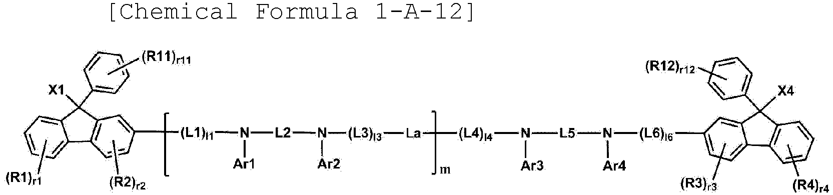

In Chemical Formulae 1-A-11 and 1-A-12,
La, L1 to L6, Ar1 to Ar4, R1 to R4, 11, 13, 14, 16, r1 to r4 and m are the same as those defined in Chemical Formula 1,
X1 and X4 are the same as or different from each other, and are each independently a photocurable group or a thermosetting group,
L11' and L14' are the same as or different from each other, and are each independently a direct bond; or -O-,
R11 to R14 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group, and
r13 and r14 are each an integer from 1 to 4, r11 and r12 are each an integer from 1 to 5, and when r11 to r14 are each 2 or higher, substituents in the parenthesis are the same as or different from each other.

In an exemplary embodiment of the present specification, La is -Lx-A-Ly-; a substituted or unsubstituted divalent dihydroanthracene group; a substituted or unsubstituted tricyclic to decacyclic divalent heterocyclic group including O, S, or Si; a substituted or unsubstituted divalent fluorenyl group; or a substituted or unsubstituted divalent spirobifluorenyl group.

In an exemplary embodiment of the present specification, La is -Lx-A-Ly-; a substituted or unsubstituted divalent dihydroanthracene group; a substituted or unsubstituted tricyclic to octacyclic divalent heterocyclic group including O, S, or Si; or a substituted or unsubstituted divalent fluorenyl group; or a substituted or unsubstituted divalent spirobifluorenyl group.

In an exemplary embodiment of the present specification, La is -Lx-A-Ly-; a substituted or unsubstituted divalent dihydroanthracene group; a substituted or unsubstituted tricyclic to hexacyclic divalent heterocyclic group including O, S, or Si; or a substituted or unsubstituted divalent fluorenyl group; or a substituted or unsubstituted divalent spirobifluorenyl group.

In an exemplary embodiment of the present specification, La is any one of the following structures.

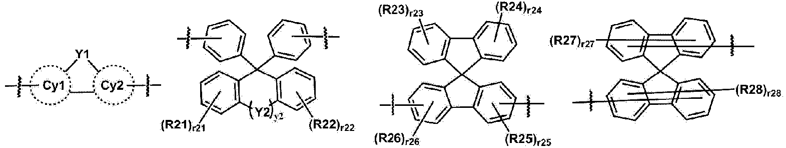

In the structures,
Cy1 and Cy2 are the same as or different from each other, and are each independently a substituted or unsubstituted aromatic ring,
Y1 and Y2 are the same as or different from each other, and are each independently O; S; CRaRb; or SiRcRd,
Ra to Rd are the same as or different from each other, and are each independently hydrogen; deuterium; a substituted or unsubstituted alkyl group; or a substituted or unsubstituted aryl group,
R21 to R28 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group, or are bonded to an adjacent group to form a substituted or unsubstituted ring,
y2 is 0 or 1,
r21 to r24 are each an integer from 1 to 4, r25 and r26 are each an integer from 1 to 3, r27 and r28 are each an integer from 1 to 7, and when r21 to r28 are 2 or higher, substituents in the parenthesis are the same as or different from each other, and 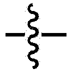
is a moiety bonded to Chemical Formula 1.

In an exemplary embodiment of the present specification, La is not substituted with a curable group.

In an exemplary embodiment of the present specification, La is a substituted or unsubstituted divalent fluorenyl group; or a substituted or unsubstituted divalent spirobifluorenyl group.

In an exemplary embodiment of the present specification, La is any one of the following structures.

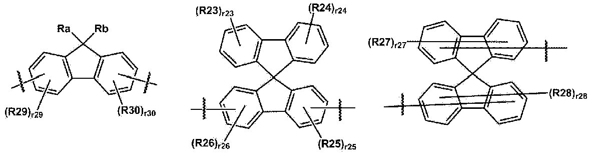

In the structures,
Ra and Rb are the same as or different from each other, and are each independently hydrogen; deuterium; a substituted or unsubstituted alkyl group; or a substituted or unsubstituted aryl group,
R23 to R30 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group,
r25, r26, r29 and r30 are each an integer from 1 to 3, r23 and r24 are each an integer from 1 to 4, r27 and r28 are each an integer from 1 to 7, and when r23 to r30 are 2 or higher, substituents in the parenthesis are the same as or different from each other, and 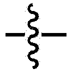
is a moiety bonded to Chemical Formula 1.

In an exemplary embodiment of the present specification, La is -Lx-A-Ly; a substituted or unsubstituted divalent dihydroanthracene group; or a substituted or unsubstituted divalent heterocyclic group.

In an exemplary embodiment of the present specification, La is the following structure.

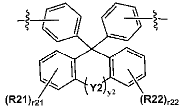

In the structure,
Y2 is O; S; CRaRb; or SiRcRd,
Ra to Rd are the same as or different from each other, and are each independently hydrogen; deuterium; a substituted or unsubstituted alkyl group; or a substituted or unsubstituted aryl group,
R21 and R22 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group, or are bonded to an adjacent group to form a substituted or unsubstituted ring,
y2 is 0 or 1,
r21 and r22 are each an integer from 1 to 4, and when r21 and r22 are each 2 or higher, substituents in the parenthesis are the same as or different from each other, and 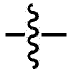
is a moiety bonded to Chemical Formula 1.

In an exemplary embodiment of the present specification, y2 is 0. In this case, the structure may be represented by

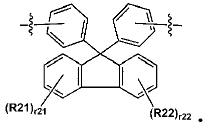

In an exemplary embodiment of the present specification, y2 is 1. In this case, the structure may be represented by

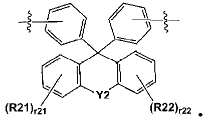

In an exemplary embodiment of the present specification, La is a substituted or unsubstituted divalent heterocyclic group.

In an exemplary embodiment of the present specification, La is the following structure.

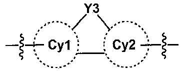

In the structure,
Cy1 and Cy2 are the same as or different from each other, and are each independently a substituted or unsubstituted aromatic ring,
Y3 is O; S; or SiRcRd,
Rc and Rd are the same as or different from each other, and are each independently hydrogen; deuterium; a substituted or unsubstituted alkyl group; or a substituted or unsubstituted aryl group, and 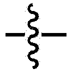
is a moiety bonded to Chemical Formula 1.

In an exemplary embodiment of the present specification, Cy1 and Cy2 are the same as or different from each other, and are each independently a substituted or unsubstituted aromatic ring having 6 to 30 carbon atoms.

In an exemplary embodiment of the present specification, Cy1 and Cy2 are the same as or different from each other, and are each independently a substituted or unsubstituted benzene ring; or a substituted or unsubstituted naphthalene ring.

In an exemplary embodiment of the present specification, Cy1 and Cy2 are the same as or different from each other, and are each independently a benzene ring; or a naphthalene ring.

In an exemplary embodiment of the present specification, La is any one of the following structures.

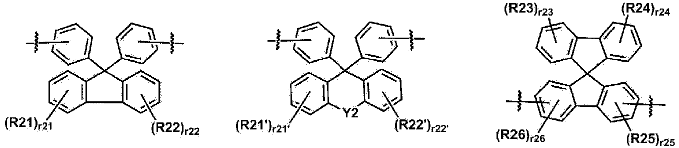

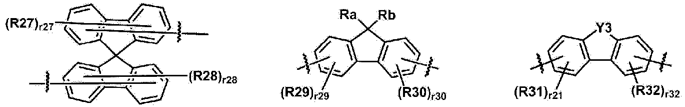

In the structures,
Y2 is O; S; CRaRb; or SiRcRd,
Y3 is O; S; or SiRcRd,
Ra to Rd are the same as or different from each other, and are each independently hydrogen; deuterium; a substituted or unsubstituted alkyl group; or a substituted or unsubstituted aryl group,
R21 to R32, R21' and R22' are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group, or adjacent groups are bonded to each other to form a substituted or unsubstituted ring,
r21 to r24, r21' and r22' are each an integer from 1 to 4, r25, r26 and r29 to r32 are each an integer from 1 to 3, r27 and r28 are each an integer from 1 to 7, and when r21 to r32, r21' and r22' are 2 or higher, substituents in the parenthesis are the same as or different from each other, and 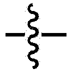
is a moiety bonded to Chemical Formula 1.

In an exemplary embodiment of the present specification, La is -Lx-A-Ly-, LX and Ly are the same as or different from each other, and are each independently a substituted or unsubstituted arylene group, and A is a substituted or unsubstituted divalent dihydroanthraene group; a substituted or unsubstituted divalent heterocyclic group; a substituted or unsubstituted divalent fluorenyl group; or a substituted or unsubstituted divalent spirobifluorene group.

In an exemplary embodiment of the present specification, Lx and Ly are the same as or different from each other, and are each independently a substituted or unsubstituted arylene group having 6 to 30 carbon atoms.

In an exemplary embodiment of the present specification, Lx and Ly are the same as or different from each other, and are each independently an arylene group having 6 to 30 carbon atoms.

In an exemplary embodiment of the present specification, Lx and Ly are each a substituted or unsubstituted phenylene group.

In an exemplary embodiment of the present specification, Lx and Ly are each a phenylene group.

In an exemplary embodiment of the present specification, A is a substituted or unsubstituted divalent heterocyclic group; a substituted or unsubstituted divalent fluorenyl group; or a substituted or unsubstituted divalent dihydroanthracene group.

In an exemplary embodiment of the present specification, -Lx-A-Ly- is 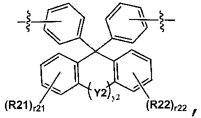 and Y2, y2, R21, R22, r21 and r22 are the same as those described above.

In the structures, when y2 is 0, A may be represented by a divalent fluorenyl group.

In the structures, when y2 is 1 and Y2 is CRaRb, A may be represented by a divalent dihydroanthracene group.

In the structures, when y2 is 1 and Y2 is O; S; or SiRcRd, A may be represented by a divalent heterocyclic group.

Specifically, -Lx-A-Ly- is any one of the following structures.

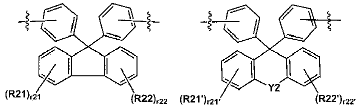

In an exemplary embodiment of the present specification, La is a substituted or unsubstituted divalent fluorenyl group. Specifically, La is

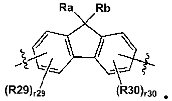

In an exemplary embodiment of the present specification, La is a substituted or unsubstituted divalent heterocyclic group. Specifically, La is

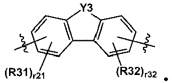

In an exemplary embodiment of the present specification, La is a substituted or unsubstituted divalent spirobifluorene group. Specifically, La is

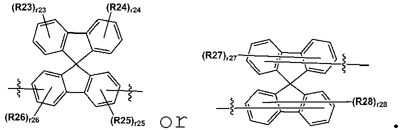

In the structures, Y2, Y3, Ra, Rb, R21 to R32, R21', R22', r21 to r32, r21' and r22' are the same as those described above.

In an exemplary embodiment of the present specification, Chemical Formula 1 is any one of the following Chemical Formulae 1-11 to 1-16.

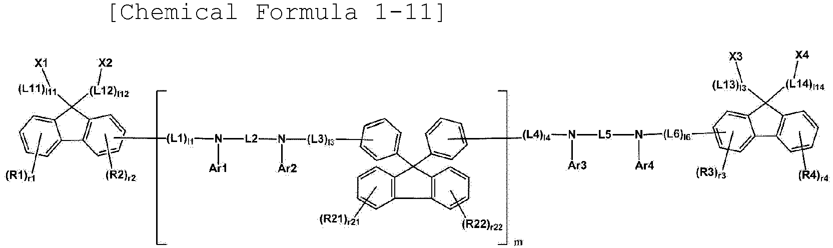

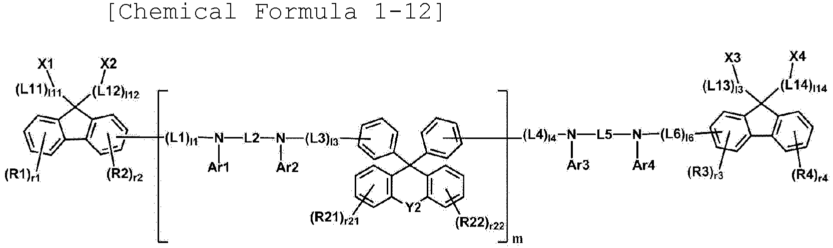

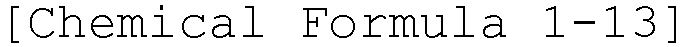

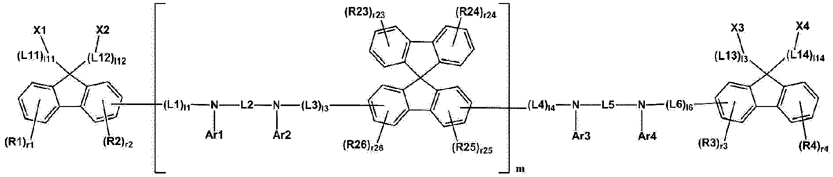

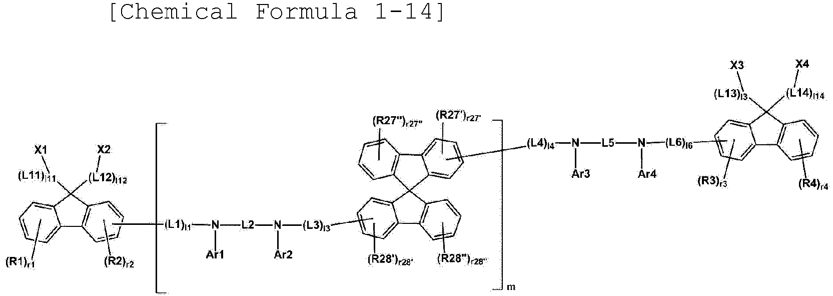

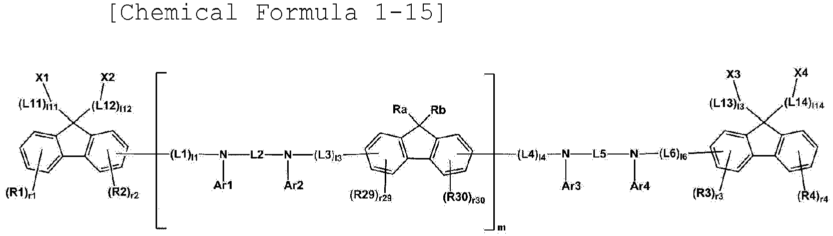

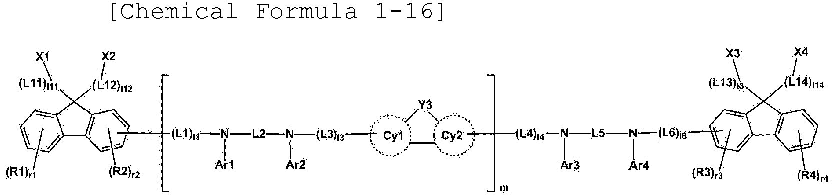

In Chemical Formulae 1-11 to 1-16,
L1 to L6, L11 to L14, Ar1 to Ar4, X1 to X4, R1 to R4, 11, 13, 14, 16, 111 to 114, r1 to r4 and m are the same as those defined in Chemical Formula 1,
Cy1 and Cy2 are the same as or different from each other, and are each independently a substituted or unsubstituted aromatic ring,
Y2 is O, S, CRaRb or SiRcRd,
Y3 is O, S or SiRcRd,
Ra to Rd are the same as or different from each other, and are each independently hydrogen; deuterium; a substituted or unsubstituted alkyl group; or a substituted or unsubstituted aryl group,
R21 to R26, R27', R27'', R28', R28'', R29 and R30 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group, or adjacent groups are bonded to each other to form a substituted or unsubstituted ring, and
r25, r26, r27', r28', r29 and r30 are each an integer from 1 to 3, r21 to r24, r27'' and r28'' are each an integer from 1 to 4, and when r21 to r26, r27', r27'', r28', r28'', r29 and r30 are each 2 or higher, substituents in the parenthesis are the same as or different from each other.

In an exemplary embodiment of the present specification, Chemical Formula 1 is any one of the following Chemical Formulae 1-21 to 1-26.

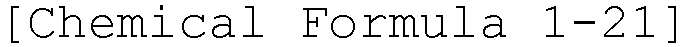

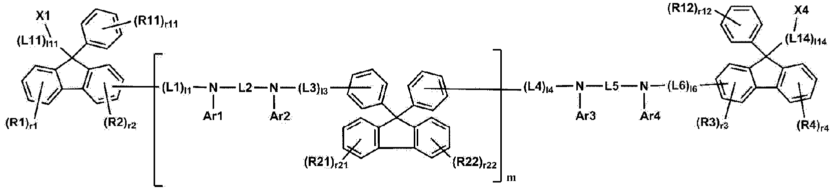

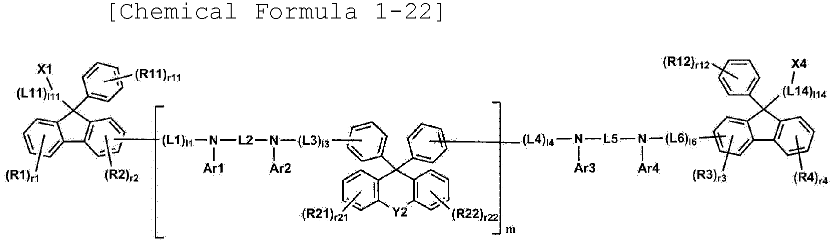

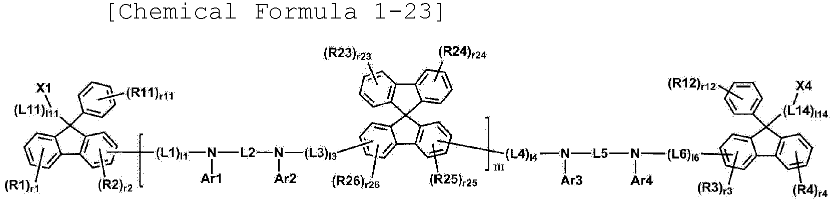

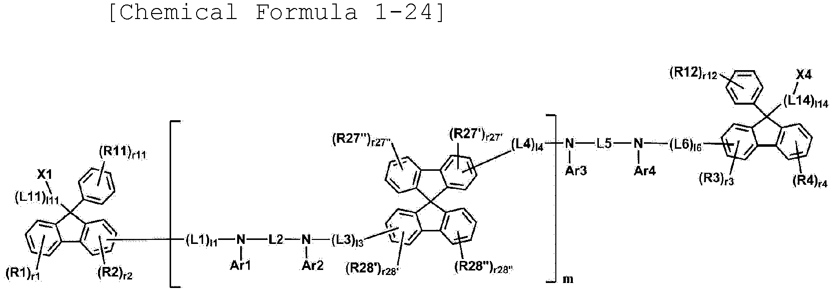

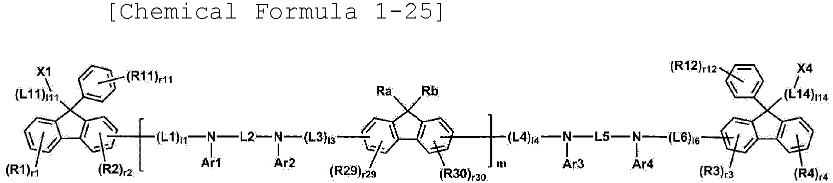

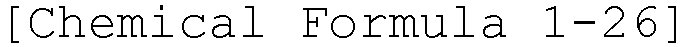

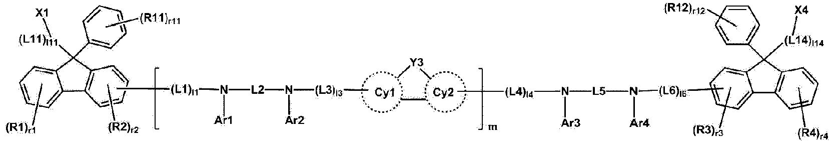

In Chemical Formulae 1-21 to 1-26,
L1 to L6, L11, L14, Ar1 to Ar4, R1 to R4, 11, 13, 14, l6, l11, l14, r1 to r4 and m are the same as those defined in Chemical Formula 1,
Cy1 and Cy2 are the same as or different from each other, and are each independently a substituted or unsubstituted aromatic ring,
Y2 is O, S, CRaRb or SiRcRd,
Y3 is O, S or SiRcRd,
X1 and X4 are the same as or different from each other, and are each independently a photocurable group or a thermosetting group,
Ra to Rd are the same as or different from each other, and are each independently hydrogen; deuterium; a substituted or unsubstituted alkyl group; or a substituted or unsubstituted aryl group,
R11 and R12 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group,
R21 to R26, R27', R27", R28', R28", R29 and R30 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group, or adjacent groups are bonded to each other to form a substituted or unsubstituted ring, and
r25, r26, r27', r28', r29 and r30 are each an integer from 1 to 3, r21 to r24, r27'' and r28'' are each an integer from 1 to 4, r11 and r12 are each an integer from 1 to 5, and when r11, r12, r21 to r26, r27', r27", r28', r28'', r29 and r30 are each 2 or higher, substituents in the parenthesis are the same as or different from each other.

In an exemplary embodiment of the present specification, Chemical Formula 1-21 is any one of the following Chemical Formulae 1-21-1 to 1-21-5, and the following structures are unsubstituted or substituted with an additional substituent.

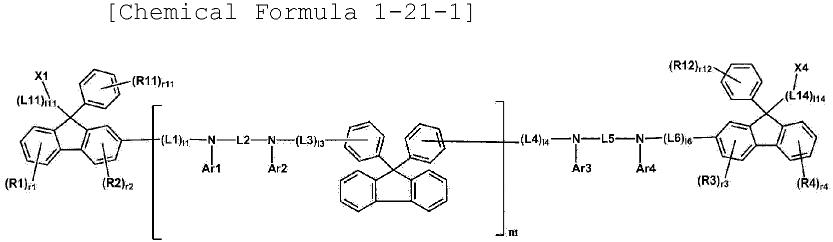

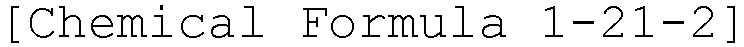

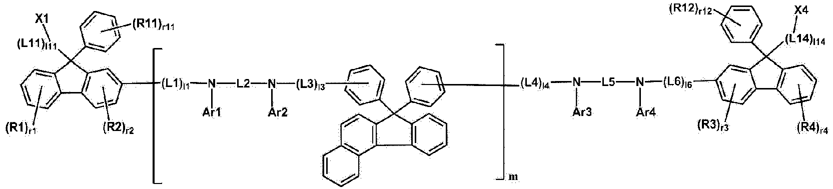

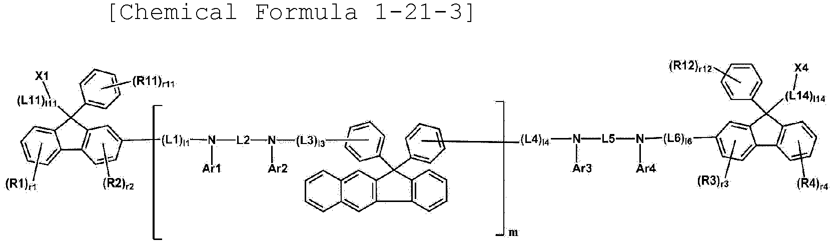

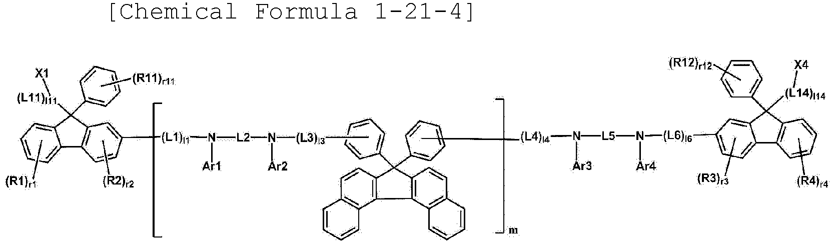

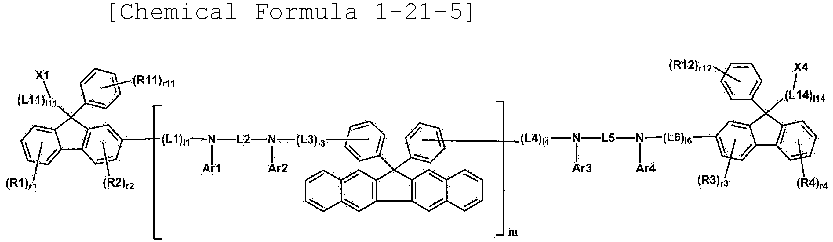

In Chemical Formulae 1-21-1 to 1-21-5, L1 to L6, L11, L14, X1, X4, Ar1 to Ar4, R1 to R4, R11, R12, 11, 13, 14, 16, 111, 114, r1 to r4, r11, r12 and m are the same as those defined in Chemical Formula 1-21.

In an exemplary embodiment of the present specification, when any one or more adjacent groups in R21 and R22 of Chemical Formula 1-21 are bonded to each other to form a ring, Chemical Formula 1 is represented by any one of Chemical Formulae 1-21-2 to 1-21-5.

In an exemplary embodiment of the present specification, Chemical Formula 1-22 is any one of the following Chemical Formulae 1-22-1 to 1-22-5, and the following structures are unsubstituted or substituted with an additional substituent.

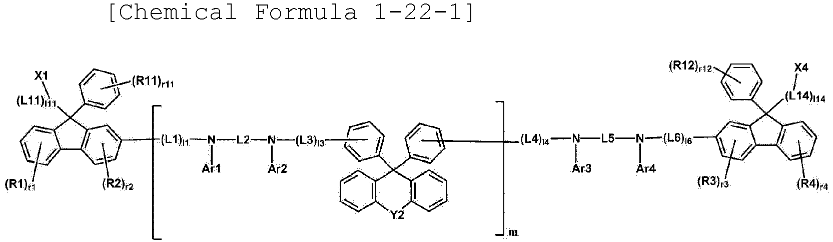

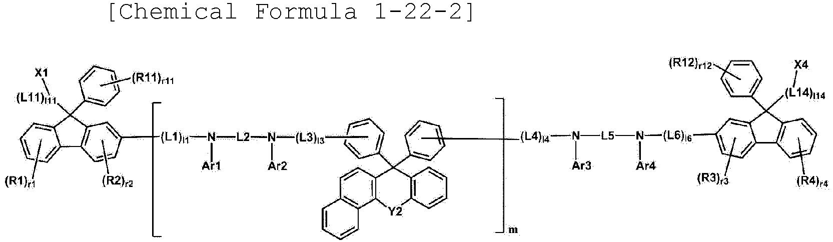

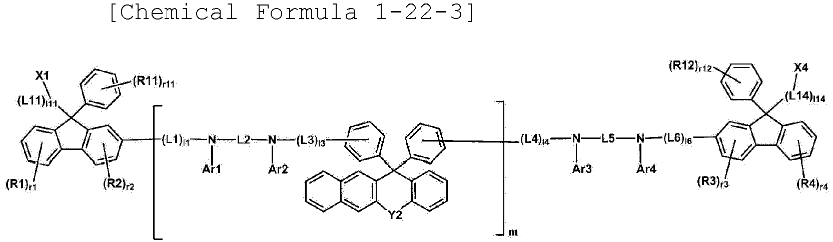

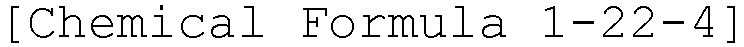

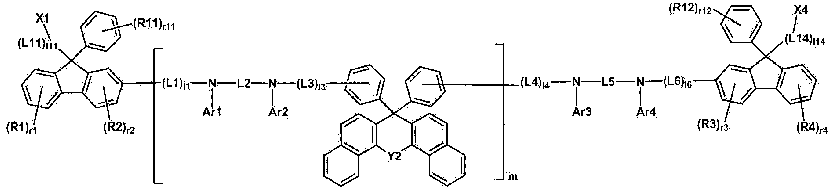

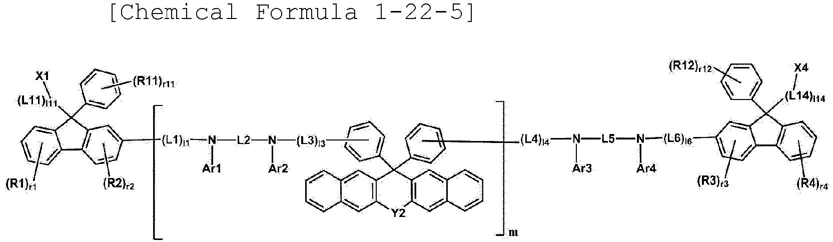

In Chemical Formulae 1-22-1 to 1-22-5, Y2, L1 to L6, L11, L14, X1, X4, Ar1 to Ar4, R1 to R4, R11, R12, l1, l3, 14, 16, 111, 114, r1 to r4, r11, r12 and m are the same as those defined in Chemical Formula 1-22.

In an exemplary embodiment of the present specification, when any one or more adjacent groups in R21 and R22 of Chemical Formula 1-22 are bonded to each other to form a ring, Chemical Formula 1 is represented by any one of Chemical Formulae 1-22-2 to 1-22-5.

In an exemplary embodiment of the present specification, the additional substituent substituted in Chemical Formulae 1-21-1 to 1-21-5 and 1-22-1 to 1-22-5 is one or more selected from the group consisting of deuterium; a halogen group; a substituted or unsubstituted alkyl group; a substituted or unsubstituted alkoxy group; a substituted or unsubstituted aryl group; and a substituted or unsubstituted heterocyclic group.

In an exemplary embodiment of the present specification, Chemical Formula 1 is any one of Chemical Formulae 1-21-1 to 1-21-5, 1-22-1 to 1-22-5 and 1-23 to 1-26, and the chemical formulae are unsubstituted or substituted with an additional substituent.

Specifically, the following structures of Chemical Formulae 1-21-1 to 1-21-5 and 1-22-1 to 1-22-5 are unsubstituted or substituted with another substituent.

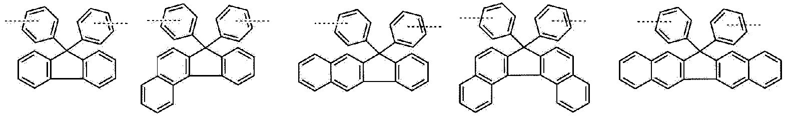

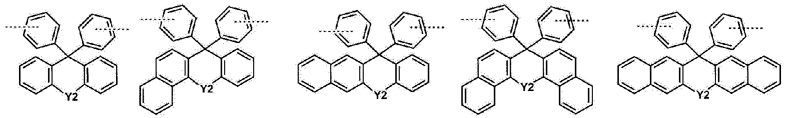

In the structures, Y2 is the same as that described above.

In an exemplary embodiment of the present specification, La is any one of the following structures, and the following structure are unsubstituted or substituted with an additional substituent.

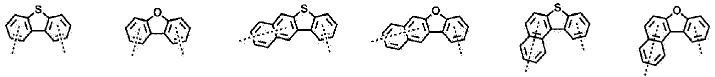

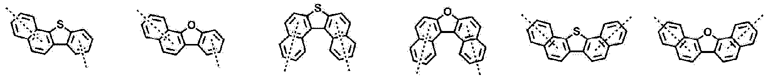

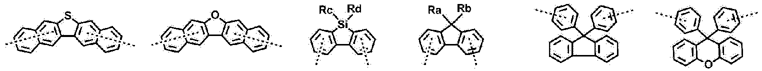

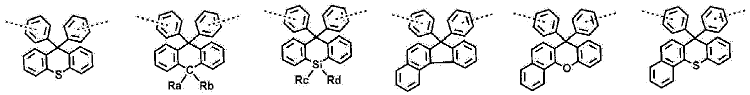

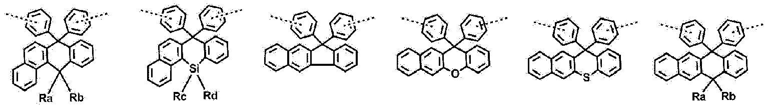

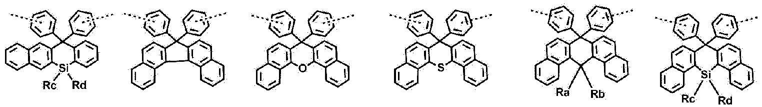

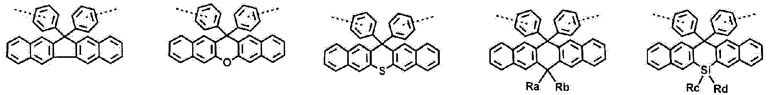

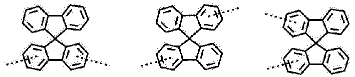

In the structures,
Ra to Rd are the same as or different from each other, and are each independently hydrogen; deuterium; a substituted or unsubstituted alkyl group; or a substituted or unsubstituted aryl group, and
------ is a moiety linked to Chemical Formula 1.

In an exemplary embodiment of the present specification, the additional substituent is one or more selected from the group consisting of deuterium; a halogen group; a substituted or unsubstituted alkyl group; a substituted or unsubstituted alkoxy group; a substituted or unsubstituted aryl group; and a substituted or unsubstituted heterocyclic group.

In an exemplary embodiment of the present specification, the additional substituent is deuterium; a halogen group; a substituted or unsubstituted alkyl group; a substituted or unsubstituted alkoxy group; a substituted or unsubstituted aryl group; or a substituted or unsubstituted heterocyclic group.

In an exemplary embodiment of the present specification, the additional substituent is deuterium; a halogen group; an alkyl group; an alkoxy group; an aryl group; or a heterocyclic group.

In an exemplary embodiment of the present specification, La is any one of the structures, and the structures are unsubstituted.

In an exemplary embodiment of the present specification, Ra to Rd are the same as or different from each other, and are each independently a substituted or unsubstituted alkyl group; or a substituted or unsubstituted aryl group.

In an exemplary embodiment of the present specification, Ra to Rd are the same as or different from each other, and are each independently a substituted or unsubstituted alkyl group having 1 to 30 carbon atoms; or a substituted or unsubstituted aryl group having 6 to 30 carbon atoms.

In an exemplary embodiment of the present specification, Ra to Rd are the same as or different from each other, and are each independently hydrogen; deuterium; an alkyl group; or an aryl group.

In an exemplary embodiment of the present specification, Ra to Rd are the same as or different from each other, and are each independently an alkyl group; or an aryl group.

In an exemplary embodiment of the present specification, Ra to Rd are the same as or different from each other, and are each independently an alkyl group having 1 to 30 carbon atoms; or an aryl group having 6 to 30 carbon atoms.

In an exemplary embodiment of the present specification, Ra to Rd are the same as or different from each other, and are each independently a methyl group; an ethyl group; a propyl group; a butyl group; a pentyl group; a hexyl group; a phenyl group; a biphenyl group; or a naphthyl group.

In an exemplary embodiment of the present specification, Ra to Rd are the same as or different from each other, and are each independently a methyl group; an ethyl group; a butyl group; a hexyl group; or a phenyl group.

In an exemplary embodiment of the present specification, the compound of Chemical Formula 1 is unsubstituted or substituted with F.

In an exemplary embodiment of the present specification, Chemical Formula 1 is the following Chemical Formula 1-B.

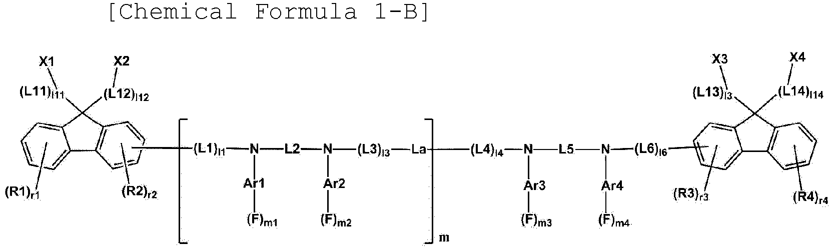

In Chemical Formula 1-B,
La, L1 to L6, L11 to L14, X1 to X4, Ar1 to Ar4, R1 to R4, m, r1 to r4, 11, 13, 14, 16 and 111 to 114 are the same as those defined in Chemical Formula 1,
m1 is 0 to the maximum bondable number of substituents capable of being bonded to Ar1,
m2 is 0 to the maximum bondable number of substituents capable of being bonded to Ar2,
m3 is 0 to the maximum bondable number of substituents capable of being bonded to Ar3, and
m4 is 0 to the maximum bondable number of substituents capable of being bonded to Ar4.

In an exemplary embodiment of the present specification, Chemical Formula 1 is the following Chemical Formula 1-B-1.

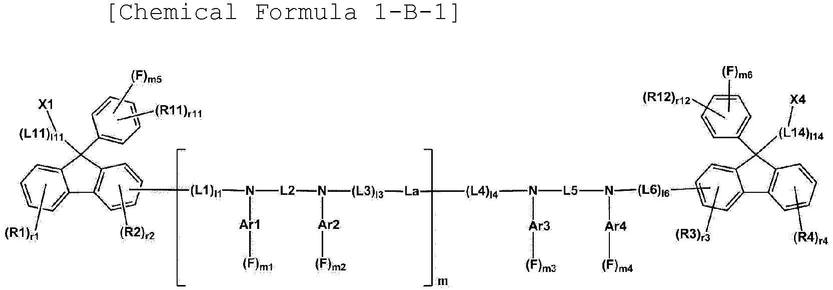

In Chemical Formula 1-B-1,
La, L1 to L6, L11, L14, Ar1 to Ar4, R1 to R4, m, r1 to r4, 11, 13, 14, 16, 111 and 114 are the same as those defined in Chemical Formula 1,
X1 and X4 are the same as or different from each other, and are each independently a photocurable group or a thermosetting group,
R11 and R12 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group,
m1 is 0 to the maximum bondable number of substituents capable of being bonded to Ar1,
m2 is 0 to the maximum bondable number of substituents capable of being bonded to Ar2,
m3 is 0 to the maximum bondable number of substituents capable of being bonded to Ar3,
m4 is 0 to the maximum bondable number of substituents capable of being bonded to Ar4,
r11 and r12 are each an integer from 1 to 5, and when r11 and r12 are 2 or higher, substituents in the parenthesis are the same as or different from each other, and
m5 and m6 are each an integer from 0 to 5, r11+m5 is 5 or lower, and r12+m6 is 5 or lower.

In the present specification, the maximum bondable number of substituents capable of being bonded to each of Ar1 to Ar4 is the number of hydrogens bonded to each of Ar1 to Ar4. For example, when Ar1 is a phenyl group, m1 is an integer from 0 to 5, and when Ar1 is a biphenyl group, m1 is an integer from 0 to 9.

In an exemplary embodiment of the present specification, the compound of Chemical Formula 1 may not be substituted with F. Specifically, m1 to m6 are may be each 0.

In an exemplary embodiment of the present specification, m1+m2+m3+m4+m5+m6 is 0 or higher.

In an exemplary embodiment of the present specification, m1+m2+m3+m4+m5+m6 is an integer from 0 to 50.

In an exemplary embodiment of the present specification, m1+m2+m3+m4+m5+m6 is an integer from 0 to 30.

In an exemplary embodiment of the present specification, m1+m2+m3+m4+m5+m6 is an integer from 0 to 10.

In an exemplary embodiment of the present specification, m1 to m4 are each an integer from 0 to 11.

In an exemplary embodiment of the present specification, m1 to m4 are each an integer from 0 to 9.

In an exemplary embodiment of the present specification, m1 to m4 are each an integer from 0 to 7.

In an exemplary embodiment of the present specification, the compound of Chemical Formula 1 may be substituted with one or more F's. Specifically, at least one of m1 to m6 may be 1 or higher.

In an exemplary embodiment of the present specification, r11+m5 is an integer from 1 to 5, and r12+m6 is an integer from 1 to 5.

In an exemplary embodiment of the present specification, one to six of m1 to m6 are 1 or higher.

In an exemplary embodiment of the present specification, two of m1 to m6 are 1 or higher.

In an exemplary embodiment of the present specification, four of m1 to m6 are 1 or higher.

In an exemplary embodiment of the present specification, six of m1 to m6 are 1 or higher.

In an exemplary embodiment of the present specification, m1+m2+m3+m4+m5+m6 is 1 or higher.

In an exemplary embodiment of the present specification, m1+m2+m3+m4+m5+m6 is an integer from 1 to 50.

In an exemplary embodiment of the present specification, m1+m2+m3+m4+m5+m6 is an integer from 1 to 30.

In an exemplary embodiment of the present specification, m1+m2+m3+m4+m5+m6 is an integer from 1 to 10.

In an exemplary embodiment of the present specification, m1+m2+m3+m4+m5+m6 is an integer from 2 to 10.

In an exemplary embodiment of the present specification, m1 to m4 are each an integer from 1 to 11.

In an exemplary embodiment of the present specification, m1 to m4 are each an integer from 1 to 9.

In an exemplary embodiment of the present specification, m1 to m4 are each an integer from 1 to 7.

In an exemplary embodiment of the present specification, m5 and m6 are each an integer from 1 to 5.

In an exemplary embodiment of the present specification, 11, 13, 14 and 16 are 0, and Chemical Formula 1 is represented by the following Chemical Formula 1-C.

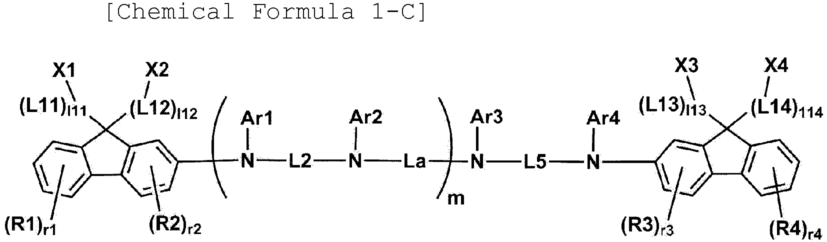

In Chemical Formula 1-C,
La, L2, L5, L11 to L14, R1 to R4, Ar1 to Ar4, X1 to X4, r1 to r4, 111 to 114 and m are those defined in Chemical Formula 1.

In an exemplary embodiment of the present specification, when La is a divalent fluorenyl group; or a divalent spirobifluorenyl group, Chemical Formula 1 is represented by Chemical Formula 1-C.

In an exemplary embodiment of the present specification, when La is a divalent fluorenyl group or a divalent spirobifluorenyl group, Chemical Formula 1 is represented by any one of the following Chemical Formulae 1-C-1 to 1-C-6.

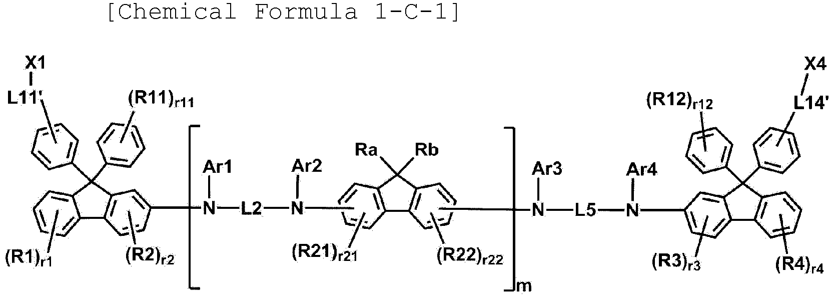

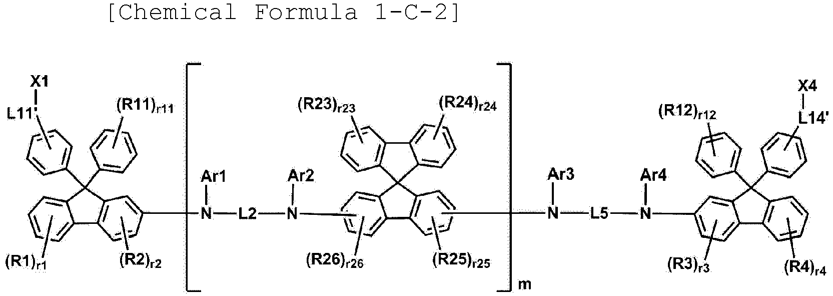

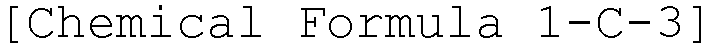

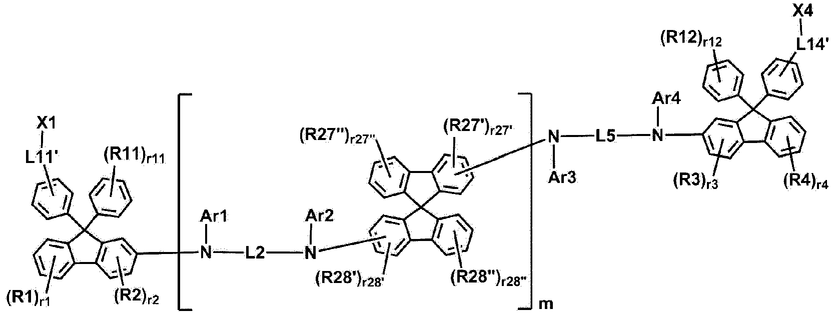

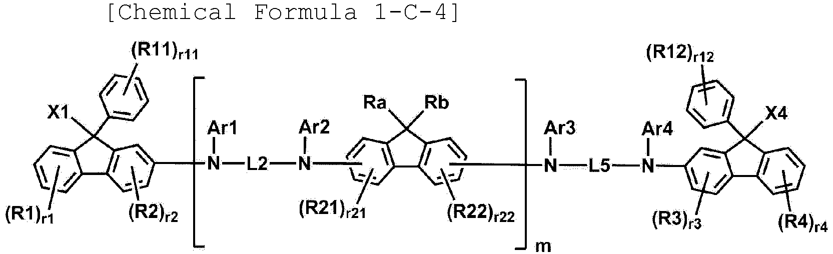

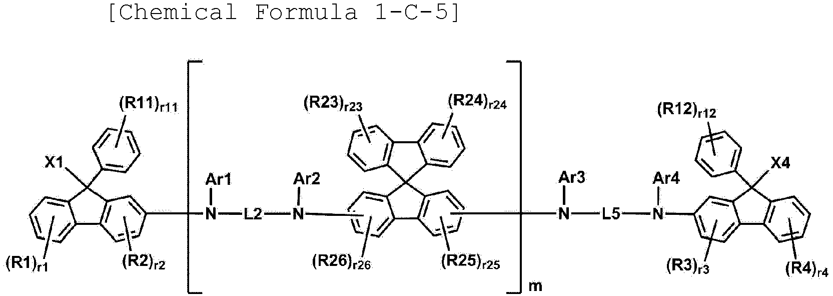

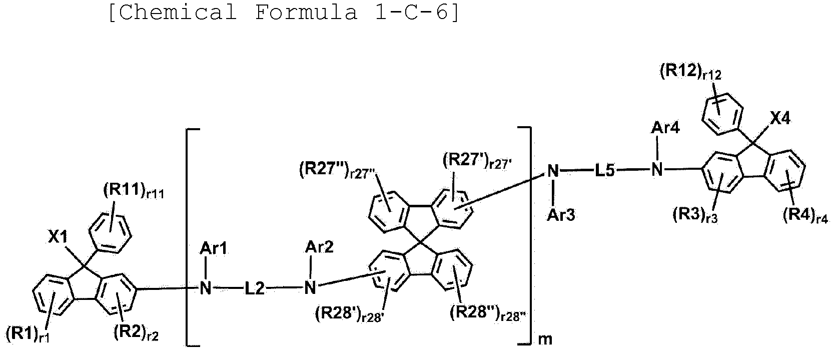

In Chemical Formulae 1-C-1 to 1-C-6,
L2, L5, Ar1 to Ar4, R1 to R4, r1 to r4 and m are the same as those defined in Chemical Formula 1,
X1 and X4 are the same as or different from each other, and are each independently a photocurable group or a thermosetting group,
L11' and L14' are the same as or different from each other, and are each independently a direct bond; or -O-,
R11, R12, R21 to R26, R27', R27'', R28' and R28'' are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group, and
r21, r22, r25, r26, r27', and r28' are each an integer from 1 to 3, r23, r24, r27'' and r28'' are each an integer from 1 to 4, r11 and r12 are each an integer from 1 to 5, and when r11, r12, r21 to r26, r27', r28', r27'' and r28'' are each 2 or higher, substituents in the parenthesis are the same as or different from each other.

In an exemplary embodiment of the present specification, when La is 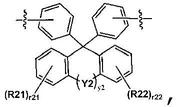 Chemical Formula 1 is represented by the following Chemical Formula 1-D1.

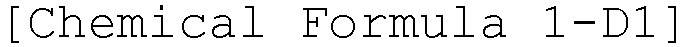

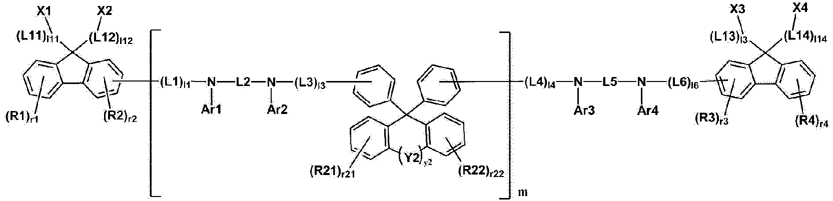

In Chemical Formula 1-D1,
L1 to L6, L11 to L14, X1 to X4, Ar1 to Ar4, R1 to R4, r1 to r4, 111 to 114, m, 11, 13, 14 and 16 are the same as those defined in Chemical Formula 1,
Y2 is O; S; CRaRb; or SiRcRd,
R21 and R22 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group,
y2 is 0 or 1, and
r21 and r22 are each an integer from 1 to 4, and when r21 and r22 are each 2 or higher, substituents in the parenthesis are the same as or different from each other.

In an exemplary embodiment of the present specification, Chemical Formula 1 is the following Chemical Formula 1-D2.

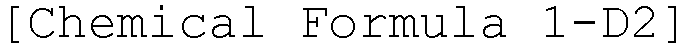

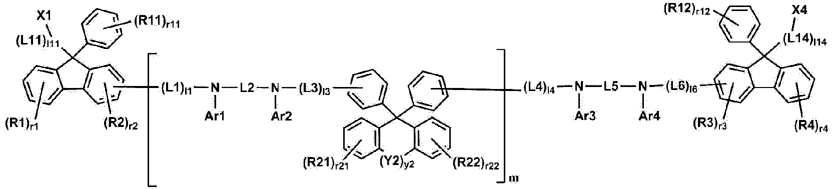

In Chemical Formula 1-D2,
L1 to L6, L11, L14, Ar1 to Ar4, R1 to R4, r1 to r4, 111, 114, m, 11, 13, 14 and 16 are the same as those defined in Chemical Formula 1,
X1 and X4 are the same as or different from each other, and are each independently a photocurable group or a thermosetting group,
Y2 is O; S; CRaRb; or SiRcRd,
R11, R12, R21 and R22 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group, and
r11 and r12 are each an integer from 1 to 5, r21 and r22 are each an integer from 1 to 4, and when r11, r12, r21 and r22 are each 2 or higher, substituents in the parenthesis are the same as or different from each other.

In an exemplary embodiment of the present specification, Chemical Formula 1-D1 is any one of the following Chemical Formulae 1-D3 to 1-D5.

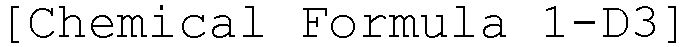

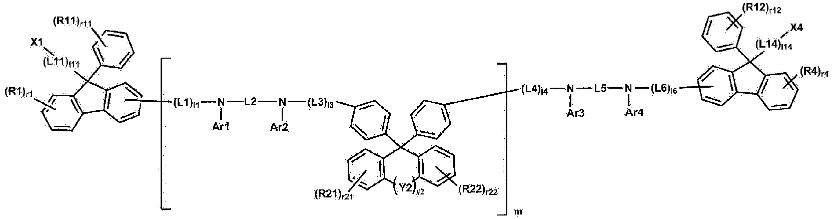

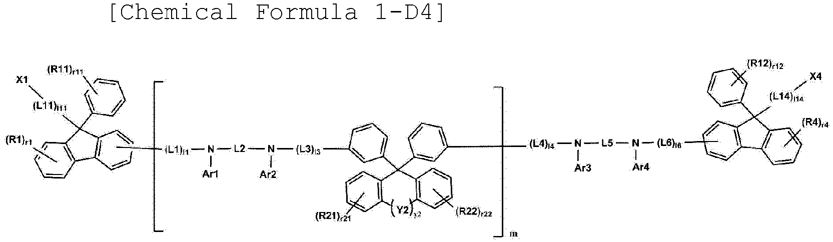

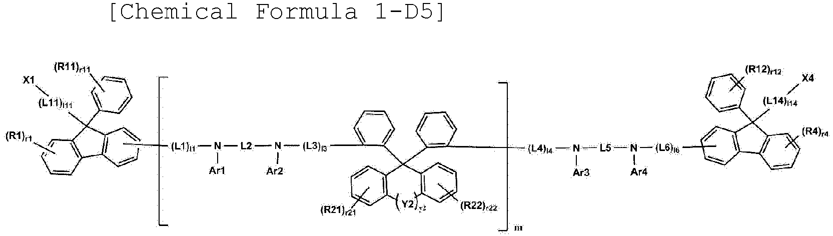

In Chemical Formulae 1-D3 to 1-D5,
L1 to L6, L11, L14, Ar1 to Ar4, R1, R4, R21, R22, 11, 13, 14, 16, 111, 114, Y2, y2, r1, r4, r21, r22 and m are the same as those defined in Chemical Formula 1-D1,
X1 and X4 are the same as or different from each other, and are each independently a photocurable group or a thermosetting group,
R11 and R12 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group, and
r11 and r12 are each an integer from 1 to 5, r21 and r22 are each an integer from 1 to 4, and when r11, r12, r21 and r22 are each 2 or higher, substituents in the parenthesis are the same as or different from each other.

In an exemplary embodiment of the present specification, Ar1 to Ar4 are the same as or different from each other, and are each independently a substituted or unsubstituted alkyl group having 1 to 30 carbon atoms; a substituted or unsubstituted aryl group having 6 to 30 carbon atoms; or a substituted or unsubstituted heterocyclic group having 2 to 30 carbon atoms.

In an exemplary embodiment of the present specification, Ar1 to Ar4 are the same as or different from each other, and are each independently a substituted or unsubstituted aryl group having 6 to 30 carbon atoms; or a substituted or unsubstituted heterocyclic group having 2 to 30 carbon atoms.

In an exemplary embodiment of the present specification, Ar1 to Ar4 are the same as or different from each other, and are each independently a group to which any one or two or more of the following structures is or are linked, and the following structures are unsubstituted or substituted with an additional substituent.

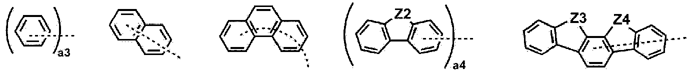

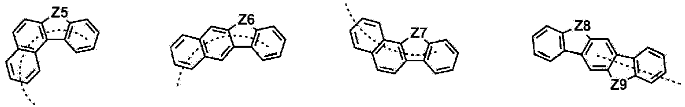

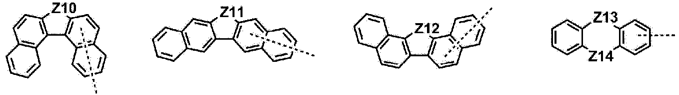

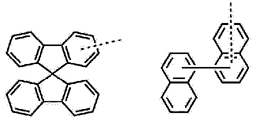

In the structures,
Z2 to Z14 are the same as or different from each other, and are each independently S; O; CRjRk; SiR1Rm; or NRn,
Rj to Rn are the same as or different from each other, and are each independently hydrogen; deuterium; a substituted or unsubstituted alkyl group; a substituted or unsubstituted aryl group; or a substituted or unsubstituted heterocyclic group,
a3 and a4 are each an integer from 1 to 5, and when a3 and a4 are each 2 or higher, structures in the parenthesis are the same as or different from each other, and
------ is a moiety bonded to Chemical Formula 1.

In an exemplary embodiment of the present specification, the additional substituent substituted in the Ar1 to Ar4 structures is one or more substituents selected from the group consisting of deuterium; a halogen group; a substituted or unsubstituted alkyl group; a substituted or unsubstituted alkoxy group; a substituted or unsubstituted aryl group; and a substituted or unsubstituted heterocyclic group.

In an exemplary embodiment of the present specification, Ar1 to Ar4 are the same as or different from each other, and are each independently a group to which any one or two or more of the following structures is or are linked, and the following structures are unsubstituted or substituted with an additional substituent.

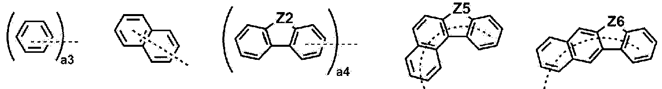

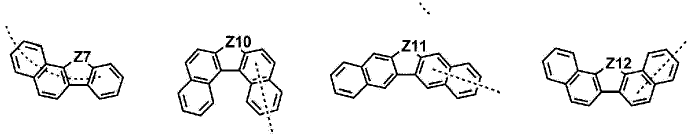

In the structures,
Z2, Z5 to Z7, Z10 to Z12, a3, a4, ------ and the additional substituent are the same as those described above.

In an exemplary embodiment of the present specification, Ar1 to Ar4 are the same as or different from each other, and are each independently an aryl group having 6 to 30 carbon atoms, which is unsubstituted or substituted with a halogen group, an alkyl group or a heterocyclic group; or a heterocyclic group having 2 to 30 carbon atoms, which is unsubstituted or substituted with a halogen group or an alkyl group.

In an exemplary embodiment of the present specification, Ar1 to Ar4 are the same as or different from each other, and are each independently a substituted or unsubstituted phenyl group; a substituted or unsubstituted biphenyl group; a substituted or unsubstituted naphthyl group; a substituted or unsubstituted fluorenyl group; a substituted or unsubstituted dibenzofuran group; or a substituted or unsubstituted dibenzothiophene group.

In an exemplary embodiment of the present specification, Ar1 to Ar4 are the same as or different from each other, and are each independently a substituted or unsubstituted phenyl group; a substituted or unsubstituted biphenyl group; a substituted or unsubstituted naphthyl group; a substituted or unsubstituted dibenzofuran group; or a substituted or unsubstituted dibenzothiophene group.

In an exemplary embodiment of the present specification, Ar1 to Ar4 are the same as or different from each other, and are each independently a phenyl group which is unsubstituted or substituted with deuterium, a halogen group, an alkyl group or a heterocyclic group; a biphenyl group which is unsubstituted or substituted with deuterium, a halogen group, an alkyl group or a heterocyclic group; a naphthyl group which is unsubstituted or substituted with deuterium, a halogen group, an alkyl group or a heterocyclic group; a dibenzofuran group which is unsubstituted or substituted with deuterium, a halogen group or an alkyl group; or a dibenzothiophene group which is unsubstituted or substituted with deuterium, a halogen group or an alkyl group.

In an exemplary embodiment of the present specification, Ar1 to Ar4 are any one of the following structures, and the following structures are unsubstituted or substituted with an additional substituent.

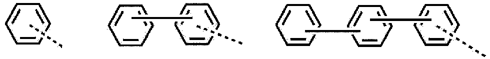

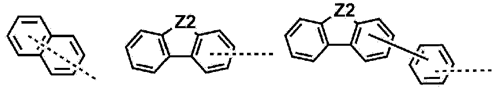

In the structures, Z2, ------ and the additional substituent are the same as those described above.

In an exemplary embodiment of the present specification, the additional substituent substituted in the Ar1 to Ar4 structures is one or more selected from the group consisting of deuterium; a halogen group; a substituted or unsubstituted alkyl group having 1 to 30 carbon atoms; a substituted or unsubstituted alkoxy group having 1 to 30 carbon atoms; a substituted or unsubstituted aryl group having 6 to 30 carbon atoms; and a substituted or unsubstituted heterocyclic group having 2 to 30 carbon atoms.

In an exemplary embodiment of the present specification, the additional substituent substituted in the Ar1 to Ar4 structures is a halogen group; an alkyl group; or a heterocyclic group.

In an exemplary embodiment of the present specification, the structures are unsubstituted or substituted with a halogen group; an alkyl group; or a heterocyclic group.

In an exemplary embodiment of the present specification, -Ar1- (F)ₘ₁, -Ar2- (F)ₘ₂, -Ar3- (F)ₘ₃ and -Ar4-(F)ₘ₄ may be each represented by any one of the following structures, and the following structures are unsubstituted or substituted with an additional substituent.

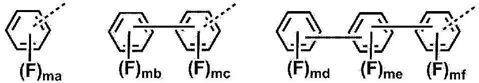

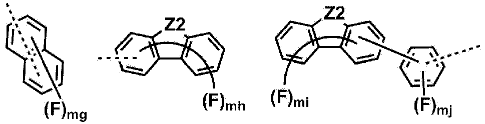

In the structures,
Z2 and ------ are the same as those described above, and
ma, mb and md are an integer from 0 to 5, mc, me, mf and mj are an integer from 0 to 4, and mg, mh and mi are an integer from 0 to 7.

In an exemplary embodiment of the present specification, the additional substituent substituted in the -Ar1-(F)ₘ₁, -Ar2-(F)ₘ₂, -Ar3-(F)ₘ₃ and -Ar4-(F)ₘ₄ structures is one or more substituents selected from the group consisting of deuterium; a halogen group; a substituted or unsubstituted alkyl group; a substituted or unsubstituted alkoxy group; a substituted or unsubstituted aryl group; and a substituted or unsubstituted heterocyclic group.

In an exemplary embodiment of the present specification, the structures are unsubstituted or substituted with an alkyl group.

In an exemplary embodiment of the present specification, ma, mb and md are an integer from 1 to 5, mc, me, mf and mj are an integer from 1 to 4, and mg, mh and mi are an integer from 1 to 7.

In an exemplary embodiment of the present specification, Z2 is O or S.

In an exemplary embodiment of the present specification, L1 to L6 are the same as or different from each other, and are each independently a substituted or unsubstituted arylene group having 6 to 30 carbon atoms; or a substituted or unsubstituted divalent heterocyclic group having 2 to 30 carbon atoms.

In an exemplary embodiment of the present specification, L1 to L6 are the same as or different from each other, and are each independently a substituted or unsubstituted arylene group having 6 to 30 carbon atoms.

In an exemplary embodiment of the present specification, L1 to L6 are the same as or different from each other, and are each independently a group to which any one or two or more of the following structures is or are linked, and the following structures are unsubstituted or substituted with an additional substituent.

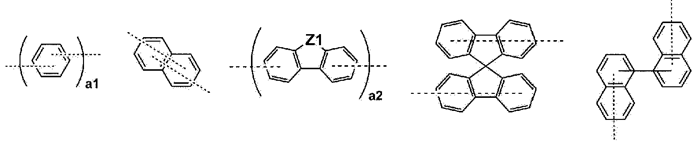

In the structures,
Z1 is S; O; CReRf; SiRgRh; or NRi,
Re to Ri are the same as or different from each other, and are each independently hydrogen; deuterium; a substituted or unsubstituted alkyl group; a substituted or unsubstituted aryl group; or a substituted or unsubstituted heterocyclic group,
a1 and a2 are each an integer from 1 to 5, and when a1 to a2 are each 2 or higher, structures in the parenthesis are the same as or different from each other, and
------ is a moiety bonded to Chemical Formula 1.

In an exemplary embodiment of the present specification, the additional substituent substituted in the L1 to L6 structures is one or more selected from the group consisting of deuterium; a halogen group; a substituted or unsubstituted alkyl group; a substituted or unsubstituted alkoxy group; a substituted or unsubstituted aryl group; and a substituted or unsubstituted heterocyclic group.

In an exemplary embodiment of the present specification, the additional substituent substituted in the L1 to L6 structures is one or more selected from the group consisting of deuterium; a halogen group; a substituted or unsubstituted alkyl group having 1 to 30 carbon atoms; a substituted or unsubstituted alkoxy group having 1 to 30 carbon atoms; a substituted or unsubstituted aryl group having 6 to 30 carbon atoms; and a substituted or unsubstituted heterocyclic group having 2 to 30 carbon atoms.

In an exemplary embodiment of the present specification, the additional substituent substituted in the L1 to L6 structures is an alkyl group or an aryl group.

In an exemplary embodiment of the present specification, the structures are unsubstituted or substituted with an alkyl group or an aryl group.

In an exemplary embodiment of the present specification, L1 to L6 are the same as or different from each other, and are each independently a substituted or unsubstituted phenylene group; a substituted or unsubstituted biphenylene group; a substituted or unsubstituted terphenylene group; a substituted or unsubstituted fluorenylene group; a substituted or unsubstituted divalent spirobifluorenyl group; a substituted or unsubstituted phenanthrenylene group; a substituted or unsubstituted naphthylene group; or a substituted or unsubstituted binaphthylene group, or a group to which two or more of the substituents are linked.

In an exemplary embodiment of the present specification, L1 to L6 are the same as or different from each other, and are each independently a substituted or unsubstituted phenylene group; a substituted or unsubstituted biphenylene group; a substituted or unsubstituted terphenylene group; or a substituted or unsubstituted divalent spirobifluorenyl group.

In an exemplary embodiment of the present specification, L1 to L6 are the same as or different from each other, and are each independently a phenylene group which is unsubstituted or substituted with an alkyl group or an aryl group; a biphenylene group which is unsubstituted or substituted with an alkyl group or an aryl group; a terphenylene group which is unsubstituted or substituted with an alkyl group or an aryl group; or a divalent spirobifluorenyl group which is unsubstituted or substituted with an alkyl group or an aryl group.

In an exemplary embodiment of the present specification, L1, L3, L4 and L6 are the same as or different from each other, and are each independently a substituted or unsubstituted phenylene group.

In an exemplary embodiment of the present specification, L1, L3, L4 and L6 are the same as or different from each other, and are each independently a phenylene group.

In an exemplary embodiment of the present specification, 11, 13, 14 and 16 are each 0 or 1.

In an exemplary embodiment of the present specification, when 11, 13, 14 and 16 are 0, L1, L3, L4 and L6 may be represented by a direct bond.

In an exemplary embodiment of the present specification, 11, 13, 14 and 16 are each O.

In an exemplary embodiment of the present specification, 11, 13, 14 and 16 are each 1.

In an exemplary embodiment of the present specification, 11 and 16 are 0, and 13 and 14 are 1.

In an exemplary embodiment of the present specification, 11 and 16 are 1, and 13 and 14 are O.

In an exemplary embodiment of the present specification, L2 and L5 are the same as or different from each other, and are each independently a substituted or unsubstituted phenylene group; a substituted or unsubstituted biphenylene group; a substituted or unsubstituted terphenylene group; a substituted or unsubstituted fluorenylene group; a substituted or unsubstituted phenanthrenylene group; a substituted or unsubstituted naphthylene group; or a substituted or unsubstituted binaphthylene group, or a group to which two or more of the substituents are linked.

In an exemplary embodiment of the present specification, L2 and L5 are the same as or different from each other, and are each independently a substituted or unsubstituted phenylene group; a substituted or unsubstituted biphenylene group; a substituted or unsubstituted terphenylene group; or a substituted or unsubstituted divalent spirobifluorenyl group.

In an exemplary embodiment of the present specification, L2 and L5 are the same as or different from each other, and are each independently a phenylene group; a biphenylene group; a terphenylene group; or a divalent spirobifluorenyl group.

In an exemplary embodiment of the present specification, L11 and L14 are the same as or different from each other, and are each independently a direct bond; -O-; a substituted or unsubstituted alkylene group having 1 to 30 carbon atoms; or a substituted or unsubstituted arylene group having 6 to 30 carbon atoms.

In an exemplary embodiment of the present specification, L11 and L14 are the same as or different from each other, and are each independently a direct bond; -O-; a methylene group; a substituted or unsubstituted ethylene group; a substituted or unsubstituted propylene group; or a substituted or unsubstituted phenylene group.

In an exemplary embodiment of the present specification, L11 and L14 are the same as or different from each other, and are each independently a direct bond; -O-; a methylene group; an ethylene group; a propylene group; or a phenylene group.

In an exemplary embodiment of the present specification, the photocurable group or the thermosetting group is any one of the following structures.

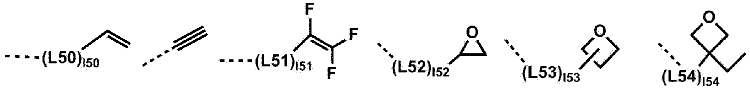

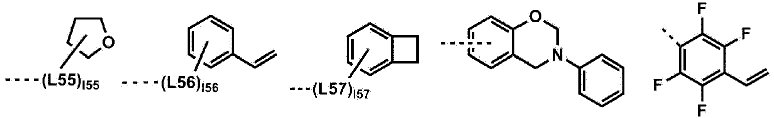

In the structures,
L50 to L57 are the same as or different from each other, and are each independently a direct bond; -O-; or a substituted or unsubstituted alkylene group,
150 to 157 are each an integer from 1 to 5, and when 150 to 157 are each 2 or higher, structures in the parenthesis are the same as or different from each other, and
------ is a moiety bonded to Chemical Formula 1.

In an exemplary embodiment of the present specification, the photocurable group or the thermosetting group is 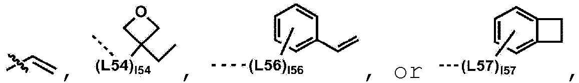 L54, L56 and L57 are the same as or different from each other, and are each independently a direct bond; -O-; or a substituted or unsubstituted alkylene group, 154, 156 and 157 are each an integer from 1 to 5, and when 154, 156 and 157 are each 2 or higher, structures in the parenthesis are the same as or different from each other.

In an exemplary embodiment of the present specification, the photocurable group or the thermosetting group is any one of the following structures.

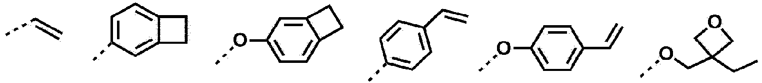

In an exemplary embodiment of the present specification, R1 to R4 are the same as or different from each other, and are each independently hydrogen; deuterium; a halogen group; or a substituted or unsubstituted alkyl group.

In an exemplary embodiment of the present specification, R1 to R4 are the same as or different from each other, and are each independently hydrogen; deuterium; a halogen group; or a substituted or unsubstituted alkyl group having 1 to 30 carbon atoms.

In an exemplary embodiment of the present specification, R1 to R4 are the same as or different from each other, and are each independently hydrogen; deuterium; a halogen group; or a substituted or unsubstituted alkyl group having 1 to 10 carbon atoms.

In an exemplary embodiment of the present specification, R1 to R4 are each hydrogen; deuterium; or a halogen group.

In an exemplary embodiment of the present specification, R1 to R4 are each hydrogen; or deuterium.

In an exemplary embodiment of the present specification, R11 and R12 are the same as or different from each other, and are each independently hydrogen; deuterium; a halogen group; or a substituted or unsubstituted alkyl group having 1 to 10 carbon atoms.

In an exemplary embodiment of the present specification, R11 and R12 are the same as or different from each other, and are each independently hydrogen; deuterium; a halogen group; or an alkyl group having 1 to 10 carbon atoms.

In an exemplary embodiment of the present specification, R11 and R12 are the same as or different from each other, and are each independently hydrogen; deuterium; a halogen group; or an alkyl group having 1 to 10 carbon atoms.

In an exemplary embodiment of the present specification, when m is 1, Chemical Formula 1 is represented by the following Chemical Formula 1-31.

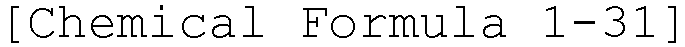

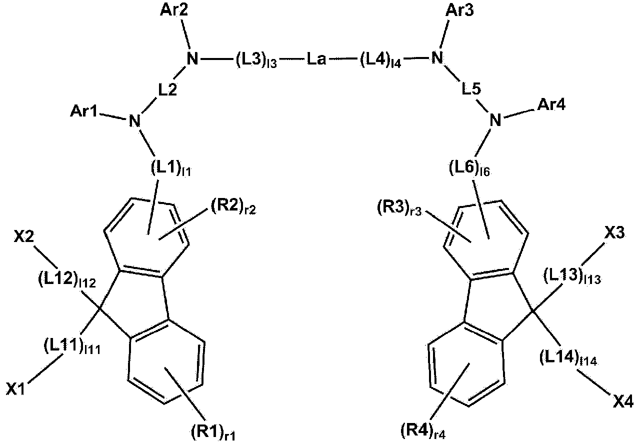

In Chemical Formula 1-31,
La, L1 to L6, L11 to L14, X1 to X4, Ar1 to Ar4, R1 to R4, 11, 13, 14, 16, 111 to 114 and r1 to r4 are the same as those defined in Chemical Formula 1.

In an exemplary embodiment of the present specification, Chemical Formula 1-31 is represented by the following Chemical Formula 1-31-1.

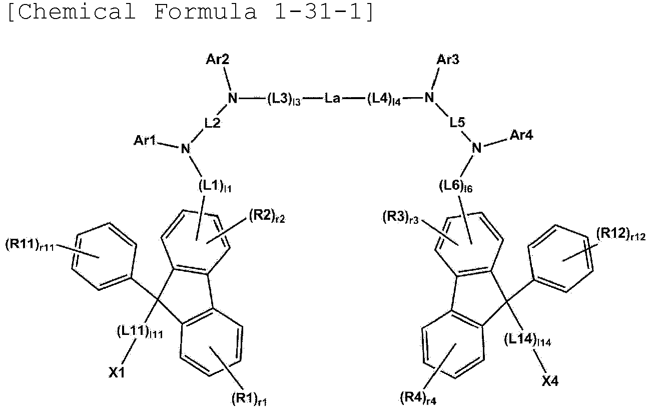

In Chemical Formula 1-31-1,
La, L1 to L6, L11, L14, Ar1 to Ar4, R1 to R4, 11, 13, 14, 16, 111, 114 and r1 to r4 are the same as those defined in Chemical Formula 1,
X1 and X4 are the same as or different from each other, and are each independently a photocurable group or a thermosetting group,
R11 and R12 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group, and
r11 and r12 are each an integer from 1 to 5, and when r11 and r12 are each 2 or higher, substituents in the parenthesis are the same as or different from each other.

In an exemplary embodiment of the present specification, when m is 2 to 10, the following structure is repeated as much as the number of m.

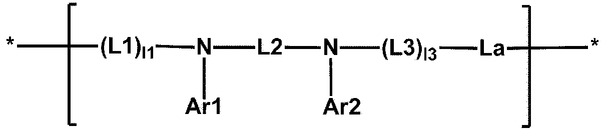

In the structure, L1 to L3, 11, 13, La, Ar1 and Ar2 are the same as those defined in Chemical Formula 1, and * is an attachment point in the compound.

For example, when m is 2, Chemical Formula 1 may be represented by the following Chemical Formula 1-32.

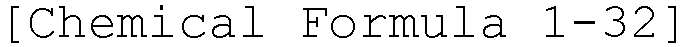

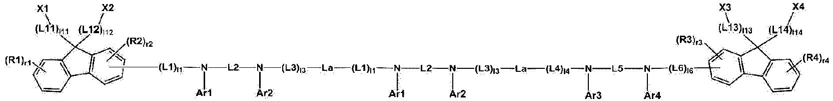

In Chemical Formula 1-32,
La, L1 to L6, L11 to L14, X1 to X4, Ar1 to Ar4, R1 to R4, 11, 13, 14, 16, 111 to 114 and r1 to r4 are the same as those defined in Chemical Formula 1.

In an exemplary embodiment of the present specification, Chemical Formula 1-32 is represented by the following Chemical Formula 1-32-1.

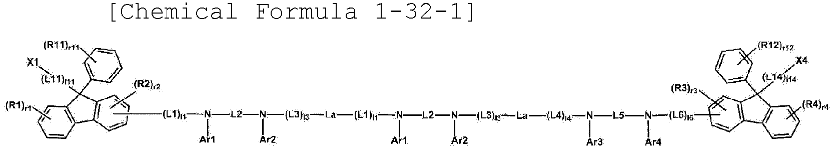

In Chemical Formula 1-32-1,
La, L1 to L6, L11, L14, Ar1 to Ar4, R1 to R4, 11, 13, 14, 16, 111, 114, and r1 to r4 are the same as those defined in Chemical Formula 1,
X1 and X4 are the same as or different from each other, and are each independently a photocurable group or a thermosetting group,
R11 and R12 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group, and
r11 and r12 are each an integer from 1 to 5, and when r11 and r12 are each 2 or higher, substituents in the parenthesis are the same as or different from each other.

In an exemplary embodiment of the present specification, when n is 2 or higher, structures in the parenthesis are the same as or different from each other.

In an exemplary embodiment of the present specification, when n is 2 or higher, structures in the parenthesis are the same as each other.

In an exemplary embodiment of the present specification, Chemical Formula 1 is any one of the following structures.

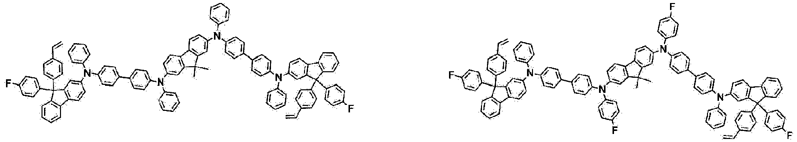

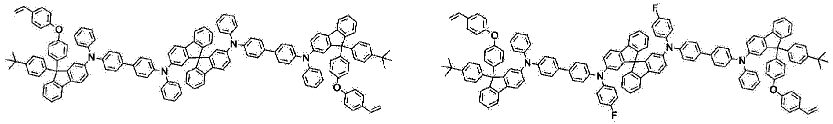

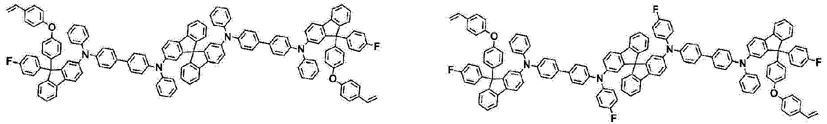

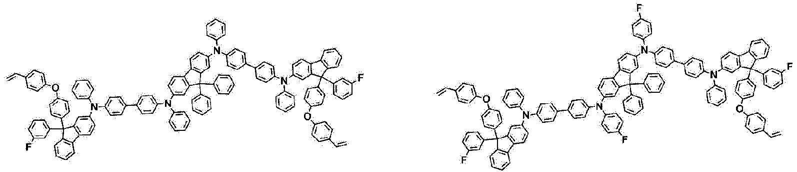

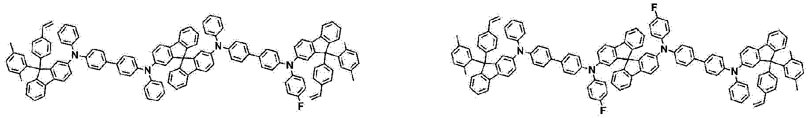

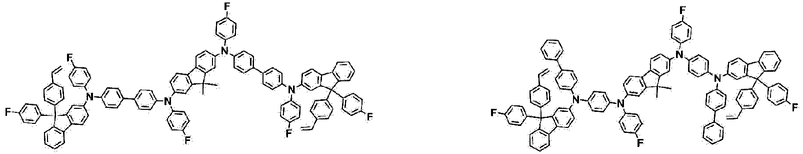

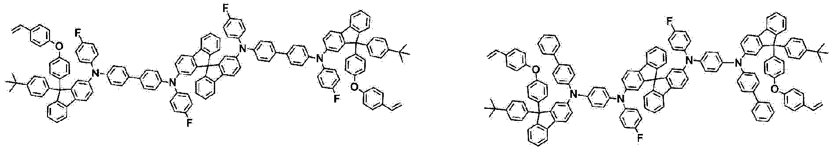

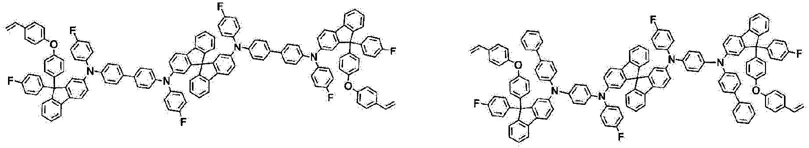

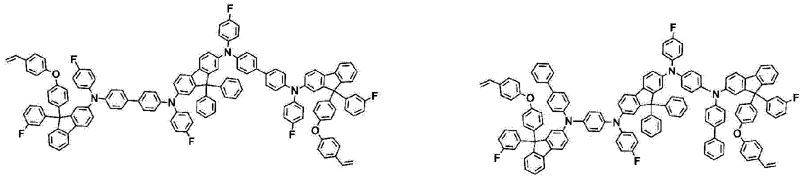

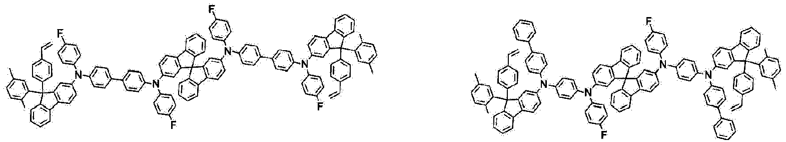

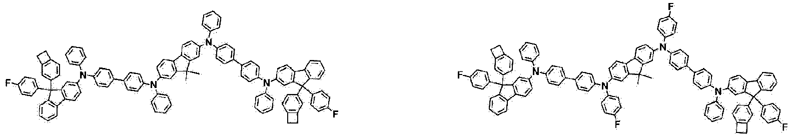

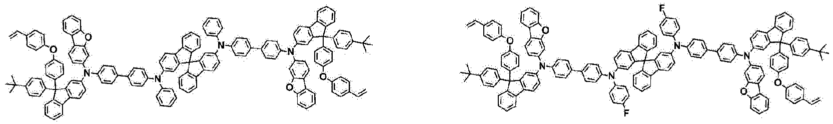

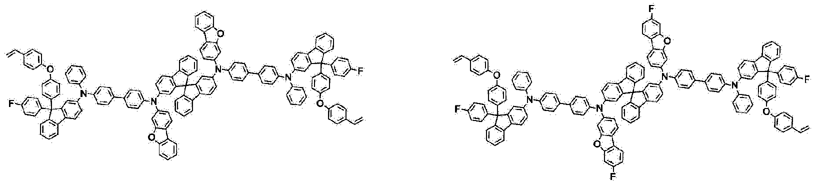

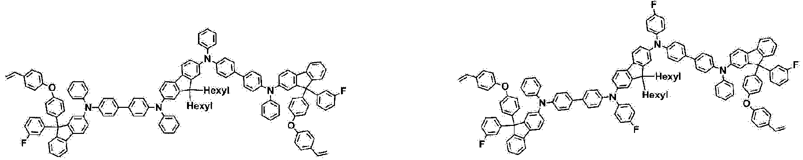

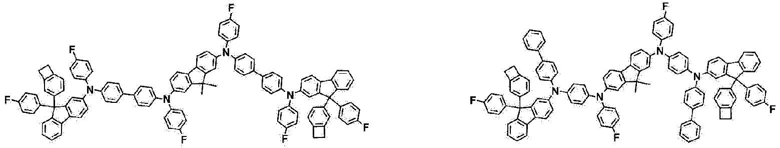

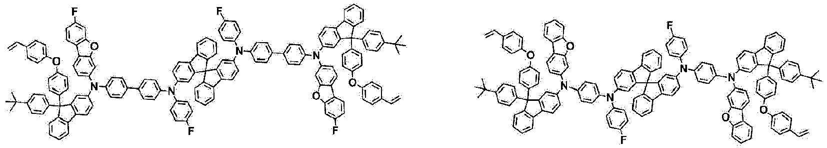

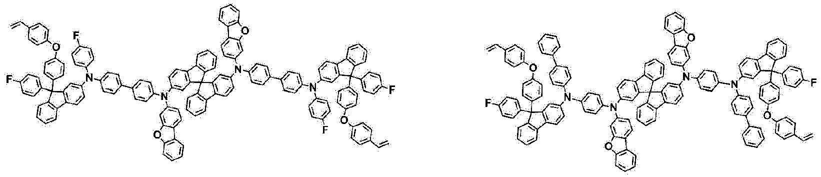

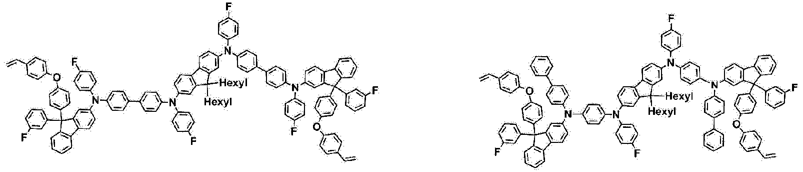

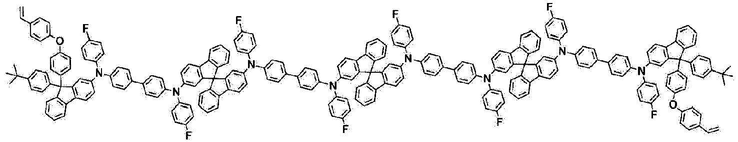

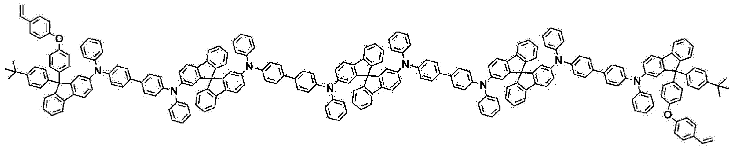

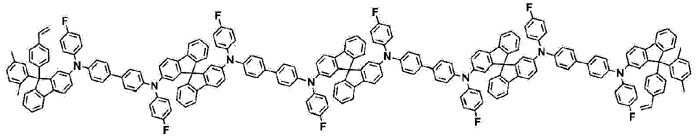

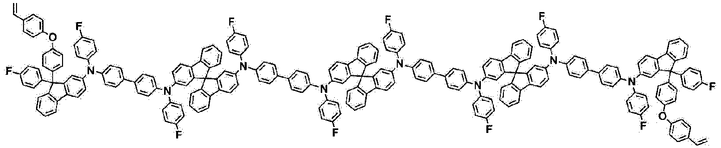

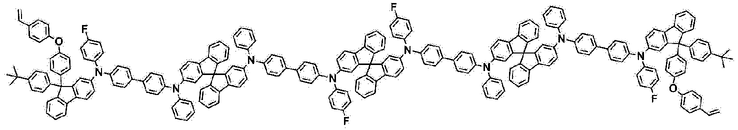

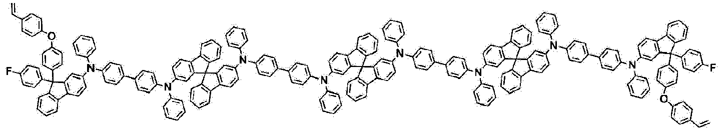

In an exemplary embodiment of the present specification, Chemical Formula 1 is any one of the following structures.

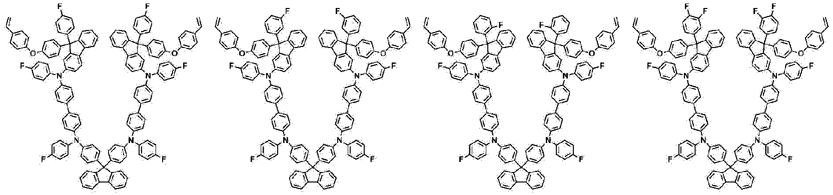

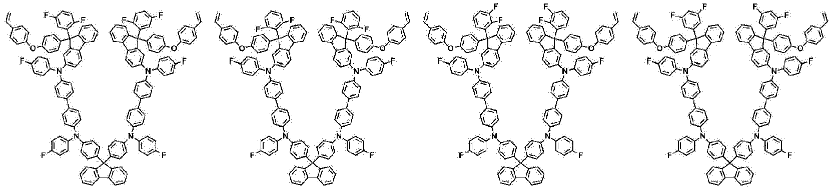

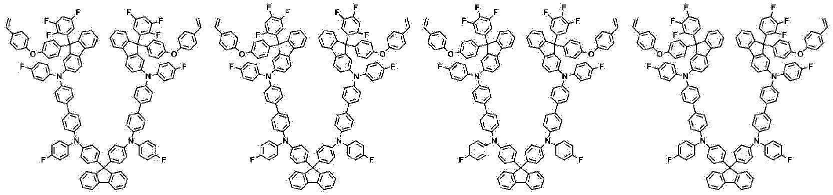

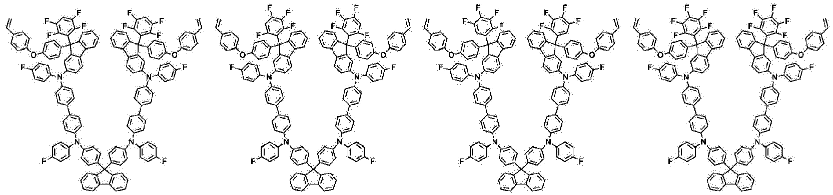

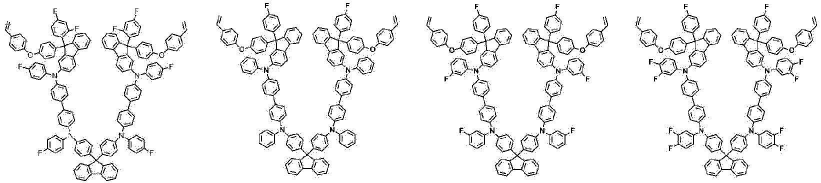

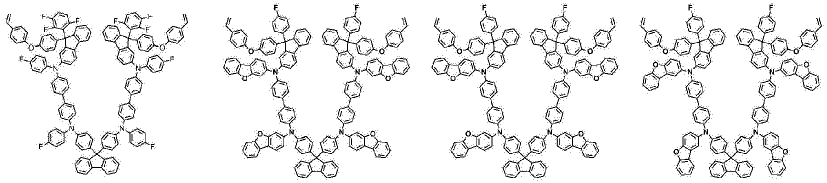

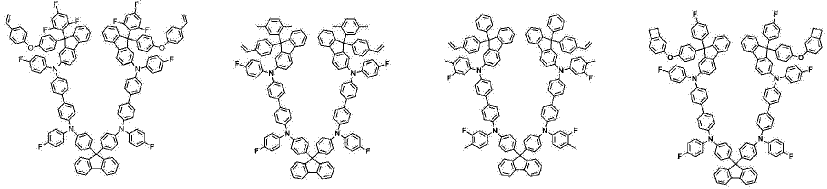

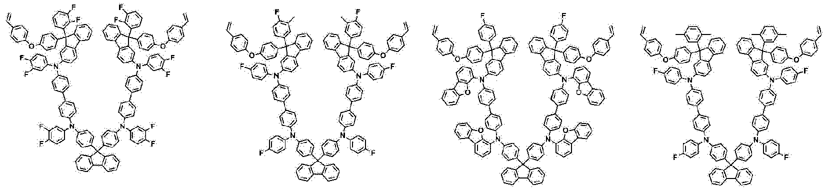

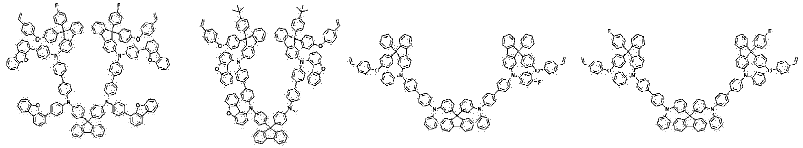

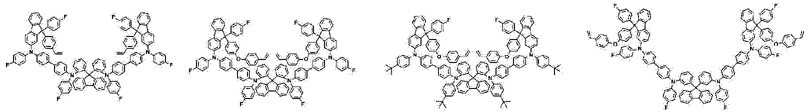

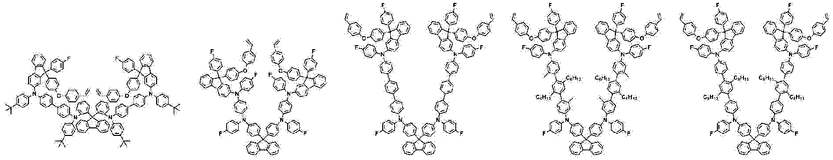

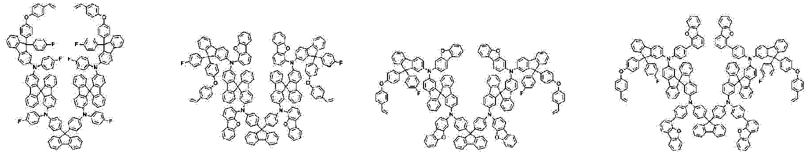

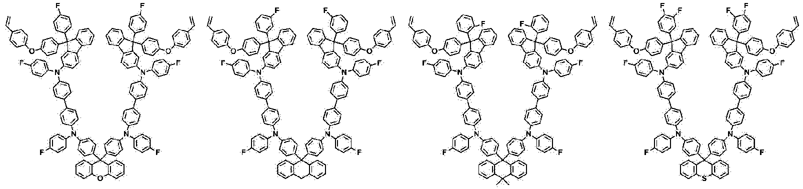

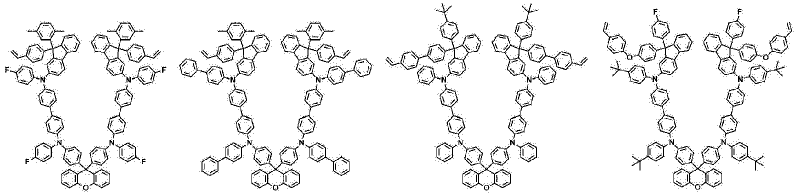

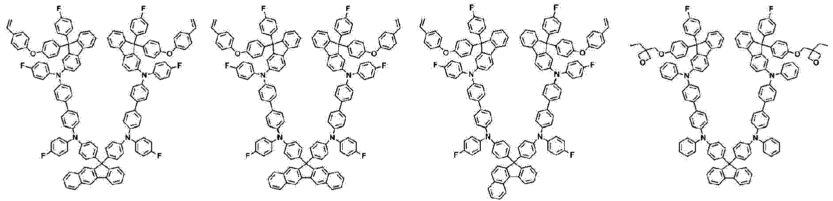

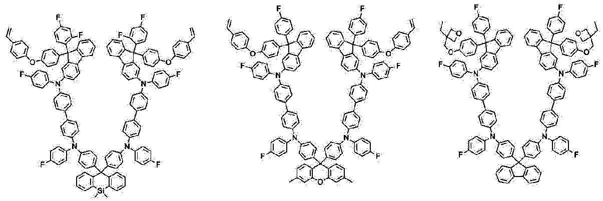

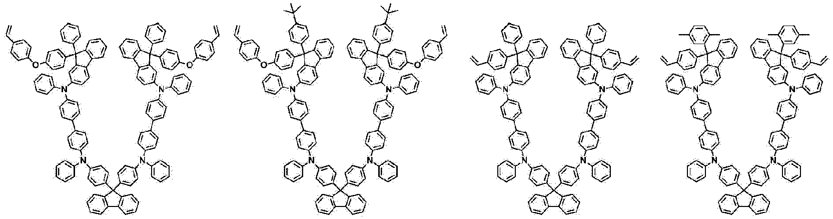

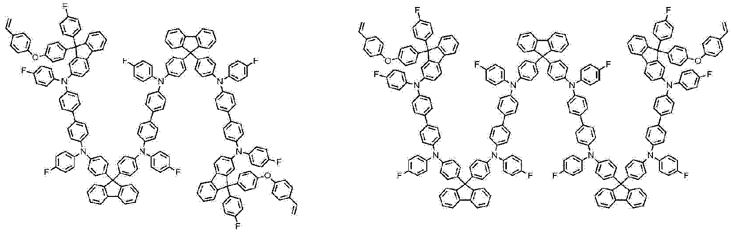

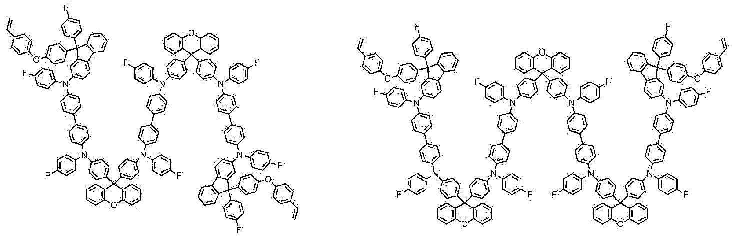

In an exemplary embodiment of the present specification, Chemical Formula 1 is any one of the following structures.

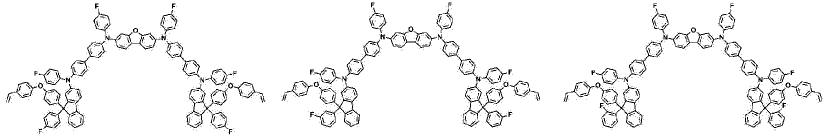

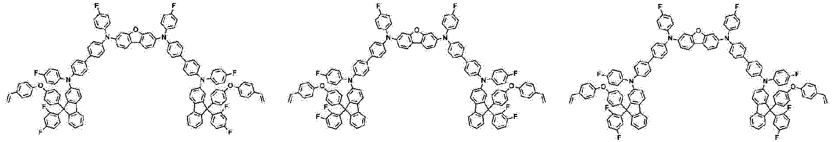

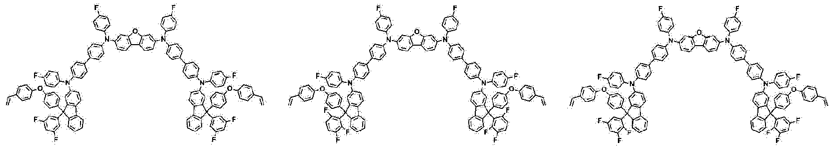

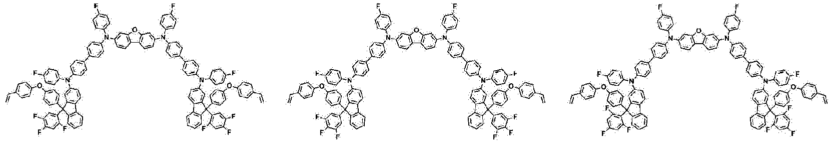

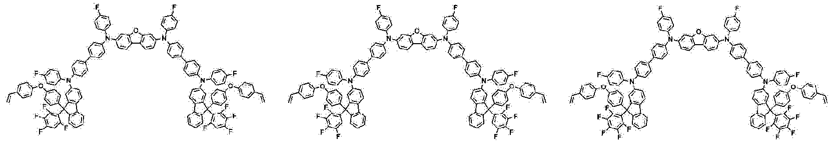

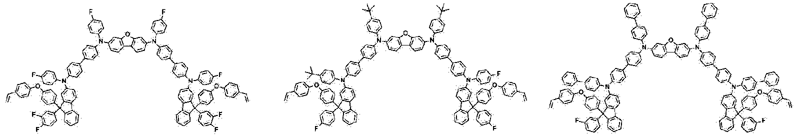

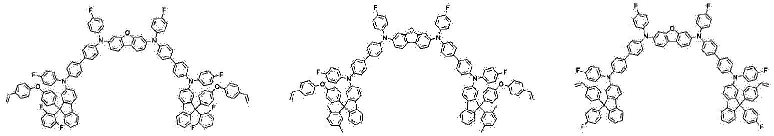

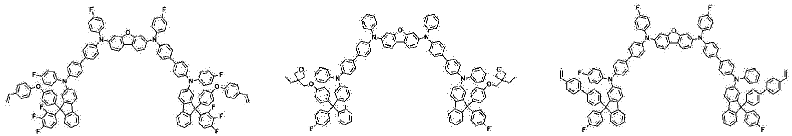

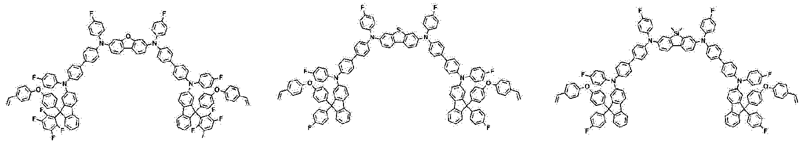

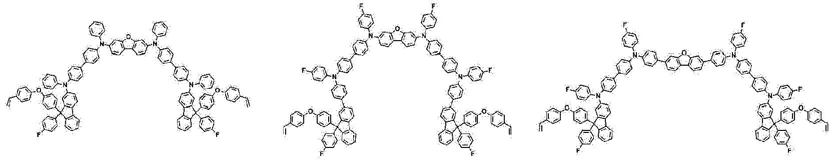

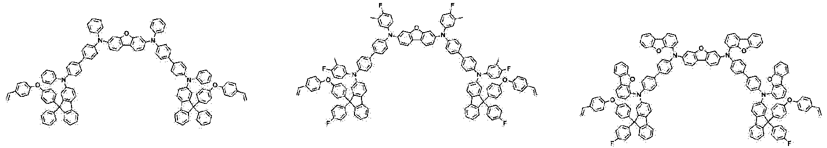

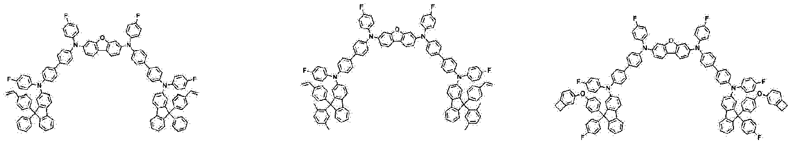

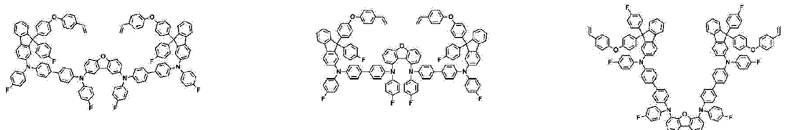

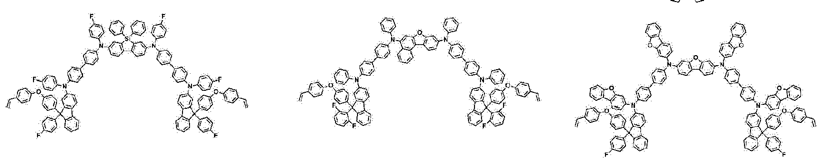

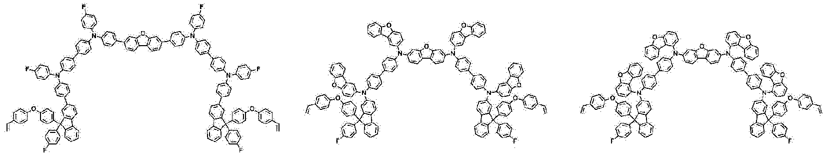

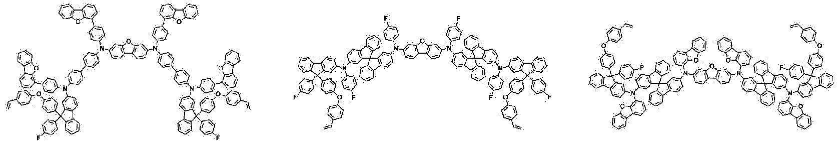

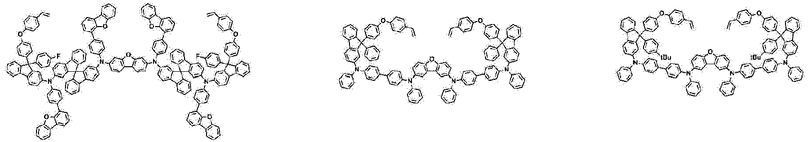

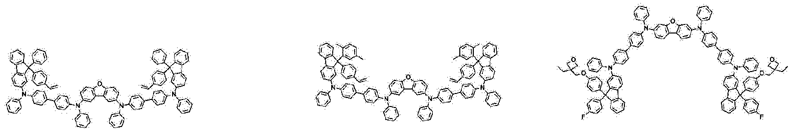

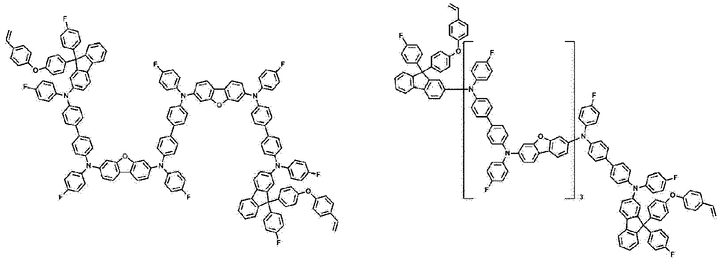

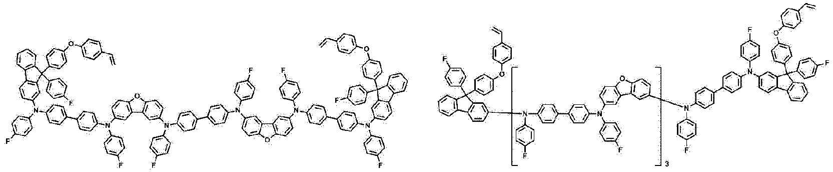

In the structures, hydrogen can be replaced with deuterium.

In an exemplary embodiment of the present specification, the compound is deuterated by 10% or more.

In the present specification, "deuteration" means that an available hydrogen of a compound is substituted with deuterium.

In the present specification, N% deuteration means that N% of hydrogen available in the corresponding structure is substituted with deuterium. For example, 50% deuteration of a phenyl group means that three of six hydrogens of the phenyl group are substituted with deuteriums.

In the present specification, the degree of deuteration may be confirmed by a publicly-known method such as nuclear magnetic resonance spectroscopy (¹H NMR) or GC/MS.

In an exemplary embodiment of the present specification, the compound is deuterated by 10% to 100%.

In an exemplary embodiment of the present specification, the compound is deuterated by 10% to 90%.

In an exemplary embodiment of the present specification, the compound is deuterated by 20% or more.

In an exemplary embodiment of the present specification, the compound is deuterated by 20% to 100%.

In an exemplary embodiment of the present specification, the compound is deuterated by 20% to 80%.

The compound according to an exemplary embodiment of the present specification may be prepared by a preparation method to be described below.

For example, the compound of Chemical Formula 1 may be prepared as a core structure as shown in the following Reaction Scheme, and may be prepared by changing the number of linkers and/or chains in the following Reaction Scheme. Further, the compound of Chemical Formula 1 may be prepared by the Buchwald-Hartwig amination reaction. In the following Reaction Scheme, the substituent may be bonded by a method known in the art, and the type and position of the substituent or the number of substituents may be changed according to the technology known in the art.

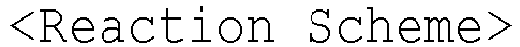

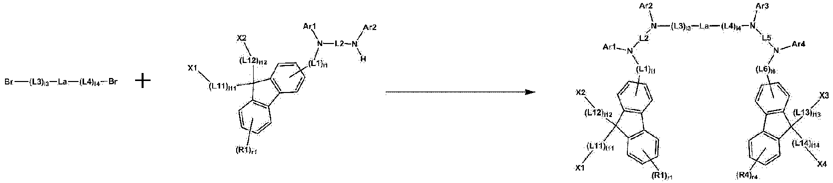

In Reaction Scheme, La, L1 to L6, L11 to L14, X1 to X4, Ar1 to Ar4, R1, R4, m, 11, 13, 14, 16, 111 to 114, r1 and r4 are the same as those defined in Chemical Formula 1.

The present specification provides a coating composition including the above-described compound of Chemical Formula 1.

In an exemplary embodiment of the present specification, the coating composition further includes a solvent. In an exemplary embodiment of the present specification, the coating composition includes the compound of Chemical Formula 1 and a solvent.

In an exemplary embodiment of the present specification, the coating composition may be in a liquid phase. The "liquid phase" means that the composition is in a liquid state at room temperature under atmospheric pressure.

In an exemplary embodiment of the present specification, when the coating composition is applied to an organic material layer, a solvent that does not dissolve a material in a lower layer is used. Accordingly, there is an advantage in that the organic material layer can be introduced by the solution process.

In an exemplary embodiment of the present specification, since the compound includes a photocurable group or a thermosetting group, the resistance to solvent is improved during the heat treatment after coating. That is, the compound is cross-linked and/or cured after coating, and thus is not dissolved in a specific solvent.

For example, even though a coating composition is prepared by using a solvent that dissolves the compound and a layer is manufactured by a solution process, the layer may have resistance to the same solvent when cured through heat treatment.

Therefore, when an organic material layer is formed by using the compound and then subjected to a heat treatment process, a solution process can be performed when another organic material layer is applied.

For example, when the coating composition is applied to a hole injection layer, there is an advantage in that when an upper layer (a hole transport layer and the like) is manufactured, the upper layer can be introduced by a solution process by using a specific solvent to which the cured coating composition shows resistance.

In an exemplary embodiment of the present specification, the solvent included in the coating composition is a solvent that dissolves the compound. Examples of the solvent include: a chlorine-based solvent such as chloroform, methylene chloride, 1,2-dichloroethane, 1,1,2-trichloroethane, chlorobenzene, and o-dichlorobenzene; an ether-based solvent such as tetrahydrofuran and dioxane; an aromatic hydrocarbon-based solvent such as toluene, xylene, trimethylbenzene, and mesitylene; a ketone-based solvent such as acetone, methyl ethyl ketone, and cyclohexanone; an ester-based solvent such as ethyl acetate, butyl acetate, and ethyl cellosolve acetate; a polyhydric alcohol such as ethylene glycol, ethylene glycol monobutyl ether, ethylene glycol monoethyl ether, ethylene glycol monomethyl ether, dimethoxy ethane, propylene glycol, diethoxymethane, triethylene glycol monoethyl ether, glycerin, and 1,2-hexanediol and a derivative thereof; an alcohol-based solvent such as methanol, ethanol, propanol, isopropanol, and cyclohexanol; a sulfoxide-based solvent such as dimethyl sulfoxide; an amide-based solvent such as N-methyl-2-pyrrolidone and N,N-dimethylformamide; a benzoate-based solvent such as methyl benzoate, butyl benzoate, and 3-phenoxybenzoate; and a solvent such as tetralin, but the solvent can be used as long as the solvent can dissolve or disperse the compound according to an exemplary embodiment of the present specification, and is not limited thereto.

In an exemplary embodiment of the present specification, the solvents may be used either alone or in a mixture of two or more solvents.

In an exemplary embodiment of the present specification, the coating composition does not further include a p-doping material.

In an exemplary embodiment of the present specification, the coating composition further includes a p-doping material.

In the present specification, the p-doping material means a material which allows a host material to have p-semiconductor characteristics. The p-semiconductor characteristics mean characteristics of injecting or transporting holes at the highest occupied molecular orbital (HOMO) energy level, that is, characteristics of a material having large hole conductivity.

In an exemplary embodiment of the present specification, the p-doping material may be any one of the following structures, but is not limited thereto.

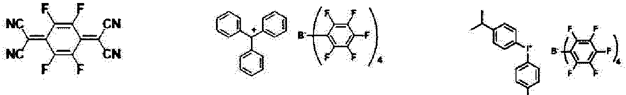

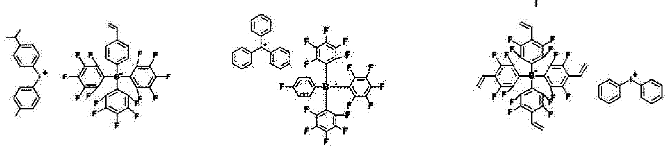

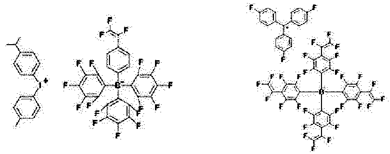

In the present specification, the p-doping material is sufficient as long as the material is a material which allows a material to have p-semiconductor characteristics, one or two or more thereof may be used, and the type thereof is not limited.

In an exemplary embodiment of the present specification, the content of the p-doping material is 0 wt% to 500 wt% based on the compound of Chemical Formula 1. Specifically, the content of the p-doping material is 100 wt% to 400 wt% based on the compound of Chemical Formula 1.

In an exemplary embodiment of the present specification, the p-doping material is included in an amount of 0 to 50 wt% based on the total solid content of the coating composition. In an exemplary embodiment of the present specification, it is preferred that the p-doping material is included in an amount of 1 to 50 wt% based on the total solid content of the coating composition, and it is more preferred that the p-doping material is included in an amount of 10 to 30 wt% based on the total solid content of the coating composition.

In another exemplary embodiment, the coating composition further includes: a single molecule including a cross-linkable functional group by heat or light; or a single molecule including an end group capable of forming a polymer by heat.

In an exemplary embodiment of the present specification, the single molecule including a cross-linkable functional group by heat or light; or the single molecule including an end group capable of forming a polymer by heat may be a compound having a molecular weight of 3,000 g/mol or less.

In an exemplary embodiment of the present specification, the single molecule including a cross-linkable functional group by heat or light; or the single molecule including an end group capable of forming a polymer by heat may mean a single molecule in which a cross-linkable functional group by heat or light or an end group capable of forming a polymer by heat is substituted in an aryl such as phenyl, biphenyl, fluorene, and naphthalene; arylamine; or fluorene.

In an exemplary embodiment of the present specification, the coating composition has a viscosity of 2 cP to 15 cP at room temperature. Specifically, the coating composition has a viscosity of 2 cP to 10 cP. When the above viscosity is satisfied, a device is easily manufactured.

The viscosity is a value measured at 25°C using an Ubbelohde viscometer after dissolving the polymer whose viscosity is to be measured in a chloroform solvent at a concentration of 0.5 g/dl.

An exemplary embodiment of the present specification provides an organic light emitting device formed by using the coating composition.

An exemplary embodiment of the present specification provides an organic light emitting device including: a first electrode; a second electrode; and an organic material layer having one or more layers, which includes a light emitting layer provided between the first electrode and the second electrode, in which one or more layers of the organic material layer include the coating composition or a cured product thereof. In this case, the cured product of the coating composition is in a state in which the coating composition is cured by a heat treatment or a light treatment.

In an exemplary embodiment of the present specification, the organic material layer including the coating composition or the cured product thereof is a hole transport layer or a hole injection layer.

In an exemplary embodiment of the present specification, the organic material layer including the coating composition or the cured product thereof is an electron transport layer or an electron injection layer.

In an exemplary embodiment of the present specification, the organic material layer including the coating composition or the cured product thereof is a light emitting layer.

In an exemplary embodiment of the present specification, the organic material layer including the coating composition or the cured product thereof is a light emitting layer, and the light emitting layer includes the compound of Chemical Formula 1 as a host of the light emitting layer.

In an exemplary embodiment of the present specification, the organic material layer including the coating composition or the cured product thereof is a light emitting layer, and the light emitting layer includes the compound of Chemical Formula 1 as a dopant of the light emitting layer.

In an exemplary embodiment of the present specification, the organic light emitting device includes one or two or more layers selected from the group consisting of a hole injection layer, a hole transport layer, an electron transport layer, an electron injection layer, an electron blocking layer, a hole blocking layer, a layer which simultaneously transports and injects holes, and a layer which simultaneously transports and injects electrons.

In an exemplary embodiment of the present specification, the first electrode is an anode, and the second electrode is a cathode.

According to another exemplary embodiment, the first electrode is a cathode, and the second electrode is an anode.

In another exemplary embodiment, the organic light emitting device may be a normal type organic light emitting device in which an anode, an organic material layer having one or more layers, and a cathode are sequentially stacked on a substrate.

In still another exemplary embodiment, the organic light emitting device may be an inverted type organic light emitting device in which a cathode, an organic material layer having one or more layers, and an anode are sequentially stacked on a substrate.

The organic material layer of the organic light emitting device of the present specification may also have a single-layered structure, but may have a multi-layered structure in which two or more organic material layers are stacked. For example, the organic light emitting device of the present invention may have a structure including a hole injection layer, a hole transport layer, a light emitting layer, an electron transport layer, an electron injection layer, a layer which simultaneously transports and injects holes, a layer which simultaneously transports and injects electrons, and the like as an organic material layer. However, the structure of the organic light emitting device is not limited thereto, and may include a fewer number of organic material layers.

For example, the structure of the organic light emitting device according to an exemplary embodiment of the present specification is exemplified in FIG. 1.

FIG. 1 exemplifies a structure of an organic light emitting device in which an anode 201, a hole injection layer 301, a hole transport layer 401, a light emitting layer 501, an electron transport and injection layer 601, and a cathode 701 are sequentially stacked on a substrate 101.

FIG. 1 exemplifies an organic light emitting device, and the structure of the organic light emitting device of the present invention is not limited thereto.

When the organic light emitting device includes a plurality of organic material layers, the organic material layers may be formed of the same material or different materials.

The organic light emitting device of the present invention may be stacked as a structure in the following examples.
(1) Anode/Hole transport layer/Light emitting layer/Cathode
(2) Anode/Hole injection layer/Hole transport layer/Light emitting layer/Cathode
(3) Anode/Hole injection layer/Hole buffer layer/Hole transport layer/Light emitting layer/Cathode
(4) Anode/Hole transport layer/Light emitting layer/Electron transport layer/Cathode
(5) Anode/Hole transport layer/Light emitting layer/Electron transport layer/Electron injection layer/Cathode
(6) Anode/Hole injection layer/Hole transport layer/Light emitting layer/Electron transport layer/Cathode
(7) Anode/Hole injection layer/Hole transport layer/Light emitting layer/Electron transport layer /Electron injection layer/Cathode
(8) Anode/Hole injection layer/Hole buffer layer/Hole transport layer/Light emitting layer/Electron transport layer/Cathode
(9) Anode/Hole injection layer/Hole buffer layer/Hole transport layer/Light emitting layer/Electron transport layer/Electron injection layer/Cathode
(10) Anode/Hole transport layer/Electron blocking layer/Light emitting layer/Electron transport layer/Cathode
(11) Anode/Hole transport layer/Electron blocking layer/Light emitting layer/Electron transport layer/Electron injection layer/Cathode
(12) Anode/Hole injection layer/Hole transport layer/Electron blocking layer/Light emitting layer/Electron transport layer/Cathode
(13) Anode/Hole injection layer/Hole transport layer/Electron blocking layer/Light emitting layer/Electron transport layer/Electron injection layer/Cathode
(14) Anode/Hole transport layer/Light emitting layer/Hole blocking layer/Electron transport layer/Cathode
(15) Anode/Hole transport layer/Light emitting layer/Hole blocking layer/Electron transport layer/Electron injection layer/Cathode
(16) Anode/Hole injection layer/Hole transport layer/Light emitting layer/Hole blocking layer/Electron transport layer/Cathode
(17) Anode/Hole injection layer/Hole transport layer/Light emitting layer/Hole blocking layer/Electron transport layer/Electron injection layer/Cathode
(18) Anode/Hole injection layer/Hole transport layer/Electron blocking layer/Light emitting layer/Hole blocking layer/Electron injection layer and transport layer/Cathode

In the structures, the "Electron transport layer/Electron injection layer" may be replaced with "Electron transport and injection layer" or "Layer which simultaneously transports and injects electrons".

For example, the organic light emitting device of the present invention may be stacked as a structure such as 'Anode/Hole injection layer/Hole transport layer/Light emitting layer/Electron transport and injection layer/Cathode' in which the Electron transport layer/Electron injection layer of (7) is replaced with the electron transport and injection layer.

Further, in the structures, the "Hole injection layer/Hole transport layer" may be replaced with the "hole injection and transport layer" or the "layer which simultaneously injects and transports holes".

The organic light emitting device of the present specification may be manufactured by the materials and methods known in the art, except that one or more layers of the organic material layer are formed by using the coating composition including the compound of Chemical Formula 1.

For example, the organic light emitting device of the present specification may be manufactured by sequentially stacking an anode, an organic material layer, and a cathode on a substrate. In this case, the organic light emitting device may be manufactured by depositing a metal or a metal oxide having conductivity, or an alloy thereof on a substrate to form an anode, forming an organic material layer including a hole injection layer, a hole transport layer, a light emitting layer, and a layer which simultaneously transports and injects electrons thereon through a solution process, a deposition process, and the like, and then depositing a material, which may be used as a cathode, thereon, by using a physical vapor deposition (PVD) method such as sputtering or e-beam evaporation. In addition to the method described above, an organic light emitting device may be made by sequentially depositing a cathode material, an organic material layer, and an anode material on a substrate.

The present specification also provides a method for manufacturing an organic light emitting device formed by using the coating composition.

Specifically, an exemplary embodiment of the present specification includes: preparing a substrate; forming a first electrode on the substrate; forming an organic material layer having one or more layers on the first electrode; and forming a second electrode on the organic material layer, in which the forming of the organic material layer includes forming an organic material layer having one or more layers by using the coating composition.

In an exemplary embodiment of the present specification, the forming of the organic material layer having one or more layers by using the coating composition uses a spin coating method.

In another exemplary embodiment, the forming of the organic material layer having one or more layers by using the coating composition uses a printing method.

In an exemplary embodiment of the present specification, examples of the printing method include inkjet printing, nozzle printing, offset printing, transfer printing or screen printing, and the like, but are not limited thereto.

A solution process is suitable for the coating composition according to an exemplary embodiment of the present specification due to the structural characteristics thereof, so that the organic material layer may be formed by a printing method, and as a result, there is an economic effect in terms of time and costs when a device is manufactured.

In an exemplary embodiment of the present specification, the forming of the organic material layer having one or more layers by using the coating composition includes: coating the coating composition on the first electrode; and heat-treating or light-treating the coated coating composition.

In an exemplary embodiment of the present specification, the heat-treating of the coated coating composition may be performed by a heat treatment. The heat treatment temperature in the heat-treating of the coated coating composition is 85°C to 250°C. The heat treatment temperature may be specifically 100°C to 250°C, more specifically 150°C to 250°C.

In an exemplary embodiment of the present specification, the heat treatment time in the heat-treating of the coated coating composition may be 1 minute to 2 hours, may be 1 minute to 1 hour according to an exemplary embodiment, and may be 30 minutes to 1 hour in another exemplary embodiment.

In an exemplary embodiment of the present specification, the light-treating of the coated coating composition may be performed by UV irradiation. The light-treating of the coated coating composition may be performed for 30 minutes to 5 hours.

In an exemplary embodiment of the present specification, the coating of the first electrode with the coating composition includes coating the first electrode with the coating composition and coating another organic material layer provided on the first electrode with the coating composition.

In an exemplary embodiment of the present specification, another organic material layer means an organic material layer formed of another material without including the coating composition or the cured product thereof.

In the coating of the first electrode with the coating composition, a solvent that does not dissolve a material in the lower layer is used. For example, when the coating composition is applied to a hole transport layer, the coating composition includes a solvent that does not dissolve the material in the lower layer (first electrode, hole injection layer, and the like). Accordingly, there is an advantage in that the hole transport layer can be introduced by the solution process.

Through the heat-treating or light-treating of the coated coating composition, a plurality of the compounds included in the coating composition may form a crosslinkage, thereby providing an organic material layer including a thin-filmed structure. In this case, it is possible to prevent the organic material layer from being dissolved, morphologically affected or decomposed by a solvent when another layer is stacked on the surface of the organic material layer formed by using the coating composition.

Therefore, when the organic material layer formed by using the coating composition is formed by a method including the heat-treating or light-treating of the coated coating composition, resistance to a solvent is increased, so that a plurality of layers may be formed by repeatedly performing solution deposition and cross-linking methods, and stability is increased, so that the service life characteristic of the device may be increased.

In an exemplary embodiment of the present specification, as the anode material, materials having a high work function are usually preferred so as to facilitate the injection of holes into an organic material layer. Specific examples of the anode material include: a metal such as vanadium, chromium, copper, zinc, and gold, or an alloy thereof; a metal oxide such as zinc oxide, indium oxide, indium tin oxide (ITO), and indium zinc oxide (IZO); a combination of a metal and an oxide, such as ZnO:Al or SnO₂:Sb; a conductive polymer such as poly(3-methylthiophene), poly[3,4-(ethylene-1,2-dioxy)thiophene] (PEDOT), polypyrrole, and polyaniline; and the like, but are not limited thereto.

In an exemplary embodiment of the present specification, as the cathode material, materials having a low work function are usually preferred so as to facilitate the injection of electrons into an organic material layer. Specific examples of the cathode material include: a metal such as barium, magnesium, calcium, sodium, potassium, titanium, indium, yttrium, lithium, gadolinium, aluminum, silver, tin, and lead, or an alloy thereof; a multi-layer structured material, such as LiF/Al or LiO₂/Al; and the like, but are not limited thereto.

In an exemplary embodiment of the present specification, the hole injection layer is a layer which injects holes from an electrode, and a hole injection material is preferably a compound which has a capability of transporting holes, and thus has an effect of injecting holes at an anode and an excellent effect of injecting holes into a light emitting layer or a light emitting material, prevents excitons produced from the light emitting layer from moving to an electron injection layer or an electron injection material, and is also excellent in the ability to form a thin film. In addition, the highest occupied molecular orbital (HOMO) of the hole injection material is preferably a value between the work function of the anode material and the HOMO of the neighboring organic material layer. Specific examples of the hole injection material include the above-described compound of Chemical Formula 1, metal porphyrin, oligothiophene, arylamine-based organic materials, hexanitrile hexaazatriphenylene-based organic materials, quinacridone-based organic materials, perylene-based organic materials, anthraquinone, polyaniline-based and polythiophene-based electrically conductive polymers, and the like, but are not limited thereto.

In an exemplary embodiment of the present specification, the hole transport layer is a layer which accepts holes from a hole injection layer and transports the holes to a light emitting layer, and a hole transport material is suitably a material having high hole mobility which may accept holes from an anode or a hole injection layer and transfer the holes to a light emitting layer. As specific examples of the hole transport material, arylamine-based organic materials, conductive polymers, block copolymers having both conjugated portions and nonconjugated portions, and the like may be used, but the hole transport material is not limited thereto. More specifically, a compound including an arylamine group may be used for the hole transport layer.

In an exemplary embodiment of the present specification, the hole transport layer includes a compound of the following Chemical Formula HT-1.

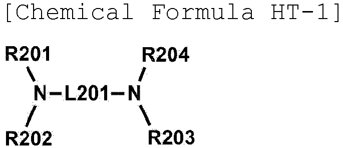

In Chemical Formula HT-1,
L201 is a substituted or unsubstituted arylene group, and
R201 to R204 are the same as or different from each other, and are each independently hydrogen; deuterium; a substituted or unsubstituted alkyl group; a substituted or unsubstituted cycloalkyl group; a substituted or unsubstituted aryl group; or a substituted or unsubstituted heterocyclic group.

In an exemplary embodiment of the present specification, L201 is a substituted or unsubstituted arylene group having 6 to 30 carbon atoms.

In an exemplary embodiment of the present specification, L201 is an arylene group.

In an exemplary embodiment of the present specification, L201 is a substituted or unsubstituted phenylene group; a substituted or unsubstituted biphenylene group; or a substituted or unsubstituted naphthylene group.

In an exemplary embodiment of the present specification, L201 is a phenylene group; a biphenylene group; or a naphthylene group.

In an exemplary embodiment of the present specification, L201 is a biphenylene group.

In an exemplary embodiment of the present specification, R201 to R204 are the same as or different from each other, and are each independently hydrogen; deuterium; a substituted or unsubstituted alkyl group having 1 to 30 carbon atoms; a substituted or unsubstituted cycloalkyl group having 3 to 30 carbon atoms; a substituted or unsubstituted aryl group having 6 to 30 carbon atoms; or a substituted or unsubstituted heterocyclic group having 2 to 30 carbon atoms.

In an exemplary embodiment of the present specification, R201 to R204 are the same as or different from each other, and are each independently a substituted or unsubstituted aryl group having 6 to 30 carbon atoms.

In an exemplary embodiment of the present specification, R201 to R204 are the same as or different from each other, and are each independently an aryl group.

In an exemplary embodiment of the present specification, R201 to R204 are the same as or different from each other, and are each independently a substituted or unsubstituted phenyl group; a substituted or unsubstituted biphenyl group; or a substituted or unsubstituted naphthyl group.

In an exemplary embodiment of the present specification, R201 to R204 are the same as or different from each other, and are each independently a phenyl group; a biphenyl group; or a naphthyl group.

In an exemplary embodiment of the present specification, Chemical Formula HT-1 is the following structure.

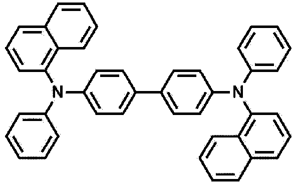

In an exemplary embodiment of the present specification, the light emitting material included in the light emitting layer is a material which may receive holes and electrons from a hole transport layer and an electron transport layer, respectively, and combine the holes and the electrons to emit light in a visible ray region, and is preferably a material having good quantum efficiency to fluorescence or phosphorescence. Specific examples thereof include: 8-hydroxy-quinoline aluminum complexes (Alq₃); carbazole-based compounds; dimerized styryl compounds; BAlq; 10-hydroxybenzoquinoline-metal compounds; benzoxazole-based, benzthiazole-based and benzimidazole-based compounds; poly(p-phenylenevinylene) (PPV)-based polymers; spiro compounds; polyfluorene, rubrene, and the like, but are not limited thereto.

In an exemplary embodiment of the present specification, the light emitting layer may include a host material and a dopant material. Examples of the host material include a fused aromatic ring derivative, a hetero ring-containing compound, or the like. Specifically, examples of the fused aromatic ring derivatives include anthracene derivatives, pyrene derivatives, naphthalene derivatives, pentacene derivatives, phenanthrene compounds, fluoranthene compounds, and the like, and examples of the hetero ring-containing compounds include carbazole derivatives, dibenzofuran derivatives, ladder-type furan compounds, pyrimidine derivatives, and the like, but the examples thereof are not limited thereto. More specifically, an anthracene derivative may be used as the host.

In an exemplary embodiment of the present specification, the host of the light emitting layer includes a compound of the following Chemical Formula EH-1.

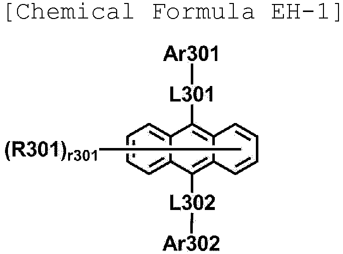

In Chemical Formula EH-1,
L301 and L302 are the same as or different from each other, and are each independently a direct bond; a substituted or unsubstituted arylene group; or a substituted or unsubstituted divalent heterocyclic group,
Ar301 and Ar302 are the same as or different from each other, and are each independently hydrogen; deuterium; a substituted or unsubstituted aryl group; or a substituted or unsubstituted heterocyclic group,
R301 is hydrogen; deuterium; a halogen group; a substituted or unsubstituted alkyl group; a substituted or unsubstituted cycloalkyl group; a substituted or unsubstituted aryl group; or a substituted or unsubstituted heterocyclic group, and
r301 is an integer from 1 to 7, and when r301 is 2 or higher, two or more R301's are the same as or different from each other.

In an exemplary embodiment of the present specification, L301 and L302 are the same as or different from each other, and are each independently a direct bond; a substituted or unsubstituted monocyclic arylene group; or a substituted or unsubstituted polycyclic arylene group.

In an exemplary embodiment of the present specification, L301 and L302 are the same as or different from each other, and are each independently a direct bond; a substituted or unsubstituted phenylene group; a substituted or unsubstituted biphenylylene group; or a substituted or unsubstituted naphthylene group.

In an exemplary embodiment of the present specification, L301 and L302 are each a direct bond.

In an exemplary embodiment of the present specification, Ar301 and Ar302 are the same as or different from each other, and are each independently a substituted or unsubstituted aryl group.

In an exemplary embodiment of the present specification, Ar301 and Ar302 are the same as or different from each other, and are each independently a substituted or unsubstituted monocyclic aryl group; or a substituted or unsubstituted polycyclic aryl group.

In an exemplary embodiment of the present specification, Ar301 and Ar302 are the same as or different from each other, and are each independently a substituted or unsubstituted phenyl group; a substituted or unsubstituted biphenyl group; a substituted or unsubstituted terphenyl group; a substituted or unsubstituted naphthyl group; a substituted or unsubstituted anthracenyl group; a substituted or unsubstituted phenanthrene group; a substituted or unsubstituted triphenylene group; a substituted or unsubstituted pyrene group; or a substituted or unsubstituted fluorenyl group.

In an exemplary embodiment of the present specification, Ar301 and Ar302 are the same as or different from each other, and are each independently a substituted or unsubstituted phenyl group; or a substituted or unsubstituted naphthyl group.

In an exemplary embodiment of the present specification, Ar301 and Ar302 are each a naphthyl group.

In an exemplary embodiment of the present specification, R301 is hydrogen.

In an exemplary embodiment of the present specification, Chemical Formula EH-1 is any one of the following structures.

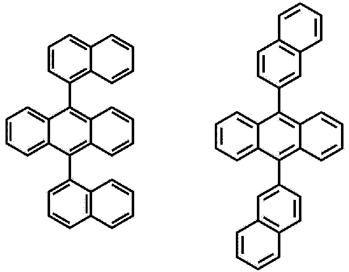

In an exemplary embodiment of the present specification, an aromatic amine derivative, a styrylamine compounds, a boron complex, a fluoranthene compound, a metal complex and the like may be used as the dopant. Specifically, the aromatic amine derivative is a fused aromatic ring derivative having a substituted or unsubstituted arylamino group, and examples thereof include a pyrene, an anthracene, a chrysene, a periflanthene, and the like, which have an arylamino group, and the styrylamine compound is a compound in which a substituted or unsubstituted arylamine is substituted with at least one arylvinyl group, and one or two or more substituents selected from the group consisting of an aryl group, a silyl group, an alkyl group, a cycloalkyl group, and an arylamino group is or are substituted or unsubstituted. Specific examples thereof include styrylamine, styryldiamine, styryltriamine, styryltetramine, and the like, but are not limited thereto. Further, examples of the metal complexes include an iridium complex, a platinum complex, and the like, but are not limited thereto. More specifically, a compound including an arylamine group may be used as the dopant.

In an exemplary embodiment of the present specification, the dopant of the light emitting layer includes a compound of the following Chemical Formula ED-1 or ED-2.

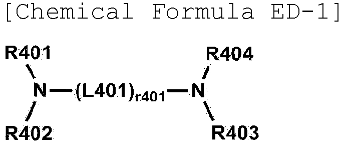

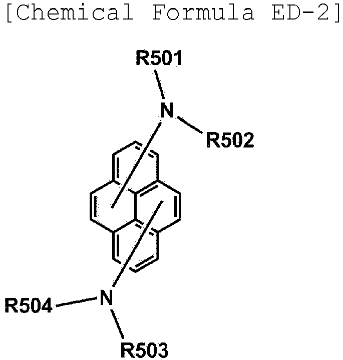

In Chemical Formulae ED-1 and ED-2,
L401 is a substituted or unsubstituted arylene group; or a substituted or unsubstituted alkenylene group,
R401 to R404 and R501 to R504 are the same as or different from each other, and are each independently hydrogen; deuterium; a substituted or unsubstituted alkyl group; a substituted or unsubstituted cycloalkyl group; a substituted or unsubstituted aryl group; or a substituted or unsubstituted heterocyclic group,
r401 is an integer from 1 to 10, and
when r401 is 2 or higher, structures in the parenthesis are the same as or different from each other.

In an exemplary embodiment of the present specification, Chemical Formula ED-2 is the following Chemical Formula ED-2-1.

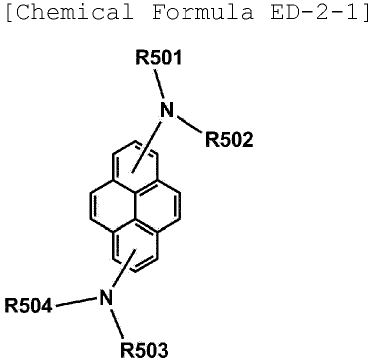

In Chemical Formula ED-2-1,
R501 to R504 are the same as those defined in Chemical Formula ED-2.

In an exemplary embodiment of the present specification, L401 is a substituted or unsubstituted arylene group having 6 to 30 carbon atoms, or a substituted or unsubstituted alkenylene group having 2 to 30 carbon atoms.

In an exemplary embodiment of the present specification, L401 is an arylene group; or an alkenylene group.

In an exemplary embodiment of the present specification,L401 is a substituted or unsubstituted phenylene group; a substituted or unsubstituted biphenylene group; a substituted or unsubstituted naphthylene group; or a substituted or unsubstituted vinylene group.

In an exemplary embodiment of the present specification, L401 is a phenylene group; a biphenylene group; a naphthylene group; or a vinylene group.

In an exemplary embodiment of the present specification, R401 to R404 and R501 to R504 are the same as or different from each other, and are each independently hydrogen; deuterium; a substituted or unsubstituted alkyl group having 1 to 30 carbon atoms; a substituted or unsubstituted cycloalkyl group having 3 to 30 carbon atoms; a substituted or unsubstituted aryl group having 6 to 30 carbon atoms; or a substituted or unsubstituted heterocyclic group having 2 to 30 carbon atoms.

In an exemplary embodiment of the present specification, R401 to R404 and R501 to R504 are the same as or different from each other, and are each independently a substituted or unsubstituted aryl group having 6 to 30 carbon atoms.

In an exemplary embodiment of the present specification, R401 to R404 and R501 to R504 are the same as or different from each other, and are each independently a substituted or unsubstituted phenyl group; a substituted or unsubstituted biphenyl group; a substituted or unsubstituted terphenyl group; or a substituted or unsubstituted naphthyl group.

In an exemplary embodiment of the present specification, R401 to R404 are the same as or different from each other, and are each independently a phenyl group which is unsubstituted or substituted with an alkyl group; a biphenyl group which is unsubstituted or substituted with an alkyl group; or a naphthyl group which is unsubstituted or substituted with an alkyl group.

In an exemplary embodiment of the present specification, R401 to R404 are the same as or different from each other, and are each independently a phenyl group which is unsubstituted or substituted with an alkyl group.

In an exemplary embodiment of the present specification, R501 to R504 are the same as or different from each other, and are each independently a phenyl group which is unsubstituted or substituted with a silyl group.

In the present specification, the silyl group is a group represented by -SiRxRyRz, and Rx, Ry and Rz are the same as or different from each other, and are each independently hydrogen; deuterium; a substituted or unsubstituted alkyl group; a substituted or unsubstituted aryl group; or a substituted or unsubstituted heterocyclic group. The number of carbon atoms of the silyl group is not particularly limited, but is preferably 1 to 60.

In an exemplary embodiment of the present specification, Chemical Formula ED-1 is the following structure.

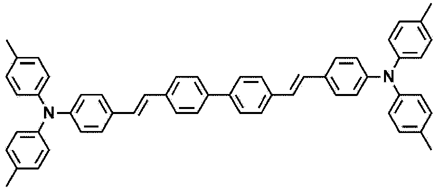

In an exemplary embodiment of the present specification, Chemical Formula ED-2 is the following structure.

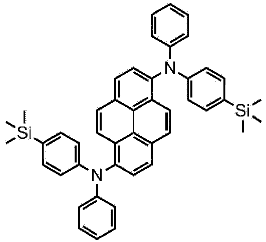

In an exemplary embodiment of the present specification, the electron transport layer is a layer that receives electrons from the electron injection layer and transports the electrons to the light emitting layer. An electron transport material is suitably a material having high electron mobility which may proficiently accept electrons from a cathode and transfer the electrons to a light emitting layer. Specific examples thereof include: Al complexes of 8-hydroxyquinoline; complexes including Alq₃; organic radical compounds; hydroxyflavone-metal complexes, and the like, but are not limited thereto. The electron transport layer may be used with any desired cathode material, as used according to the related art. In particular, appropriate examples of the cathode material are a typical material which has a low work function, followed by an aluminum layer or a silver layer. Specific examples thereof include cesium, barium, calcium, ytterbium, and samarium, in each case followed by an aluminum layer or a silver layer.

In an exemplary embodiment of the present specification, the electron injection layer is a layer that injects electrons from the electrode. An electron injection material is preferably a compound which has a capability of transporting electrons, an effect of injecting electrons from a cathode, and an excellent effect of injecting electrons into a light emitting layer or a light emitting material, prevents excitons produced from a light emitting layer from moving to a hole injection layer, and is also excellent in the ability to form a thin film. Specific examples thereof include fluorenone, anthraquinodimethane, diphenoquinone, thiopyran dioxide, oxazole, oxadiazole, triazole, imidazole, benzoimidazole, perylenetetracarboxylic acid, phenanthroline, fluorenylidene methane, anthrone, and the like and derivatives thereof, metal complex compounds, nitrogen-containing 5-membered ring derivatives, and the like, but are not limited thereto.

In an exemplary embodiment of the present specification, examples of the metal complex compounds include 8-hydroxyquinolinato lithium, bis(8-hydroxyquinolinato) zinc, bis(8-hydroxyquinolinato) copper, bis(8-hydroxyquinolinato) manganese, tris(8-hydroxyquinolinato) aluminum, tris(2-methyl-8-hydroxyquinolinato) aluminum, tris(8-hydroxyquinolinato) gallium, bis(10-hydroxybenzo[h]quinolinato) beryllium, bis(10-hydroxybenzo[h]quinolinato) zinc, bis(2-methyl-8-quinolinato) chlorogallium, bis(2-methyl-8-quinolinato) (o-cresolato) gallium, bis(2-methyl-8-quinolinato) (1-naphtholato) aluminum, bis(2-methyl-8-quinolinato) (2-naphtholato) gallium, and the like, but are not limited thereto.

In an exemplary embodiment of the present specification, the electron transport layer and electron injection layer may be formed as a layer which simultaneously transports and injects electrons, and may be expressed as an electron transport and injection layer. In this case, materials applied to the electron transport and injection layer can be both the electron transport material and electron injection material described above. For example, a benzoimidazole-based compound may be used in the electron transport and injection layer.

In an exemplary embodiment of the present specification, the electron transport and injection layer includes a compound of the following Chemical Formula ET-1 or ET-2.

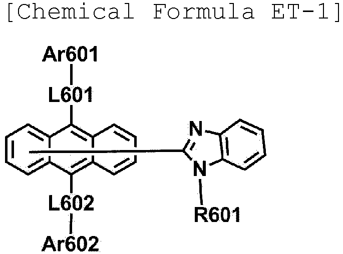

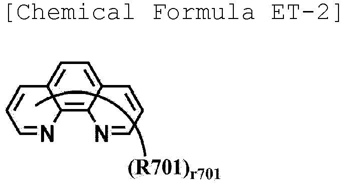

In Chemical Formulae ET-1 and ET-2,
L601 and L602 are the same as or different from each other, and are each independently a direct bond; a substituted or unsubstituted arylene group; or a substituted or unsubstituted divalent heterocyclic group,
Ar601 and Ar602 are the same as or different from each other, and are each independently hydrogen; deuterium; a substituted or unsubstituted aryl group; or a substituted or unsubstituted heterocyclic group,
R601 and R701 are the same as or different from each other, and are each independently hydrogen; deuterium; a halogen group; a substituted or unsubstituted alkyl group; a substituted or unsubstituted cycloalkyl group; a substituted or unsubstituted aryl group; or a substituted or unsubstituted heterocyclic group, and
r701 is an integer from 1 to 8, and when r701 is 2 or higher, two or more R701's are the same as or different from each other.

In an exemplary embodiment of the present specification, L601 and L602 are the same as or different from each other, and are each independently a direct bond; a substituted or unsubstituted monocyclic arylene group; or a substituted or unsubstituted polycyclic arylene group.

In an exemplary embodiment of the present specification, L601 and L602 are the same as or different from each other, and are each independently a direct bond; a substituted or unsubstituted phenylene group; a substituted or unsubstituted biphenylylene group; or a substituted or unsubstituted naphthylene group.

In an exemplary embodiment of the present specification, L601 and L602 are each a direct bond.

In an exemplary embodiment of the present specification, Ar601 and Ar602 are the same as or different from each other, and are each independently a substituted or unsubstituted aryl group.

In an exemplary embodiment of the present specification, Ar601 and Ar602 are the same as or different from each other, and are each independently a substituted or unsubstituted monocyclic aryl group; or a substituted or unsubstituted polycyclic aryl group.

In an exemplary embodiment of the present specification, Ar601 and Ar602 are the same as or different from each other, and are each independently a substituted or unsubstituted phenyl group; a substituted or unsubstituted biphenyl group; a substituted or unsubstituted terphenyl group; a substituted or unsubstituted naphthyl group; a substituted or unsubstituted anthracenyl group; a substituted or unsubstituted phenanthrenyl group; a substituted or unsubstituted triphenylenyl group; a substituted or unsubstituted pyrenyl group; or a substituted or unsubstituted fluorenyl group.

In an exemplary embodiment of the present specification, Ar601 and Ar602 are the same as or different from each other, and are each independently a substituted or unsubstituted phenyl group; or a substituted or unsubstituted naphthyl group.

In an exemplary embodiment of the present specification, Ar601 and Ar602 are each a naphthyl group.

In an exemplary embodiment of the present specification, R601 and R701 are the same as or different from each other, and are each independently hydrogen; deuterium; a substituted or unsubstituted alkyl group having 1 to 30 carbon atoms; or a substituted or unsubstituted aryl group having 6 to 30 carbon atoms.

In an exemplary embodiment of the present specification, R601 is a substituted or unsubstituted aryl group.

In an exemplary embodiment of the present specification, R601 is a substituted or unsubstituted phenyl group.

In an exemplary embodiment of the present specification, R601 is a phenyl group.

In an exemplary embodiment of the present specification, R701 is hydrogen; deuterium; an alkyl group; or an aryl group.

In an exemplary embodiment of the present specification, R701 is hydrogen; deuterium; a substituted or unsubstituted methyl group; a substituted or unsubstituted ethyl group; a substituted or unsubstituted propyl group; a substituted or unsubstituted butyl group; a substituted or unsubstituted phenyl group; a substituted or unsubstituted naphthyl group; or a substituted or unsubstituted biphenyl group.

In an exemplary embodiment of the present specification, R701 is hydrogen; deuterium; a methyl group; an ethyl group; a propyl group; a butyl group; a phenyl group; a naphthyl group; or a biphenyl group.

In an exemplary embodiment of the present specification, R701 is hydrogen; a methyl group; or a phenyl group.

In an exemplary embodiment of the present specification, Chemical Formula ET-1 is the following structure.

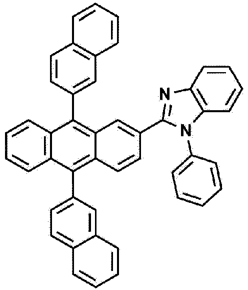

In an exemplary embodiment of the present specification, Chemical Formula ET-2 is the following structure.

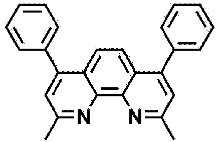

In an exemplary embodiment of the present specification, the hole blocking layer is a layer which blocks holes from reaching a cathode, and may be generally formed under the same conditions as those of the hole injection layer. Specific examples of the hole blocking material include oxadiazole derivatives or triazole derivatives, phenanthroline derivatives, BCP, aluminum complexes, and the like, but are not limited thereto.

In an exemplary embodiment of the present specification, the electron blocking layer is a layer which blocks electrons from reaching an anode, and materials known in the art may be used.

The organic light emitting device according to the present specification may be a top emission type, a bottom emission type, or a dual emission type according to the materials to be used.

### [Mode for Invention]

Hereinafter, the present specification will be described in detail with reference to Examples for specifically describing the present specification. However, the Examples according to the present specification may be modified in various forms, and it is not interpreted that the scope of the present specification is limited to the Examples described below. The Examples of the present specification are provided to more completely explain the present specification to a person with ordinary skill in the art.

### <Synthesis Examples>

### Synthesis Example 1. Synthesis of Compound 1

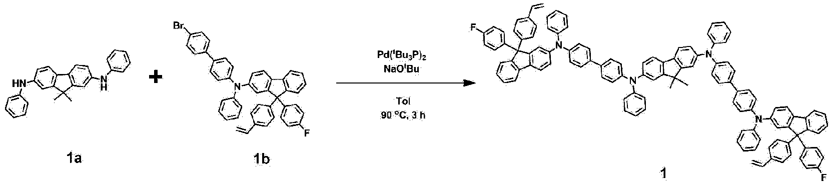

Compound 1a (1.0 equiv.), Compound 1b (2.2 equiv.), sodium tert-butoxide (NaO^{t}Bu) (3.0 equiv.) were put into a round bottom flask (RBF), and then toluene (Tol) (0.1 M ) was added thereto. After the temperature was increased to 90°C, bis(tri-tert-butylphosphine)palladium(0) (Pd(^{t}Bu₃P)₂) (0.05 equiv.) was added thereto, and the resulting mixture was stirred for 3 hours. Water was added thereto, and an organic layer was extracted with dichloromethane (DCM), and then dried over magnesium sulfate (MgSO₄), and column purified with dichloromethane/hexane to obtain Compound 1. [M+H]⁺=1584

### Synthesis Example 2. Synthesis of Compound 2

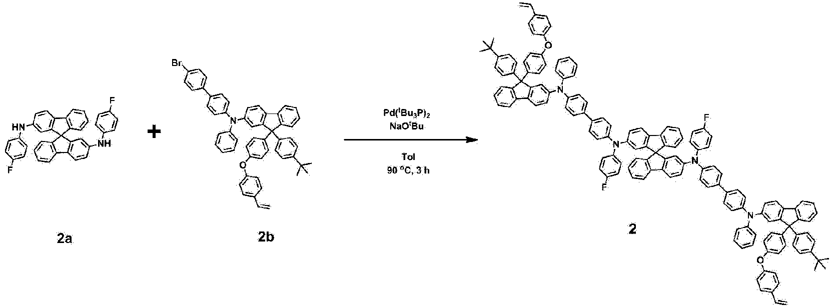

Compound 2 was synthesized in the same manner as in Synthesis Example 1, except that Compound 2a and Compound 2b were used instead of Compound 1a and Compound 1b, respectively, in Synthesis Example 1. [M+H]⁺=2002

### Synthesis Example 3. Synthesis of Compound 3

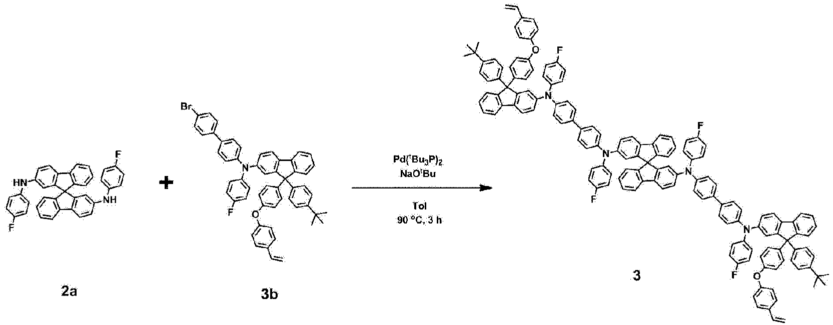

Compound 3 was synthesized in the same manner as in Synthesis Example 1, except that Compound 2a and Compound 3b were used instead of Compound 1a and Compound 1b, respectively, in Synthesis Example 1. [M+H]⁺=2038

### Synthesis Example 4. Synthesis of Compound 4

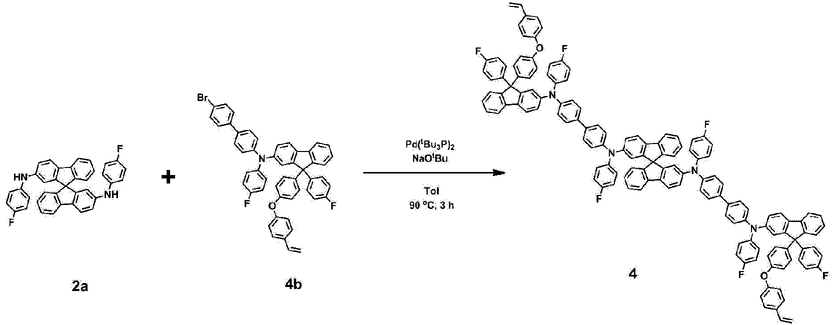

Compound 4 was synthesized in the same manner as in Synthesis Example 1, except that Compound 2a and Compound 4b were used instead of Compound 1a and Compound 1b, respectively, in Synthesis Example 1. [M+H]⁺=1962

### Synthesis Example 5. Synthesis of Compound 5

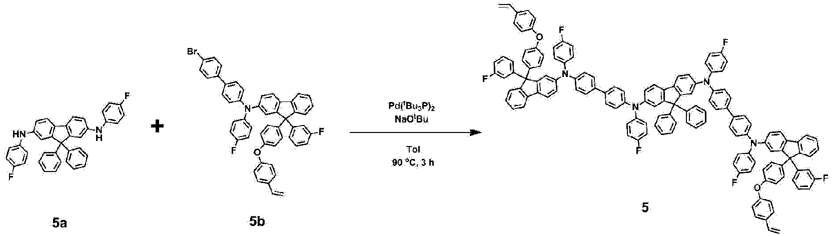

Compound 5 was synthesized in the same manner as in Synthesis Example 1, except that Compound 5a and Compound 5b were used instead of Compound 1a and Compound 1b, respectively, in Synthesis Example 1. [M+H]⁺=1964

### Synthesis Example 6. Synthesis of Compound 6

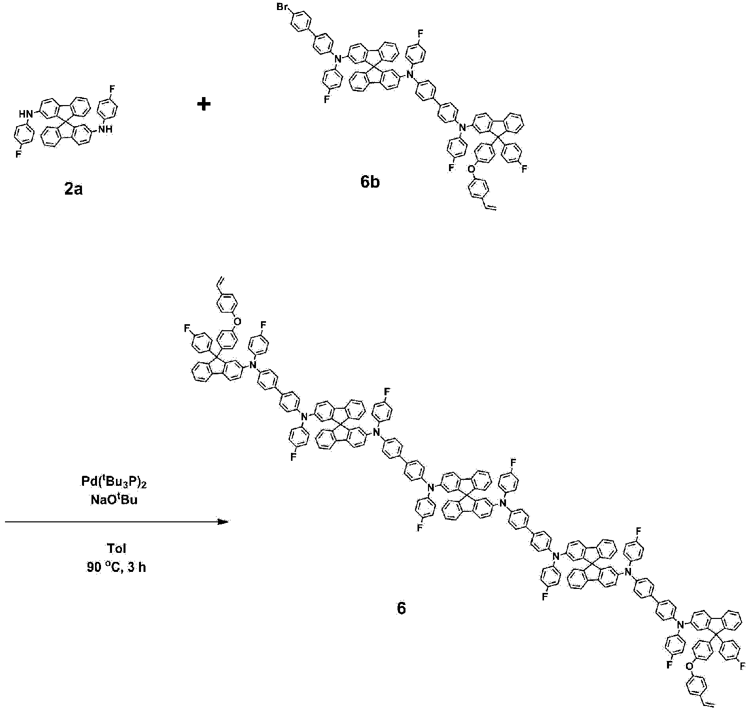

Compound 6 was synthesized in the same manner as in Synthesis Example 1, except that Compound 2a and Compound 6b were used instead of Compound 1a and Compound 1b, respectively, in Synthesis Example 1. [M+H]⁺=3330

### Synthesis Example 7. Synthesis of Compound 7

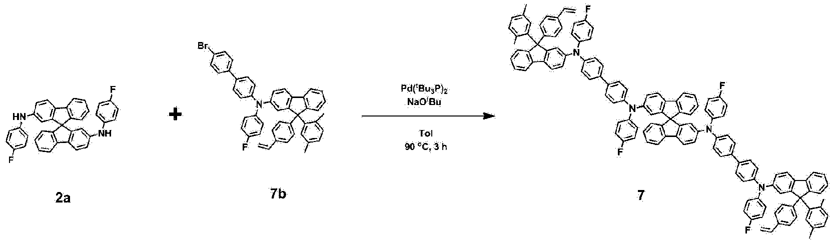

Compound 7 was synthesized in the same manner as in Synthesis Example 1, except that Compound 2a and Compound 7b were used instead of Compound 1a and Compound 1b, respectively, in Synthesis Example 1. [M+H]⁺=1798

### Synthesis Example 8. Synthesis of Compound 8

### (1) Synthesis of Intermediate 8-1

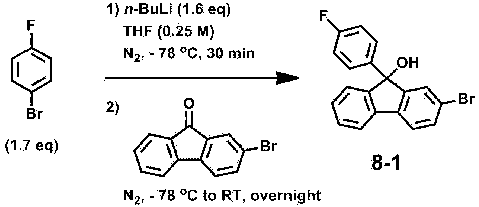

1-bromo-4-fluorobenzene (27.9 mL, 255 mmol, 1.7 eq) was put into tetrahydrofuran (THF) (500 mL). After substitution with nitrogen, the mixture was cooled to -78°C. n-Butyllithium (n-BuLi) (2.5 M in Hex) (96 mL, 240 mmol, 1.6 eq) was put into a dropping funnel, and then slowly added to the reaction mixture, and then the resulting mixture was stirred at -78°C for 30 minutes. 2-Bromofluorenone (38.9 g, 150 mmol) was added thereto. The resulting mixture was stirred overnight while slowly increasing the temperature to room temperature (RT). After the reaction was terminated by adding distilled water thereto, extraction was performed with ethyl acetate and water. After an organic layer was collected, the organic layer was dried using magnesium sulfate (MgSO₄) and filtered. Intermediate 8-1 was obtained by drying the filtrate using a vacuum rotary concentrator to remove the organic solvent.

### (2) Synthesis of Intermediate 8-2

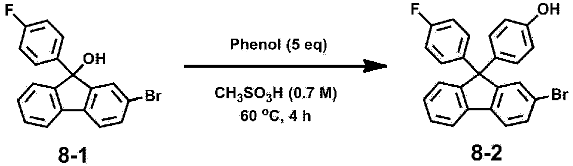

Intermediate 8-1 (53 g, 150 mmol), phenol (70.6 g, 750 mmol, 5 eq) were put into a round bottom flask (RBF). After methanesulfonic acid (CH₃SO₃H) (214 mL, 0.7 M) was added thereto, the resulting mixture was stirred at 60°C for 4 hours. After ice water was added thereto, extraction was performed with ethyl acetate and water. After an organic layer was collected, the organic layer was dried using MgSO₄ and filtered. The filtrate was dried using a vacuum rotary concentrator to remove the organic solvent. After column purification, crystallization was performed under dichloromethane/heptane conditions to obtain 40.6 g of Intermediate 8-2.

### (3) Synthesis of Intermediate 8-3

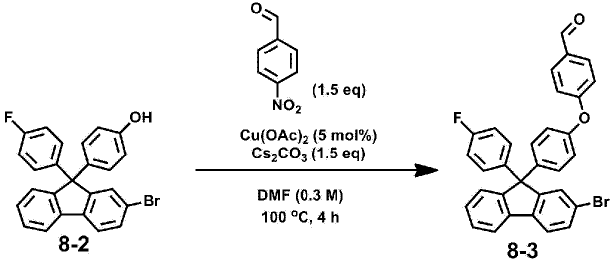

Intermediate 8-2 (40 g, 92.7 mmol), 4-nitrobenzaldehyde (21.2 g, 139 mmol, 1.5 eq), copper(II)acetate (Cu(OAc)₂) (842 mg, 4.64 mmol, 5 mol%), and cesium carbonate (Cs₂CO₃) (45.3 g, 139 mmol, 1.5 eq) were put into RBF. Dimethylformamide (DMF) (310 mL) was added thereto, and the resulting mixture was stirred at 100°C for 4 hours. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/heptane conditions to obtain 38.7 g of Intermediate 8-3.

### (4) Synthesis of Intermediate 8-4

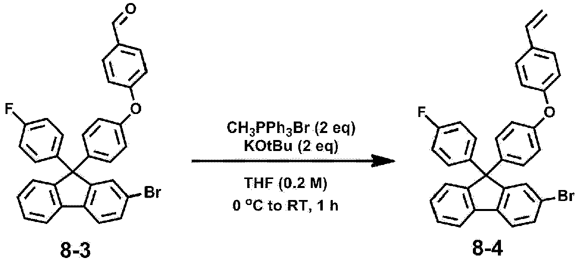

After methyltriphenylphosphonium bromide (CH₃PPh₃Br) (51.6 g, 144.6 mmol, 2 eq), potassium tertiary-butoxide (KOtBu) (16.2 g, 144.6 mmol, 2 eq), and THF (217 mL) were put into RBF, the resulting mixture was cooled to 0°C. A solution of Intermediate 8-3 (38.7 g, 72.3 mmol) in tetrahydrofuran (THF) (144 mL) was added to the reaction mixture. The resulting mixture was stirred for 1 hour while being warmed to room temperature. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under dichloromethane/ethanol (DCM/EtOH) conditions to obtain 33.7 g of Intermediate 8-4.

### (5) Synthesis of Intermediate 8-5

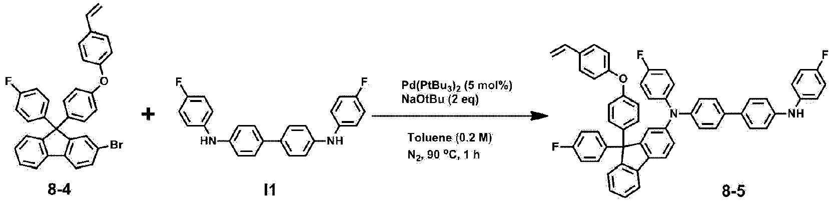

Intermediate 8-4 (10.7 g, 20 mmol), Compound I1 (14.9 g, 40 mmol), bis(tri-tert-butylphosphine) palladium (0) (Pd(PtBu₃)₂) (511 mg, 1 mmol, 5 mol%) and sodium tert-butoxide (NaOtBu) (3.84 g, 40 mmol, 2 eq) were put into RBF. After substitution with nitrogen, toluene (100 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 8.3 g of Intermediate 8-5.

### (6) Synthesis of Compound 8

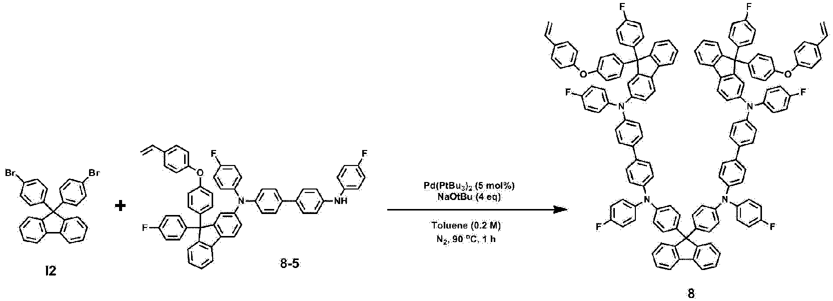

Compound I2 (1.19 g, 2.5 mmol), Intermediate 8-5 (4.54 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 2.9 g of Compound 8. It was confirmed through LC-MS and NMR that Compound 8 was synthesized. MS: [M+H]+ = 1964

NMR measurement value of Compound 8: ¹H NMR (500 MHz, DMSO-d6) δ 7.90 (d, 2H), 7.80 (d, 4H), 7.46 - 7.30 (m, 20H), 7.30 - 7.22 (m, 4H), 7.13 - 7.01 (m, 34H), 6.97 - 6.83 (m, 22H), 6.61 (m, 2H), 5.65 (d, 2H), 5.11 (d, 2H)

### Synthesis Example 9. Synthesis of Compound 9

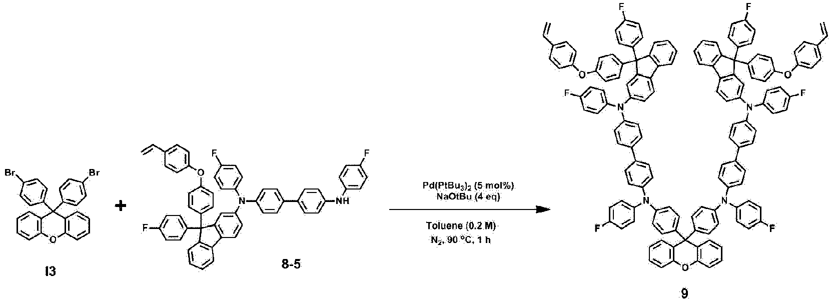

Compound I3 (1.23 g, 2.5 mmol), Intermediate 8-5 (4.54 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 3.21 g of Compound 9. It was confirmed through LC-MS and NMR that Compound 9 was synthesized. MS: [M+H]+ = 1981

NMR measurement value of Compound 9: ¹H NMR (500 MHz, DMSO-d6) δ 7.80 (d, 4H), 7.46 - 7.30 (m, 20H), 7.30 - 7.17 (m, 6H), 7.13 - 7.01 (m, 34H), 6.97 - 6.83 (m, 22H), 6.61 (m, 2H), 5.65 (d, 2H), 5.11 (d, 2H)

### Synthesis Example 10. Synthesis of Compound 10

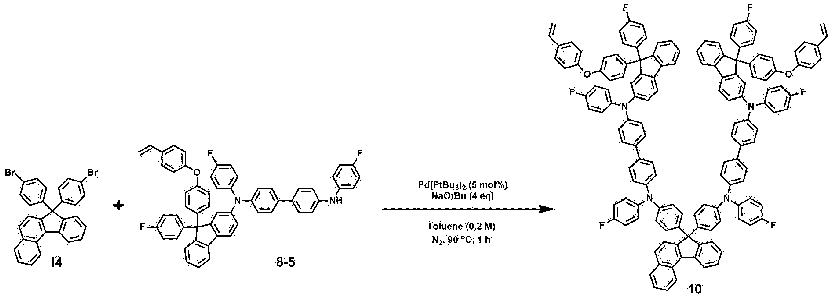

Compound I4 (1.32 g, 2.5 mmol), Intermediate 8-5 (4.54 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 3.3 g of Compound 10. It was confirmed through LC-MS and NMR that Compound 10 was synthesized. MS: [M+H]+ = 2015

NMR measurement value of Compound 10: ¹H NMR (500 MHz, DMSO-d6) δ 8.90 (d, 1H), 8.25 (d, 1H), 8.09 (d, 1H), 7.90 - 7.80 (m, 5H), 7.46 - 7.30 (m, 20H), 7.30 - 7.22 (m, 4H), 7.13 - 7.01 (m, 34H), 6.97 - 6.83 (m, 22H), 6.61 (m, 2H), 5.65 (d, 2H), 5.11 (d, 2H)

### Synthesis Example 11. Synthesis of Compound 11

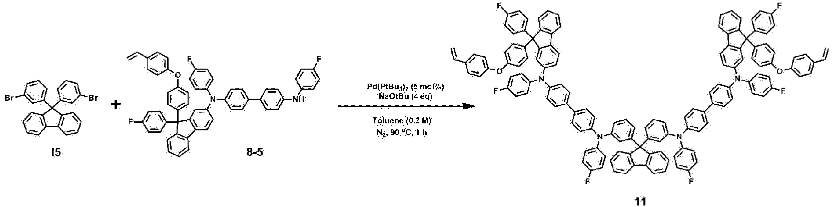

Compound I5 (1.19 g, 2.5 mmol), Intermediate 8-5 (4.54 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 3.0 g of Compound 11. It was confirmed through LC-MS and NMR that Compound 11 was synthesized. MS: [M+H]+ = 1964

NMR measurement value of Compound 11: ¹H NMR (500 MHz, DMSO-d6) δ 7.90 (d, 2H), 7.80 (d, 4H), 7.46 - 7.30 (m, 20H), 7.30 - 7.22 (m, 4H), 7.19 - 7.01 (m, 34H), 6.97 - 6.83 (m, 22H), 6.61 (m, 2H), 5.65 (d, 2H), 5.11 (d, 2H)

### Synthesis Example 12. Synthesis of Compound 12

### (1) Synthesis of Intermediate 12-1

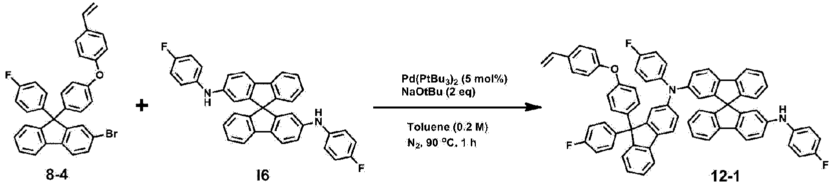

Intermediate 8-4 (10.7 g, 20 mmol), Compound I6 (21.4 g, 40 mmol), bis(tri-tert-butylphosphine) palladium (0) (Pd(PtBu₃)₂) (511 mg, 1 mmol, 5 mol%) and sodium tert-butoxide (NaOtBu) (3.84 g, 40 mmol, 2 eq) were put into RBF. After substitution with nitrogen, toluene (100 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 10.4 g of Intermediate 12-1.

### (2) Synthesis of Compound 12

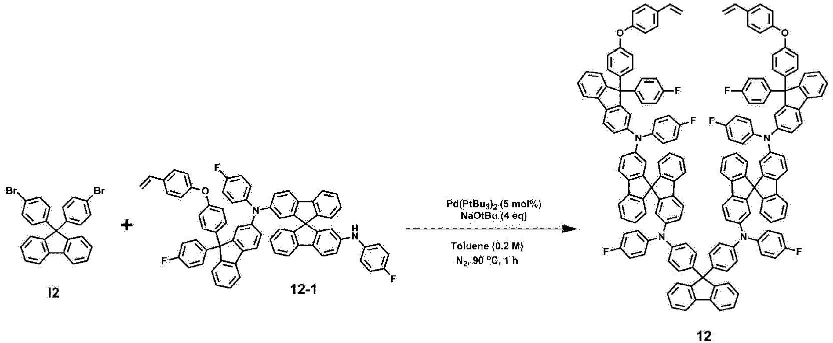

Compound I2 (1.19 g, 2.5 mmol), Intermediate 12-1 (5.43 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 3.7 g of Compound 12. It was confirmed through LC-MS and NMR that Compound 12 was synthesized. MS: [M+H]+ = 2288

NMR measurement value of Compound 12: ¹H NMR (500 MHz, DMSO-d6) δ 7.90 - 7.80 (m, 14H), 7.60 - 7.52 (m, 8H), 7.48 - 7.22 (m, 30H), 7.18 - 6.95 (m, 42H), 6.86 (d, 4H), 6.63 (m, 2H), 5.65 (d, 2H), 5.11 (d, 2H)

### Synthesis Example 13. Synthesis of Compound 13

### (1) Synthesis of Intermediate 13-1

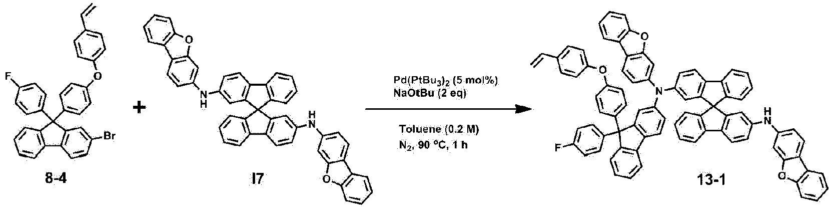

Intermediate 8-4 (10.7 g, 20 mmol), Compound I7 (27.2 g, 40 mmol), bis(tri-tert-butylphosphine) palladium (0) (Pd(PtBu₃)₂) (511 mg, 1 mmol, 5 mol%) and sodium tert-butoxide (NaOtBu) (3.84 g, 40 mmol, 2 eq) were put into RBF. After substitution with nitrogen, toluene (100 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 11 g of Intermediate 13-1.

### (2) Synthesis of Compound 13

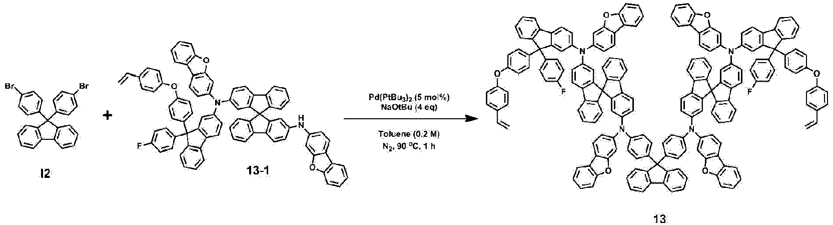

Compound I2 (1.19 g, 2.5 mmol), Intermediate 13-1 (6.22 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 4.0 g of Compound 13. It was confirmed through LC-MS and NMR that Compound 13 was synthesized. MS: [M+H]+ = 2578

NMR measurement value of Compound 13: ¹H NMR (500 MHz, DMSO-d6) δ 7.93 (s, 4H), δ 7.90 - 7.80 (m, 18H), 7.72 (d, 4H), 7.60 - 7.52 (m, 8H), 7.48-7.22 (m, 42H), 7.18 - 6.95 (m, 26H), 6.86 - 6.80 (m, 8H), 6.63 (m, 2H), 5.65 (d, 2H), 5.11 (d, 2H)

### Synthesis Example 14. Synthesis of Compound 14

### (1) Synthesis of Intermediate 14-1

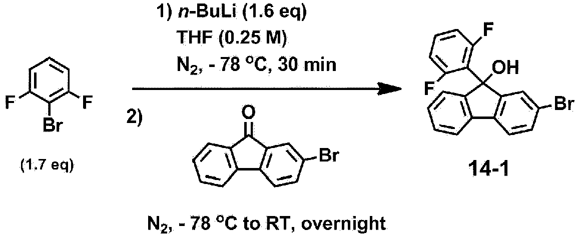

Intermediate 14-1 was prepared in the same manner as in (1) of Synthesis Example 8, except that 1-bromo-2,6-difluorobenzene (29.4 mL, 255 mmol, 1.7 eq) was used instead of 1-bromo-4-fluorobenzene in (1) of Synthesis Example 8.

### (2) Synthesis of Intermediate 14-2

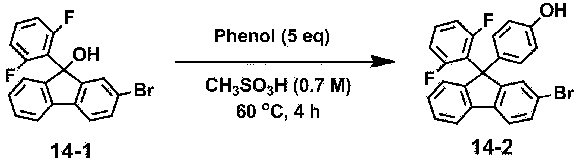

45.2 g of Intermediate 14-2 was obtained by preparation in the same manner as in (2) of Synthesis Example 8, except that Intermediate 14-1 (56 g, 150 mmol) was used instead of Intermediate 8-1 in (2) of Synthesis Example 8.

### (3) Synthesis of Intermediate 14-3

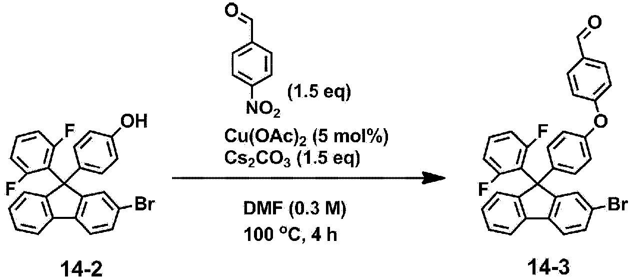

39.8 g of Intermediate 14-3 was obtained by preparation in the same manner as in (3) of Synthesis Example 8, except that Intermediate 14-2 (45.2 g, 100 mmol) was used instead of Intermediate 8-2 in (3) of Synthesis Example 8.

### (4) Synthesis of Intermediate 14-4

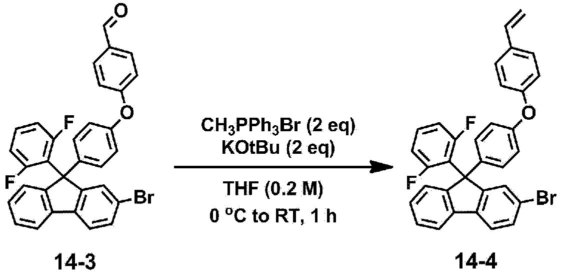

34.8 g of Intermediate 14-4 was obtained by preparation in the same manner as in (4) of Synthesis Example 8, except that Intermediate 14-3 (39.8 g, 72 mmol) was used instead of Intermediate 8-3 in (4) of Synthesis Example 8.

### (5) Synthesis of Intermediate 14-5

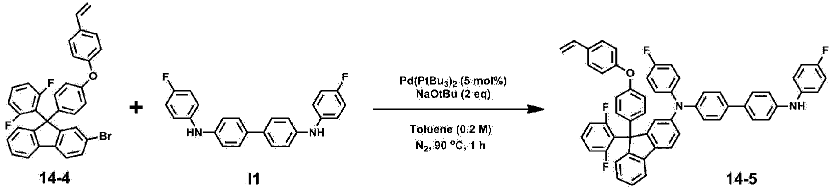

Intermediate 14-4 (11.0 g, 20 mmol), Compound I1 (14.9 g, 40 mmol), bis(tri-tert-butylphosphine) palladium (0) (Pd(PtBu₃)₂) (511 mg, 1 mmol, 5 mol%) and sodium tert-butoxide (NaOtBu) (3.84 g, 40 mmol, 2 eq) were put into RBF. After substitution with nitrogen, toluene (100 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 8.6 g of Compound 14-5.

### (6) Synthesis of Compound 14

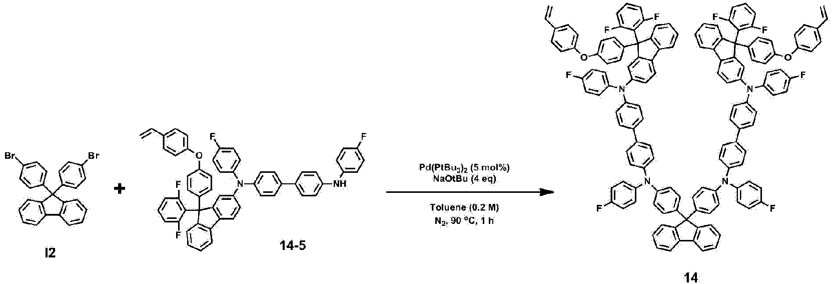

Compound I2 (1.19 g, 2.5 mmol), Intermediate 14-5 (4.64 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 3.6 g of Compound 14. It was confirmed through LC-MS and NMR that Compound 14 was synthesized. MS: [M+H]+ = 2001

NMR measurement value of Compound 14: ¹H NMR (500 MHz, DMSO-d6) δ 7.90 (d, 2H), 7.80 (d, 4H), 7.67 (t, 2H), 7.46 - 7.31 (m, 24H), 7.30 - 7.22 (m, 4H), 7.13 - 7.01 (m, 26H), 6.97 - 6.83 (m, 22H), 6.61 (m, 2H), 5.65 (d, 2H), 5.11 (d, 2H)

### Synthesis Example 15. Synthesis of Compound 15

### (1) Synthesis of Intermediate 15-1

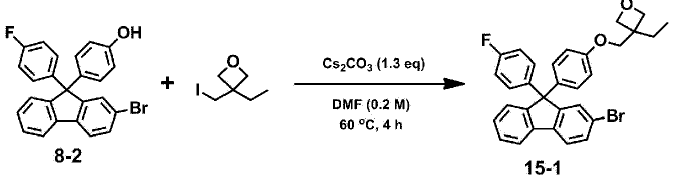

Intermediate 8-2 (8.63 g, 20 mmol) and Cs₂CO₃ (8.47 g, 26 mmol, 1.3 eq) were put into RBF. DMF (100 mL) and 3-ethyl-3-iodomethyloxetane (4 mL, 26 mmol) were added thereto, and the resulting mixture was stirred at 60°C for 4 hours. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/heptane conditions to obtain 12.3 g of Intermediate 15-1.

### (2) Synthesis of Intermediate 15-2

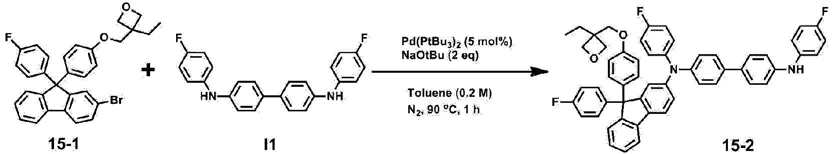

Intermediate 15-1 (10.6 g, 20 mmol), Compound I1 (14.9 g, 40 mmol), bis(tri-tert-butylphosphine) palladium (0) (Pd(PtBu₃)₂) (511 mg, 1 mmol, 5 mol%) and sodium tert-butoxide (NaOtBu) (3.84 g, 40 mmol, 2 eq) were put into RBF. After substitution with nitrogen, toluene (100 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 11.2 g of Intermediate 15-2.

### (3) Synthesis of Compound 15

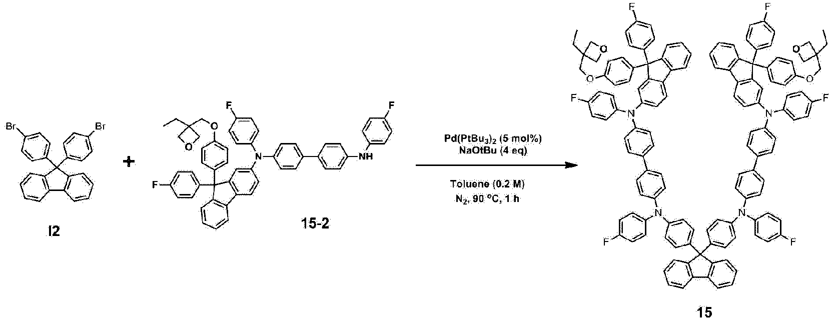

Compound I2 (1.19 g, 2.5 mmol), Intermediate 15-2 (4.52 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 3.6 g of Compound 15. It was confirmed through LC-MS and NMR that Compound 15 was synthesized. MS: [M+H]+ = 1957

NMR measurement value of Compound 15: ¹H NMR (500 MHz, DMSO-d6) δ 7.90 (d, 2H), 7.80 (d, 4H), 7.46 - 7.31 (m, 20H), 7.29 - 7.22 (m, 4H), 7.13 - 7.01 (m, 34H), 6.97 - 6.83 (m, 22H), 4.37 (d, 4H), 4.28 (d, 4H), 4.00 (s, 4H), 1.71 (q, 4H), 0.82 (t, 6H)

### Synthesis Example 16. Synthesis of Compound 16

### (1) Synthesis of Intermediate 16-1

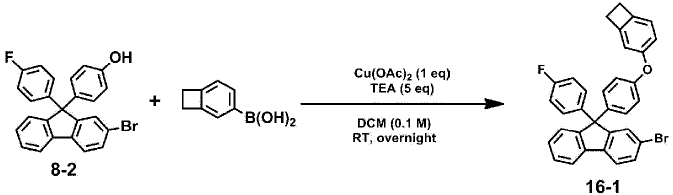

Intermediate 8-2 (39 g, 90 mmol), benzocyclobutane-4-boronic acid (20 g, 135 mmol), Cu(OAc)₂ (16.4 g, 90 mmol, 1 eq), and 4 Å molecular sieve (90 g) were put into RBF. DCM (900 mL) and triethylamine (TEA) (63 mL, 450 mmol, 5 eq) were added thereto, and the resulting mixture was stirred at room temperature overnight. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 15 g of Intermediate 16-1.

### (2) Synthesis of Intermediate 16-2

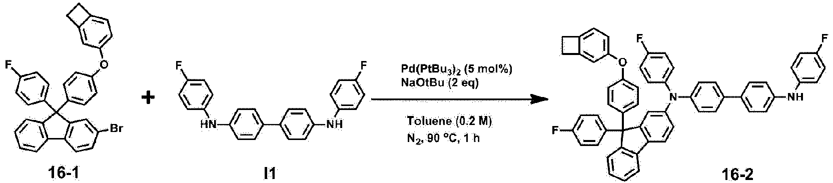

Intermediate 16-1 (10.7 g, 20 mmol), Compound I1 (14.9 g, 40 mmol), bis(tri-tert-butylphosphine) palladium (0) (Pd(PtBu₃)₂) (511 mg, 1 mmol, 5 mol%) and sodium tert-butoxide (NaOtBu) (3.84 g, 40 mmol, 2 eq) were put into RBF. After substitution with nitrogen, toluene (100 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 8.7 g of Intermediate 16-2.

### (3) Synthesis of Compound 16

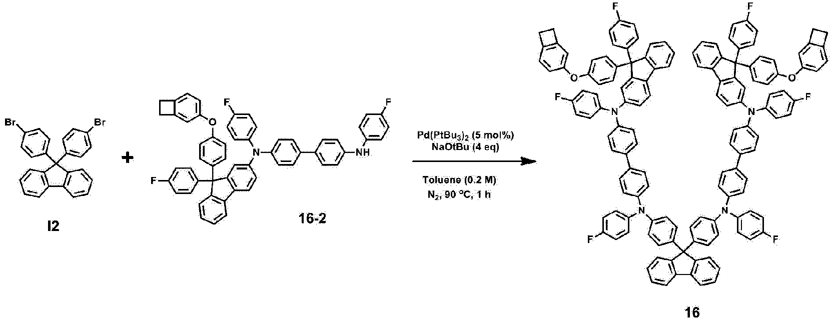

Compound I2 (1.19 g, 2.5 mmol), Intermediate 16-2 (4.54 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 2.9 g of Compound 16. It was confirmed through LC-MS and NMR that Compound 16 was synthesized. MS: [M+H]+ = 1964

NMR measurement value of Compound 16: ¹H NMR (500 MHz, DMSO-d6) δ 7.90 (d, 2H), 7.80 (d, 4H), 7.46 - 7.31 (m, 18H), 7.30 - 7.22 (m, 4H), 7.13 - 7.01 (m, 32H), 6.97 - 6.83 (m, 24H), 3.00 (m, 8H)

### Synthesis Example 17. Synthesis of Compound 17

### 1) Synthesis of Intermediate 17-1

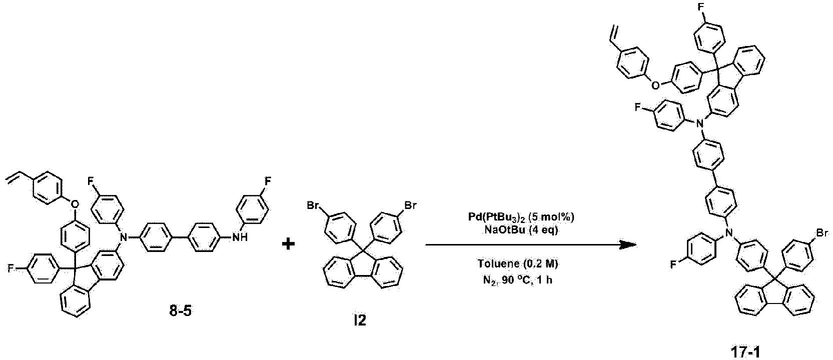

Intermediate 8-5 (8.25 g, 10 mmol), Compound I2 (9.52 g, 20 mmol), bis(tri-tert-butylphosphine)palladium(0) (Pd(PtBu₃)₂) (256 mg, 0.5 mmol, 5 mol%) and sodium tert-butoxide (NaOtBu) (3.84 g, 40 mmol, 4 eq) were put into RBF. After substitution with nitrogen, toluene (50 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 7.2 g of Intermediate 17-1.

### (2) Synthesis of Compound 17

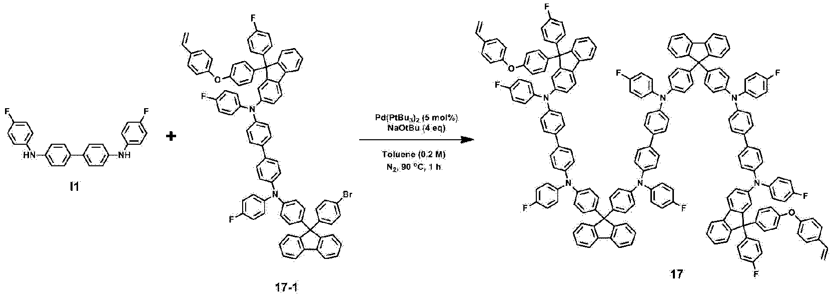

Compound I1 (931 mg, 2.5 mmol), Intermediate 17-1 (6.71 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 5 g of Compound 17. It was confirmed through LC-MS and NMR that Compound 17 was synthesized. MS: [M+H]+ = 2650

NMR measurement value of Compound 17: ¹H NMR (500 MHz, DMSO-d6) δ 7.90 (d, 4H), 7.80 (d, 4H), 7.46 - 7.18 (m, 52H), 7.13 - 6.98 (m, 50H), 6.75 (d, 8H), 6.61 (m, 2H), 5.65 (d, 2H), 5.11 (d, 2H)

### Synthesis Example 18. Synthesis of Compound 18

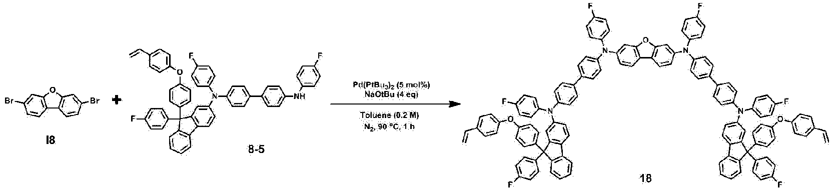

Compound I8 (815 mg, 2.5 mmol), Intermediate 8-5 (4.54 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 2.7 g of Compound 18. It was confirmed through LC-MS and NMR that Compound 18 was synthesized. MS: [M+H]+ = 1814

NMR measurement value of Compound 18: ¹H NMR (500 MHz, DMSO-d6) δ 7.93 (d, 2H), 7.80 - 7.72 (m, 4H), 7.69 (d, 2H), 7.60 - 7.48 (m, 14H), 7.44 - 7.25 (m, 18H), 7.10 - 6.96 (m, 34H), 6.82 (m, 2H), 6.64 (m, 2H), 5.66 (d, 2H), 5.14 (d, 2H)

### Synthesis Example 19. Synthesis of Compound 19

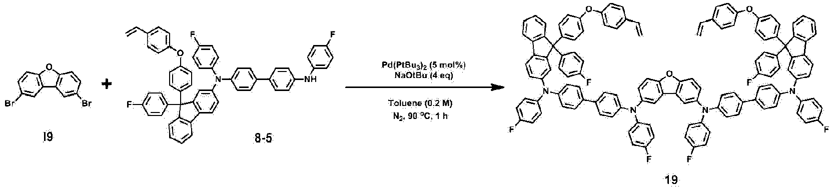

Compound I9 (815 mg, 2.5 mmol), Intermediate 8-5 (4.54 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 2.8 g of Compound 19. It was confirmed through LC-MS and NMR that Compound 19 was synthesized. MS: [M+H]+ = 1814

NMR measurement value of Compound 19: ¹H NMR (500 MHz, DMSO-d6) δ 8.10 (d, 2H), 7.80 - 7.72 (m, 4H), 7.60 - 7.43 (m, 16H), 7.43 - 7.25 (m, 18H), 7.10 - 6.87 (m, 36H), 6.64 (m, 2H), 5.66 (d, 2H), 5.14 (d, 2H)

### Synthesis Example 20. Synthesis of Compound 20

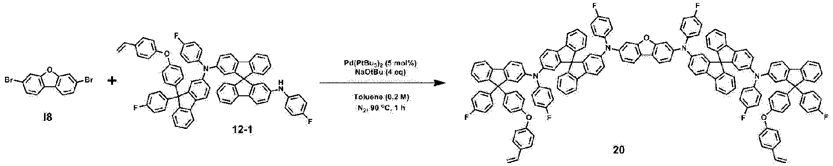

Compound I8 (815 mg, 2.5 mmol), Intermediate 12-1 (5.43 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 3.4 g of Compound 20. It was confirmed through LC-MS and NMR that Compound 20 was synthesized. MS: [M+H]+ = 2138

NMR measurement value of Compound 20: ¹H NMR (500 MHz, DMSO-d6) δ 7.94 (d, 2H), 7.80 - 7.70 (m, 14H), 7.52 - 7.43 (m, 8H), 7.38 -6.96 (m, 62H), 6.82 (m, 2H), 6.63 (m, 2H), 5.66 (d, 2H), 5.14 (d, 2H)

### Synthesis Example 21. Synthesis of Compound 21

### (1) Synthesis of Intermediate 21-1

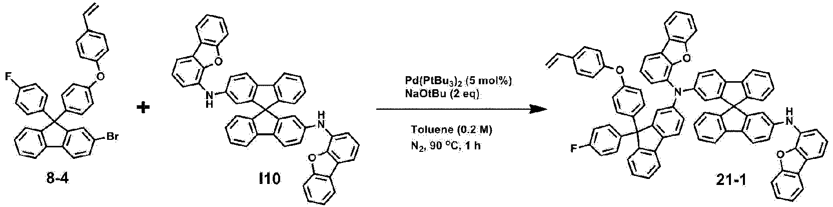

Intermediate 8-4 (10.7 g, 20 mmol), Compound I10 (27.2 g, 40 mmol), bis(tri-tert-butylphosphine) palladium (0) (Pd(PtBu₃)₂) (511 mg, 1 mmol, 5 mol%) and sodium tert-butoxide (NaOtBu) (3.84 g, 40 mmol, 2 eq) were put into RBF. After substitution with nitrogen, toluene (100 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 11 g of Intermediate 21-1.

### (2) Synthesis of Compound 21

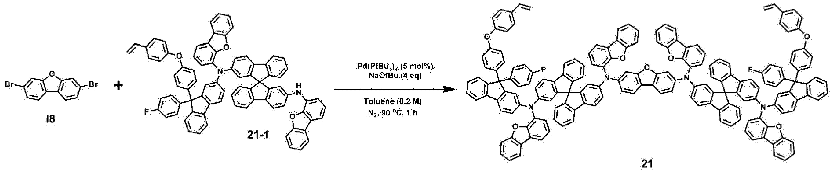

Compound I8 (815 mg, 2.5 mmol), Intermediate 21-1 (6.22 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 3.7 g of Compound 21. It was confirmed through LC-MS and NMR that Compound 21 was synthesized. MS: [M+H]+ = 2426

NMR measurement value of Compound 21: ¹H NMR (500 MHz, DMSO-d6) δ 7.93 - 7.85 (m, 6H), 7.80 - 7.70 (m, 14H), 7.54 - 7.43 (m, 16H), 7.38 -7.06 (m, 52H), 6.94 - 6.82 (m, 12H), 6.62 (m, 2H), 5.66 (d, 2H), 5.14 (d, 2H)

### Synthesis Example 22. Synthesis of Compound 22

### (1) Synthesis of Intermediate 22-1

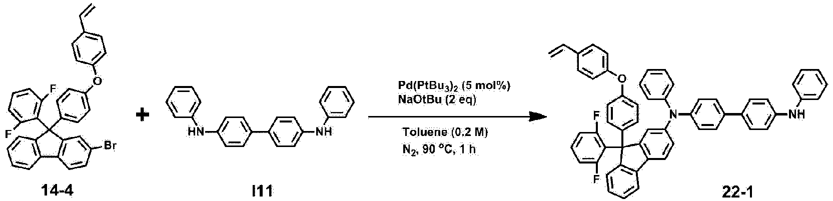

Intermediate 14-4 (11.0 g, 20 mmol), Compound I11 (13.5 g, 40 mmol), bis(tri-tert-butylphosphine)palladium(0) (Pd(PtBu₃)₂) (511 mg, 1 mmol, 5 mol%) and sodium tert-butoxide (NaOtBu) (3.84 g, 40 mmol, 2 eq) were put into RBF. After substitution with nitrogen, toluene (100 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 9.2 g of Intermediate 22-1.

### (2) Synthesis of Compound 22

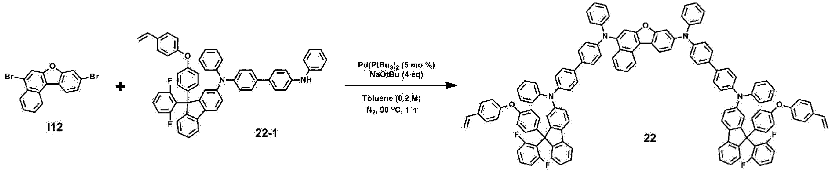

Compound I12 (940 mg, 2.5 mmol), Intermediate 22-1 (4.44 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 2.6 g of Compound 22. It was confirmed through LC-MS and NMR that Compound 22 was synthesized. MS: [M+H]+ = 1828

NMR measurement value of Compound 22: ¹H NMR (500 MHz, DMSO-d6) δ 8.85 (d, 1H), 8.02 (d, 1H), 7.95 (m, 2H), 7.80 - 7.70 (m, 5H), 7.58 - 7.43 (m, 18H), 7.32 - 6.98 (m, 52H), 6.83 (s, 1H), 6.62 (m, 2H), 5.66 (d, 2H), 5.15 (d, 2H)

### Synthesis Example 23. Synthesis of Compound 23

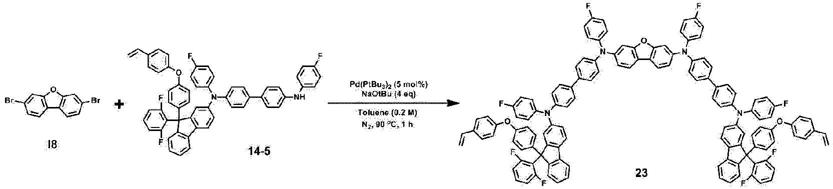

Compound I8 (815 mg, 2.5 mmol), Intermediate 14-5 (4.64 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 3.0 g of Compound 23. It was confirmed through LC-MS and NMR that Compound 23 was synthesized. MS: [M+H]+ = 1850

NMR measurement value of Compound 23: ¹H NMR (500 MHz, DMSO-d6) δ 7.93 (d, 2H), 7.80 - 7.72 (m, 4H), 7.69 (d, 2H), 7.58 - 7.48 (m, 16H), 7.30 - 7.19 (m, 22H), 7.10 - 6.96 (m, 26H), 6.82 (m, 2H), 6.64 (m, 2H), 5.66 (d, 2H), 5.14 (d, 2H)

### Synthesis Example 24. Synthesis of Compound 24

### (1) Synthesis of Intermediate 24-1

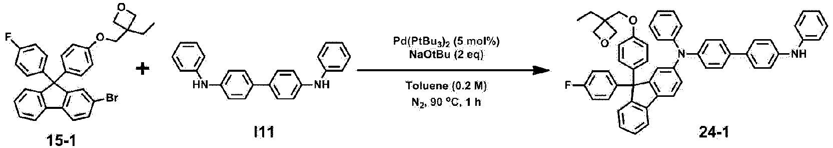

Intermediate 15-1 (10.6 g, 20 mmol), Compound I11 (13.5 g, 40 mmol), bis(tri-tert-butylphosphine) palladium (0) (Pd(PtBu₃)₂) (511 mg, 1 mmol, 5 mol%) and sodium tert-butoxide (NaOtBu) (3.84 g, 40 mmol, 2 eq) were put into RBF. After substitution with nitrogen, toluene (100 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 10.7 g of Intermediate 24-1.

### (2) Synthesis of Compound 24

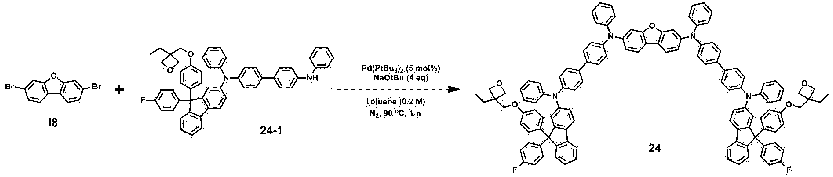

Compound I8 (815 mg, 2.5 mmol), Intermediate 24-1 (4.32 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 2.8 g of Compound 24. It was confirmed through LC-MS and NMR that Compound 24 was synthesized. MS: [M+H]+ = 1734

NMR measurement value of Compound 24: ¹H NMR (500 MHz, DMSO-d6) δ 7.93 (d, 2H), 7.80 - 7.72 (m, 4H), 7.69 (d, 2H), 7.49 - 7.40 (m, 10H), 7.31 - 6.90 (m, 48H), 6.83 - 6.74 (m, 6H), 4.37 (d, 4H), 4.28 (d, 4H), 4.00 (s, 4H), 1.71 (q, 4H), 0.82 (t, 6H)

### Synthesis Example 25. Synthesis of Compound 25

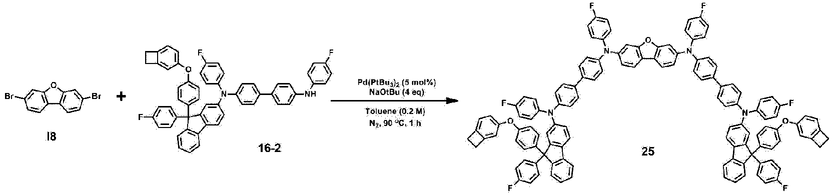

Compound I8 (815 mg, 2.5 mmol), Intermediate 16-2 (4.54 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 2.6 g of Compound 25. It was confirmed through LC-MS and NMR that Compound 25 was synthesized. MS: [M+H]+ = 1814

NMR measurement value of Compound 25: ¹H NMR (500 MHz, DMSO-d6) δ 7.93 (d, 2H), 7.80 - 7.72 (m, 4H), 7.69 (d, 2H), 7.49 - 7.40 (m, 10H), 7.32 - 6.87 (m, 52H), 6.86 - 6.79 (m, 4H), 3.00 (m, 8H)

### Synthesis Example 26. Synthesis of Compound 26

### (1) Synthesis of Intermediate 26-1

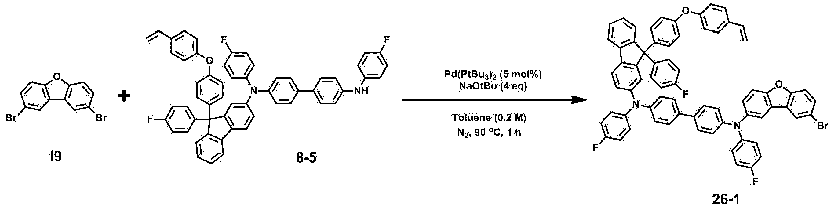

Intermediate 8-5 (8.25 g, 10 mmol), Compound I9 (6.52 g, 20 mmol), bis(tri-tert-butylphosphine)palladium(0) (Pd(PtBu₃)₂) (256 mg, 0.5 mmol, 5 mol%) and sodium tert-butoxide (NaOtBu) (1.92 g, 20 mmol, 2 eq) were put into RBF. After substitution with nitrogen, toluene (50 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 6 g of Intermediate 26-1.

### (2) Synthesis of Compound 26

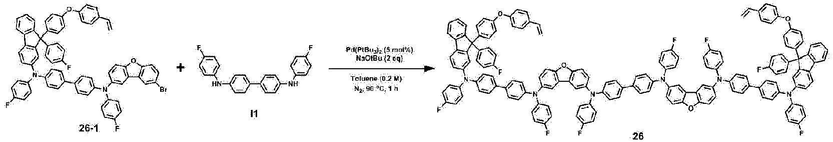

Compound I1 (931 mg, 2.5 mmol), Intermediate 26-1 (5.9 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 3.7 g of Compound 26. It was confirmed through LC-MS and NMR that Compound 26 was synthesized. MS: [M+H]+ = 2350

NMR measurement value of Compound 26: ¹H NMR (500 MHz, DMSO-d6) δ 8.11 (d, 4H), 7.80 - 7.72 (m, 4H), 7.55 - 7.43 (m, 22H), 7.35 - 7.21 (m, 22H), 7.11 - 6.88 (m, 46H), 6.64 (m, 2H), 5.66 (d, 2H), 5.14 (d, 2H)

### <Examples>

### Example 1.

A glass substrate thinly coated with indium tin oxide (ITO) to have a thickness of 1,500 Å was put into distilled water in which a detergent was dissolved, and ultrasonically washed. In this case, a product manufactured by the Fischer Co., was used as the detergent, and distilled water twice filtered using a filter manufactured by Millipore Co., was used as the distilled water. After the ITO was washed for 30 minutes, ultrasonic washing was conducted twice repeatedly using distilled water for 10 minutes. After the washing with distilled water was completed, the substrate was ultrasonically washed with isopropyl alcohol and acetone solvents, and dried, and then the substrate was cleaned for 5 minutes, and the substrate was transported to a glovebox.

On the ITO transparent electrode prepared above, a 1.5 wt% cyclohexanone ink including Compound 1 previously prepared in Synthesis Example 1 and the following Compound P at a weight ratio (Compound 1:Compound P) of 8:2 was spin-coated onto the ITO surface, and heat-treated (cured) at 230°C for 30 minutes, thereby forming a hole injection layer having a thickness of 30 nm. A 2 wt% toluene ink of the following α-NPD Compound was spin-coated onto the hole injection layer, thereby forming a hole transport layer having a thickness of 40 nm. Thereafter, the ITO transparent electrode was transferred to a vacuum deposition machine, and then the following ADN compound and the following DPAVBi compound were vacuum-deposited on the hole transport layer at a weight ratio (ADN:DPAVBi) of 20:1 to have a thickness of 20 nm, thereby forming a light emitting layer. The following BCP compound was vacuum-deposited on the light emitting layer to have a thickness of 35 nm, thereby forming an electron transport and injection layer. Lithium fluoride (LiF) and aluminum were sequentially deposited on the layer which simultaneously transports and injects electrons to have a thickness of 1 nm and 100 nm, respectively, thereby forming a cathode.

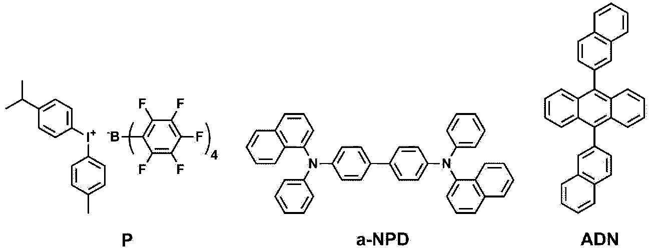

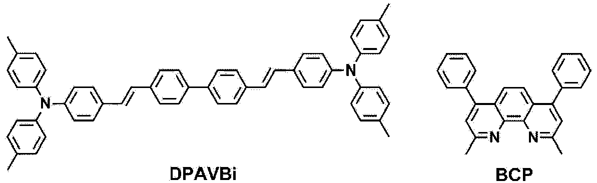

In the aforementioned procedure, the deposition rate of the organic material was maintained at 0.4 Å/sec to 0.7 Å/sec, the deposition rates of lithium fluoride and aluminum of the cathode were maintained at 0.3 Å/sec and at 2 Å/sec, respectively, and the degree of vacuum during the deposition was maintained at 2x10⁻⁷ torr to 5x10⁻⁶ torr.

### Example 2.

An organic light emitting device was manufactured in the same manner as in Example 1, except that Compound 2 was used instead of Compound 1.

### Example 3.

An organic light emitting device was manufactured in the same manner as in Example 1, except that Compound 3 was used instead of Compound 1.

### Example 4.

An organic light emitting device was manufactured in the same manner as in Example 1, except that Compound 4 was used instead of Compound 1.

### Example 5.

An organic light emitting device was manufactured in the same manner as in Example 1, except that Compound 5 was used instead of Compound 1.

### Example 6.

An organic light emitting device was manufactured in the same manner as in Example 1, except that Compound 6 was used instead of Compound 1.

### Example 7.

An organic light emitting device was manufactured in the same manner as in Example 1, except that Compound 7 was used instead of Compound 1.

### Comparative Example 1.

An organic light emitting device was manufactured in the same manner as in Example 1, except that the following Compound A-1 was used instead of Compound 1.

### Comparative Example 2.

An organic light emitting device was manufactured in the same manner as in Example 1, except that the following Compound A-2 was used instead of Compound 1.

### Comparative Example 3.

An organic light emitting device was manufactured in the same manner as in Example 1, except that the following Compound A-3 was used instead of Compound 1.

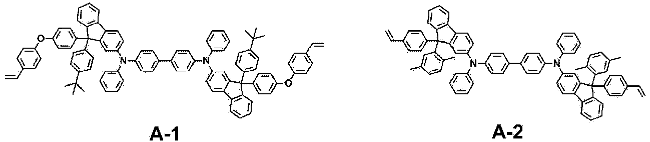

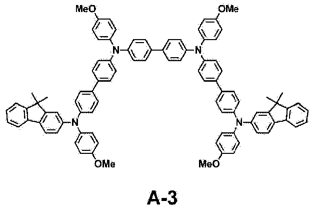

For the organic light emitting devices manufactured in Examples 1 to 7 and Comparative Examples 1 to 3, the driving voltage, current efficiency, quantum efficiency (QE), and luminance values were measured at an electric current of 10 mA/cm², and time (T95) taken for the luminance to become 95% of the initial luminance (1000 nit) was measured. The results are shown in the following Table 1.

**[Table 1]**

| | Driving voltage (V) | Current efficiency (Cd/A) | Power efficiency (lm/W) | Luminance (Cd/m²) | T95 (h) |
|---|---|---|---|---|---|
| Example 1 | 4.56 | 5.11 | 3.52 | 511.10 | 153 |
| Example 2 | 4.44 | 5.10 | 3.61 | 510.05 | 147 |
| Example 3 | 4.50 | 5.09 | 3.55 | 509.24 | 132 |
| Example 4 | 4.52 | 5.21 | 3.62 | 521.03 | 138 |
| Example 5 | 4.57 | 5.20 | 3.57 | 520.32 | 149 |
| Example 6 | 4.48 | 5.18 | 3.63 | 518.14 | 150 |
| Example 7 | 4.43 | 5.05 | 3.58 | 505.43 | 151 |
| Comparative Example 1 | 5.35 | 3.85 | 2.26 | 385.35 | 65 |
| Comparative Example 2 | 5.32 | 3.88 | 2.29 | 388.23 | 59 |
| Comparative Example 3 | 5.55 | 3.92 | 2.22 | 392.24 | 51 |

Through Table 1, it can be confirmed that organic light emitting devices (Examples 1 to 7) including the compound according to the present invention have lower driving voltage, higher efficiency and luminance and longer service life than an organic light emitting devices (Comparative Examples 1 and 2) using a compound including only two amine groups and an organic light emitting device (Comparative Example 3) using a compound including the different core structure (La) from that of the present invention, and not including a curable group.

### Example 8.

A glass substrate thinly coated with indium tin oxide (ITO) to have a thickness of 1,500 Å was put into distilled water in which a detergent was dissolved, and ultrasonically washed. In this case, a product manufactured by the Fischer Co., was used as the detergent, and distilled water twice filtered using a filter manufactured by Millipore Co., was used as the distilled water. After the ITO was washed for 30 minutes, ultrasonic washing was conducted twice repeatedly using distilled water for 10 minutes. After the washing with distilled water was completed, the substrate was ultrasonically washed with a solvent of isopropyl alcohol and acetone, and dried, and then the substrate was cleaned for 5 minutes, and the substrate was transported to a glovebox.

A 2 wt% cyclohexanone ink including Compound 8 prepared in Synthesis Example 8 and the following Compound G at a weight ratio of 8:2 was spin-coated onto the ITO surface, and heat-treated at 220°C for 30 minutes, thereby forming a hole injection layer having a thickness of 400 Å.

A 2 wt% toluene ink of the following Compound A was spin-coated onto the hole injection layer, and the coated material was heat-treated at 120°C for 10 minutes, thereby forming a hole transport layer having a thickness of 200 Å. The following Compound B and Compound C were vacuum-deposited at a weight ratio of 92:8 on the hole transport layer, thereby forming a light emitting layer having a thickness of 200 Å. The following Compound D was vacuum-deposited on the light emitting layer, thereby forming an electron transport and injection layer having a thickness of 350 Å. LiF and aluminum were sequentially deposited on the electron transport and injection layer to have a thickness of 10 Å and 1000 Å, respectively, thereby forming a cathode.

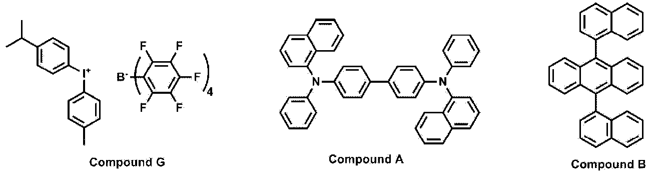

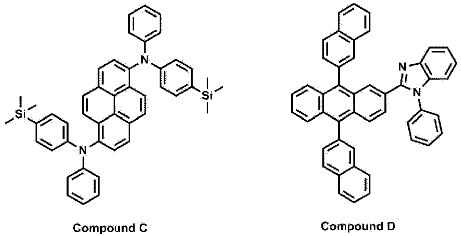

In the aforementioned procedure, the deposition rate of the organic material was maintained at 0.4 Å/sec to 0.7 Å/sec, the deposition rates of lithium fluoride and aluminum of the cathode were maintained at 0.3 Å/sec and at 2 Å/sec, respectively, and the degree of vacuum during the deposition was maintained at 2 × 10⁻⁷ torr to 5 × 10⁻⁸ torr.

### Examples 9 to 26

Organic light emitting devices were manufactured in the same manner as in Example 8, except that the compounds described in the following Table 2 were used instead of Compound 8 during the manufacture of the hole injection layer (HIL).

### Comparative Examples 4 to 8

Organic light emitting devices were manufactured in the same manner as in Example 8, except that the compounds described in the following Table 2 were used instead of Compound 8 during the manufacture of the hole injection layer.

Compounds CE1 to CE5 used in Comparative Examples 4 to 8 are as follows.

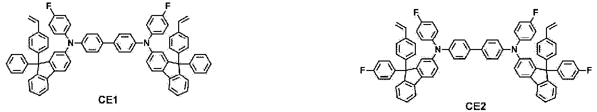

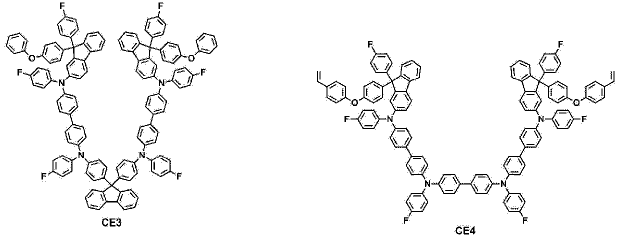

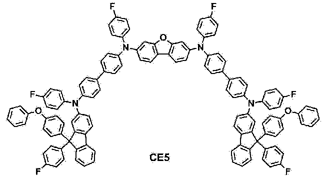

The results of measuring the driving voltage, external quantum efficiency, luminance, and service life of each of the organic light emitting devices manufactured in Examples 8 to 26 and Comparative Examples 4 to 8 at a current density of 10 mA/cm² are shown in the following Table 2.

The external quantum efficiency was obtained by (the number of emitted photons)/(the number of injected charge carriers). T95 means the time (hr) taken for the luminance to be reduced to 95% of the initial luminance (500 nit).

**[Table 2]**

| | HIL host | Driving voltage (V) | External quantum efficiency (%) | Luminance (cd/m₂) | T95 (hr) @500 nit |
|---|---|---|---|---|---|
| Example 8 | Compound 8 | 4.72 | 5.5 | 565 | 170 |
| Example 9 | Compound 9 | 4.66 | 5.6 | 559 | 168 |
| Example 10 | Compound 10 | 4.67 | 5.3 | 502 | 151 |
| Example 11 | Compound 11 | 4.49 | 5.4 | 518 | 187 |
| Example 12 | Compound 12 | 4.84 | 5.7 | 603 | 165 |
| Example 13 | Compound 13 | 4.48 | 5.8 | 618 | 188 |
| Example 14 | Compound 14 | 4.69 | 5.4 | 522 | 193 |
| Example 15 | Compound 15 | 4.82 | 5.2 | 497 | 151 |
| Example 16 | Compound 16 | 4.77 | 5.2 | 509 | 159 |
| Example 17 | Compound 17 | 4.57 | 5.3 | 552 | 161 |
| Example 18 | Compound 18 | 4.42 | 5.3 | 562 | 169 |
| Example 19 | Compound 19 | 4.41 | 5.4 | 567 | 178 |
| Example 20 | Compound 20 | 4.59 | 5.5 | 571 | 183 |
| Example 21 | Compound 21 | 4.42 | 5.8 | 604 | 164 |
| Example 22 | Compound 22 | 4.77 | 5.7 | 596 | 155 |
| Example 23 | Compound 23 | 4.49 | 5.2 | 553 | 168 |
| Example 24 | Compound 24 | 4.85 | 5.1 | 525 | 160 |
| Example 25 | Compound 25 | 4.80 | 5.0 | 512 | 151 |
| Example 26 | Compound 26 | 4.33 | 5.2 | 533 | 159 |
| Comparative Example 4 | CE1 | 5.23 | 4.7 | 421 | 125 |
| Comparative Example 5 | CE2 | 5.10 | 5.0 | 492 | 146 |
| Comparative Example 6 | CE3 | 8.13 | 2.0 | 208 | 51 |
| Comparative Example 7 | CE4 | 5.49 | 3.2 | 389 | 99 |
| Comparative Example 8 | CE5 | 9.31 | 2.3 | 216 | 42 |

The compound of Chemical Formula 1 according to the present invention was used as a host of a hole injection layer in Examples 8 to 26, and Compounds CE1 and CE2 including only two amine groups, Compounds CE3 and CE5 including no curing group, or Compound CE4 whose core structure (La) is a biphenylene group were used in Comparative Examples 4 to 8.

As shown in Table 2, it can be confirmed that the driving voltage is reduced and the service life is improved in the organic light emitting devices (Examples 8 to 26) in which the compound of Chemical Formula 1 according to the present invention is used as a host of a hole injection layer, compared to the organic light emitting devices of Comparative Examples 4 to 8. Further, it can be confirmed that the efficiency and luminance are increased in the organic light emitting devices of Examples 8 to 26 compared to the organic light emitting devices of Comparative Examples 4 to 8. Through this, it could be confirmed that the movement of holes in the molecule was facilitated by including four or more N's in the molecule to improve the hole mobility, thereby improving the performance of the organic light emitting device. In addition, since Comparative Examples 6 and 8, in which the compound including no curing group is used, do not have solvent resistance even after heat treatment, it can be seen that the device does not properly function because most of the materials are washed away during the film-formation of the hole transport layer (HTL). Furthermore, Comparative Example 7, in which a compound having a biphenylene group as the core structure is used, exhibited characteristics that the solubility of the compound was reduced, which affected film properties to show the low device performance.

Although the preferred exemplary embodiments (the hole injection layer) of the present invention have been described above, the present invention is not limited thereto, and various modifications can be made and carried out within the scope of the claims and the detailed description of the invention, and also fall within the scope of the invention.

## Claims

1. A compound of the following Chemical Formula 1: 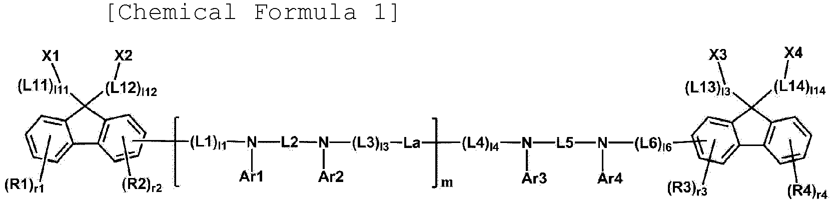 wherein, in Chemical Formula 1,
La is -Lx-A-Ly-; a substituted or unsubstituted divalent dihydroanthracene group; a substituted or unsubstituted divalent heterocyclic group; a substituted or unsubstituted divalent fluorenyl group; or a substituted or unsubstituted divalent spirobifluorene group,
Lx and Ly are the same as or different from each other, and are each independently a substituted or unsubstituted arylene group,
A is a substituted or unsubstituted divalent dihydroanthracene group; a substituted or unsubstituted divalent heterocyclic group; a substituted or unsubstituted divalent fluorenyl group; or a substituted or unsubstituted divalent spirobifluorene group,
L1 to L6 are the same as or different from each other, and are each independently a substituted or unsubstituted arylene group; or a substituted or unsubstituted divalent heterocyclic group,
L11 to L14 are the same as or different from each other, and are each independently a direct bond; -O-; a substituted or unsubstituted alkylene group; a substituted or unsubstituted arylene group; or a substituted or unsubstituted divalent heterocyclic group,
Ar1 to Ar4 are the same as or different from each other, and are each independently a substituted or unsubstituted alkyl group; a substituted or unsubstituted aryl group; or a substituted or unsubstituted heterocyclic group,
X1 to X4 are the same as or different from each other, and are each independently hydrogen; deuterium; a halogen group; a substituted or unsubstituted alkyl group; a substituted or unsubstituted alkoxy group; a substituted or unsubstituted aryl group; a substituted or unsubstituted heterocyclic group; a photocurable group or a thermosetting group, and two or more of X1 to X4 are a photocurable group or a thermosetting group,
R1 to R4 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group,
l1, l3, l4 and l6 are each an integer from 0 to 3, and when l1, l3, l4 and l6 are each 2 or higher, structures in the parenthesis are the same as or different from each other,
l11 to l14 are each an integer from 1 to 3, and when l11 to l14 are each 2 or higher, structures in the parenthesis are the same as or different from each other,
r1 and r4 are each an integer from 1 to 4, r2 and r3 are each an integer from 1 to 3, and when r1 to r4 are each 2 or higher, substituents in the parenthesis are the same as or different from each other, and
m is an integer from 1 to 10, and when m is 2 or higher, structures in the parenthesis are the same as or different from each other.

2. The compound of claim 1, wherein the photocurable group or thermosetting group is any one of the following structures: 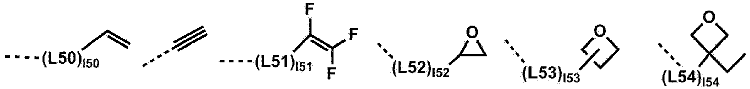 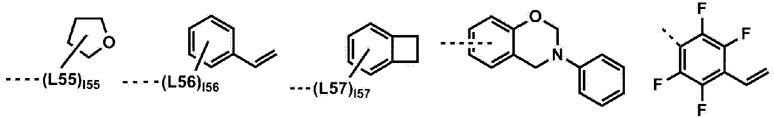 in the structures,
L50 to L57 are the same as or different from each other, and are each independently a direct bond; -O-; or a substituted or unsubstituted alkylene group,
l50 to l57 are each an integer from 1 to 5, and when l50 to l57 are each 2 or higher, structures in the parenthesis are the same as or different from each other, and
------ is a moiety bonded to Chemical Formula 1.

3. The compound of claim 1, wherein La is any one of the following structures: 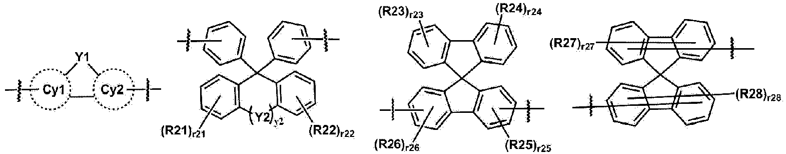 in the structures,
Cy1 and Cy2 are the same as or different from each other, and are each independently a substituted or unsubstituted aromatic ring,
Y1 and Y2 are the same as or different from each other, and are each independently O; S; CRaRb; or SiRcRd,
Ra to Rd are the same as or different from each other, and are each independently hydrogen; deuterium; a substituted or unsubstituted alkyl group; or a substituted or unsubstituted aryl group,
R21 to R28 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group, or are bonded to an adjacent group to form a substituted or unsubstituted ring,
y2 is 0 or 1,
r21 to r24 are each an integer from 1 to 4, r25 and r26 are each an integer from 1 to 3, r27 and r28 are each an integer from 1 to 7, and when r21 to r28 are 2 or higher, substituents in the parenthesis are the same as or different from each other, and 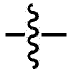
is a moiety bonded to Chemical Formula 1.

4. The compound of claim 1, wherein La is any one of the following structures: 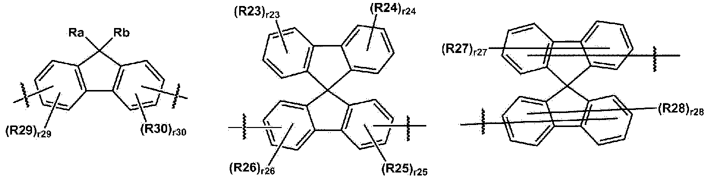 in the structures,
Ra and Rb are the same as or different from each other, and are each independently hydrogen; deuterium; a substituted or unsubstituted alkyl group; or a substituted or unsubstituted aryl group,
R23 to R30 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group,
r25, r26, r29 and r30 are each an integer from 1 to 3, r23 and r24 are each an integer from 1 to 4, r27 and r28 are each an integer from 1 to 7, and when r23 to r30 are 2 or higher, substituents in the parenthesis are the same as or different from each other, and 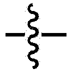
is a moiety bonded to Chemical Formula 1.

5. The compound of claim 1, wherein La is the following structure: 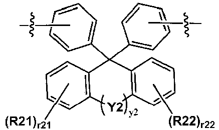 in the structure,
Y2 is O; S; CRaRb; or SiRcRd,
Ra to Rd are the same as or different from each other, and are each independently hydrogen; deuterium; a substituted or unsubstituted alkyl group; or a substituted or unsubstituted aryl group,
R21 and R22 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group, or are bonded to an adjacent group to form a substituted or unsubstituted ring,
y2 is 0 or 1,
r21 and r22 are each an integer from 1 to 4, and when r21 and r22 are each 2 or higher, substituents in the parenthesis are the same as or different from each other, and 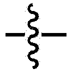
is a moiety bonded to Chemical Formula 1.

6. The compound of claim 1, wherein La is the following structure: 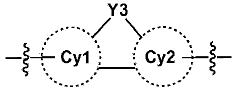 in the structure,
Cy1 and Cy2 are the same as or different from each other, and are each independently a substituted or unsubstituted aromatic ring,
Y3 is O; S; or SiRcRd,
Rc and Rd are the same as or different from each other, and are each independently hydrogen; deuterium; a substituted or unsubstituted alkyl group; or a substituted or unsubstituted aryl group, and 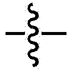
is a moiety bonded to Chemical Formula 1.

7. The compound of claim 1, wherein La is any one of the following structures, and the following structures are unsubstituted or substituted with an additional substituent: 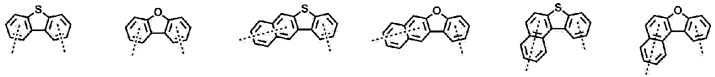 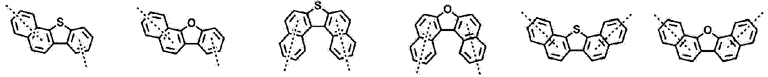 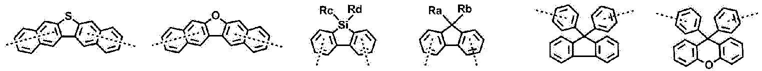 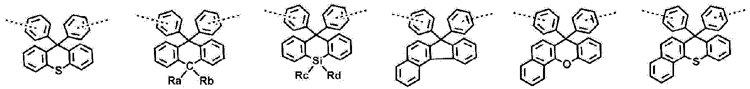 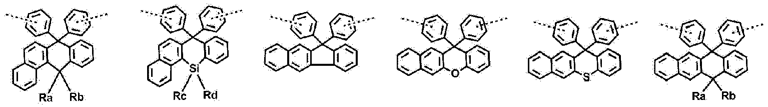 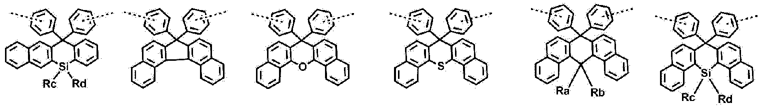 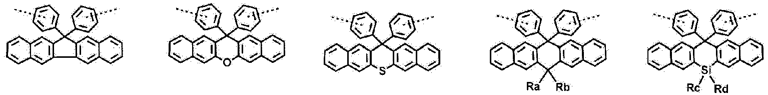 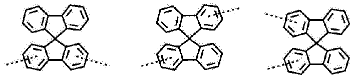 in the structures,
Ra to Rd are the same as or different from each other, and are each independently hydrogen; deuterium; a substituted or unsubstituted alkyl group; or a substituted or unsubstituted aryl group, and
------ is a moiety linked to Chemical Formula 1.

8. The compound of claim 1, wherein Chemical Formula 1 is the following Chemical Formula 1-1: 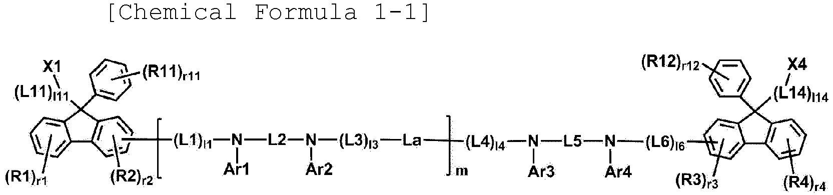 in Chemical Formula 1-1,
La, L1 to L6, L11, L14, Ar1 to Ar4, R1 to R4, l1, l3, l4, l6, l11, l14, r1 to r4 and m are the same as those defined in Chemical Formula 1,
X1 and X4 are the same as or different from each other, and are each independently a photocurable group or a thermosetting group,
R11 and R12 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heterocyclic group, and
r11 and r12 are each an integer from 1 to 5, and when r11 and r12 are each 2 or higher, substituents in the parenthesis are the same as or different from each other.

9. The compound of claim 1, wherein Chemical Formula 1 is any one of the following structures:

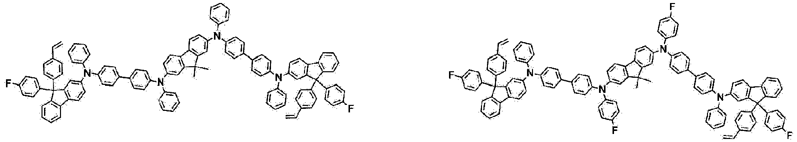

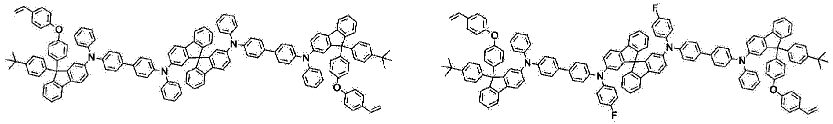

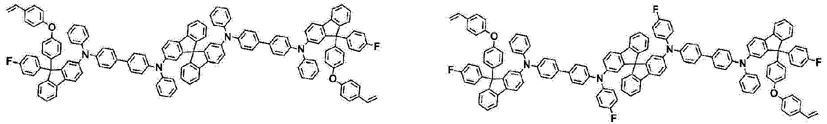

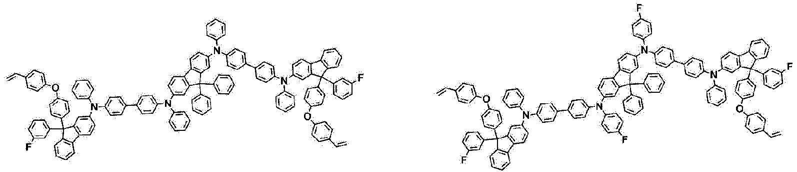

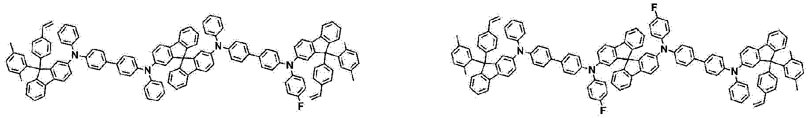

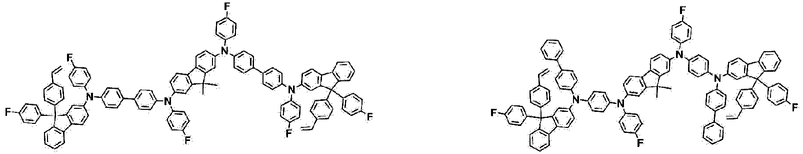

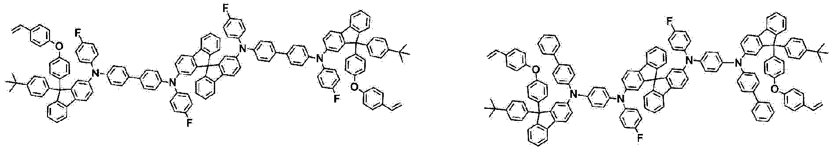

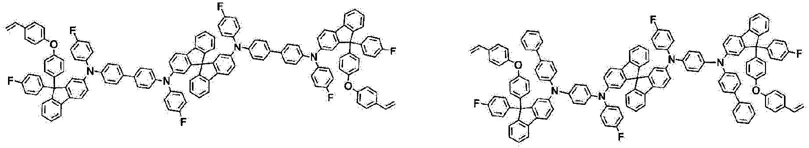

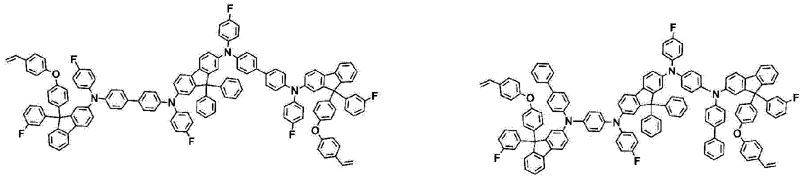

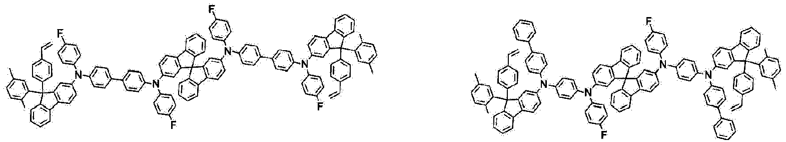

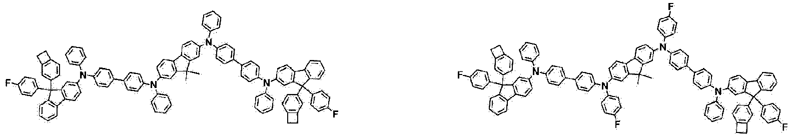

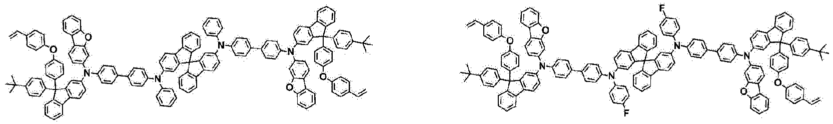

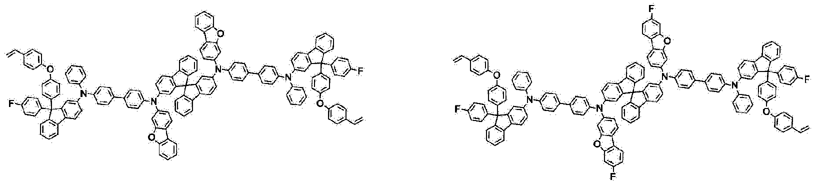

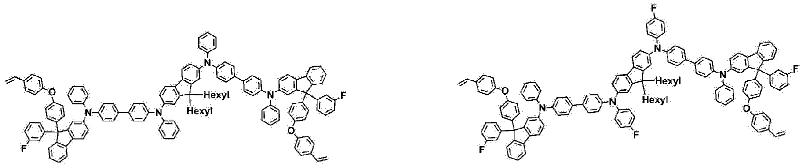

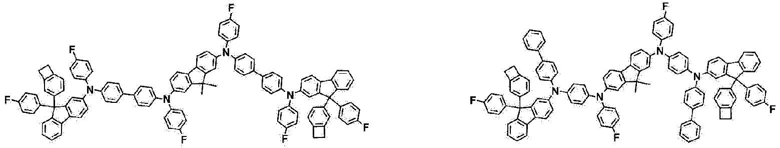

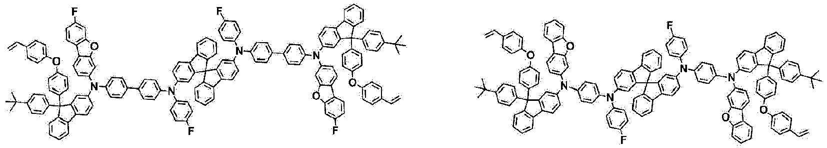

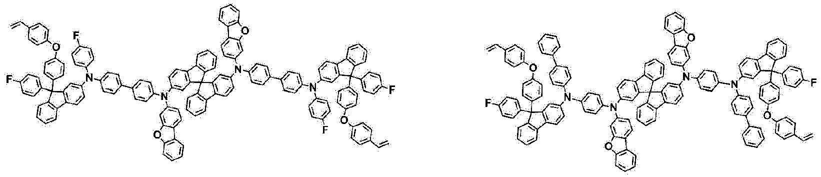

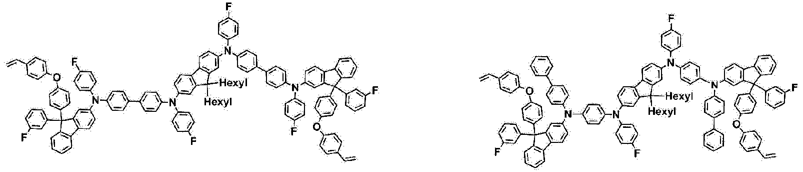

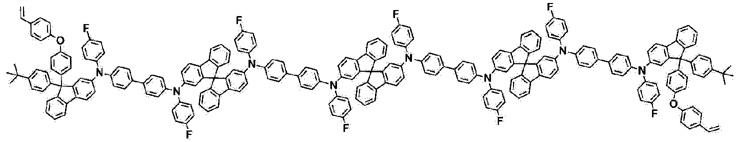

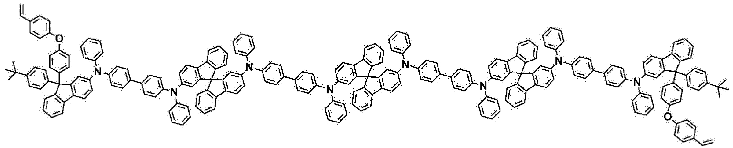

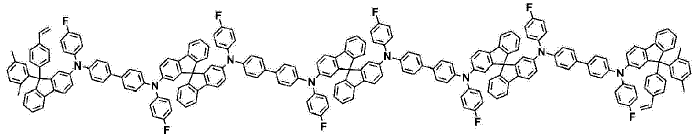

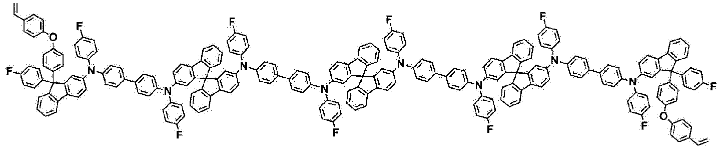

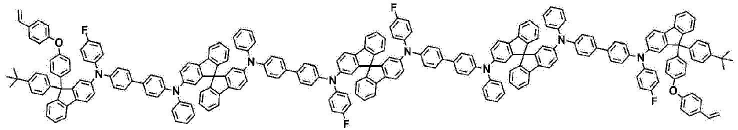

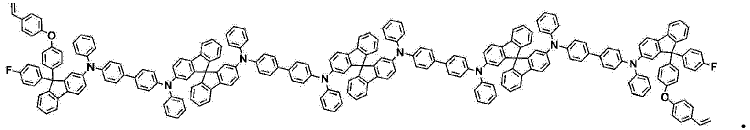

10. The compound of claim 1, wherein Chemical Formula 1 is any one of the following structures:

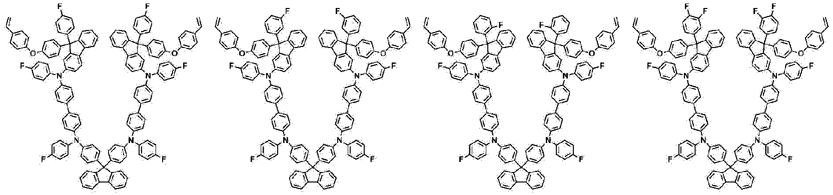

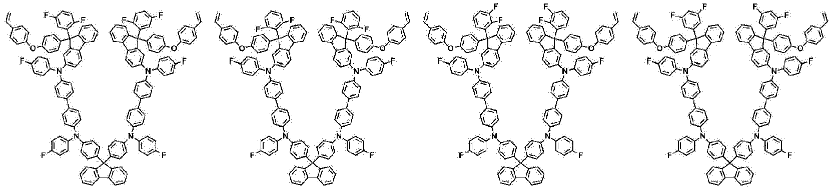

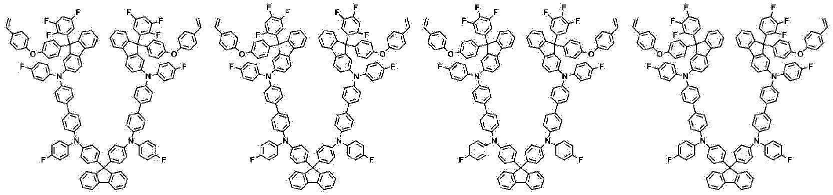

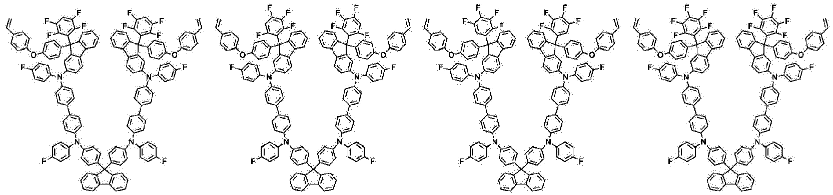

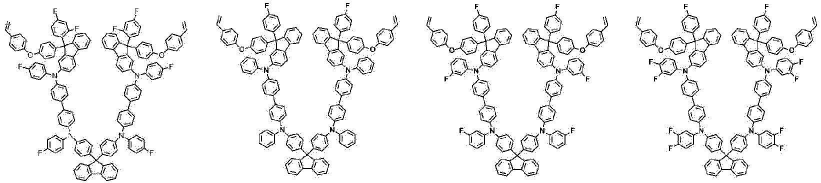

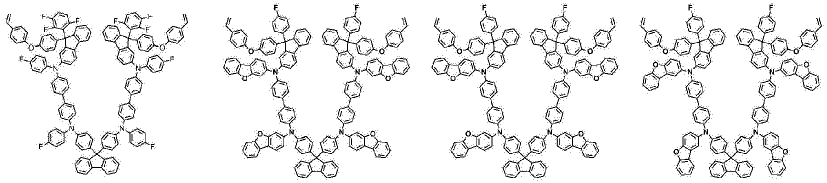

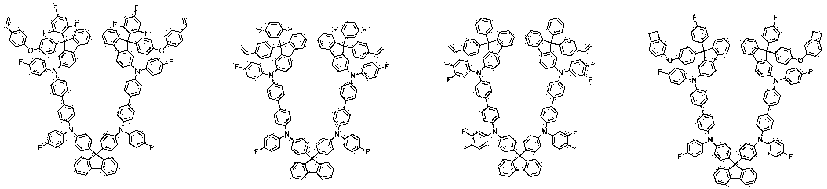

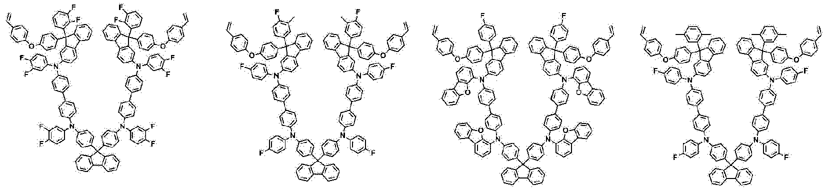

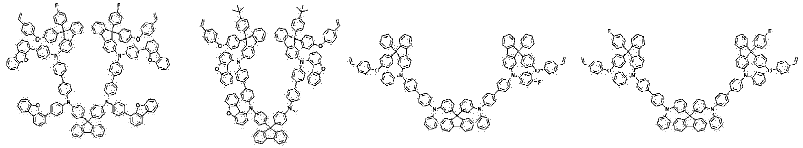

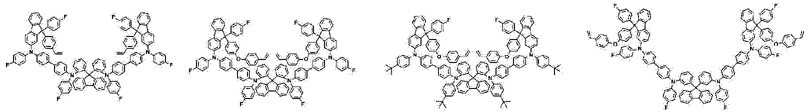

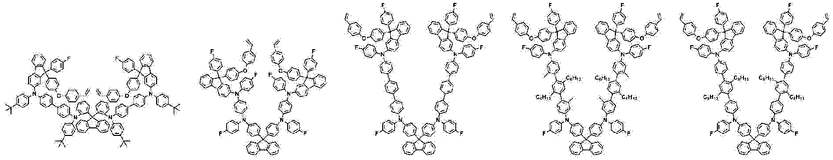

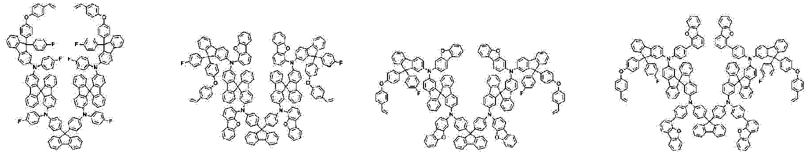

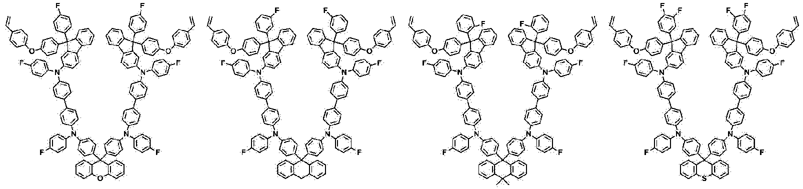

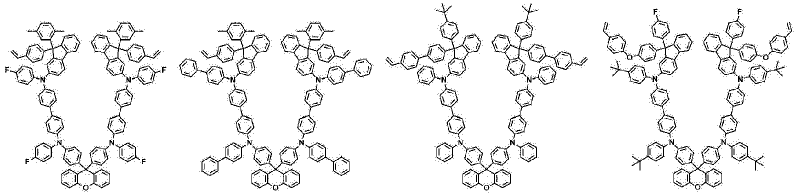

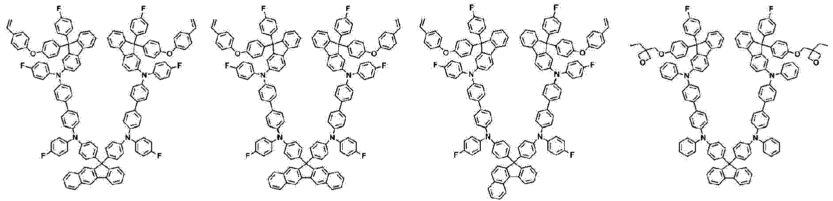

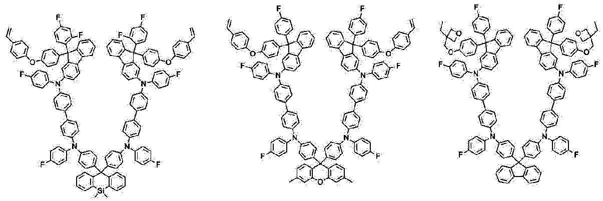

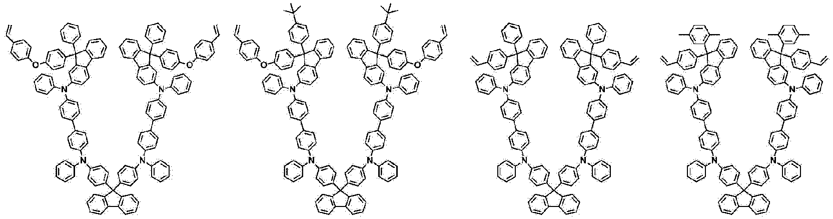

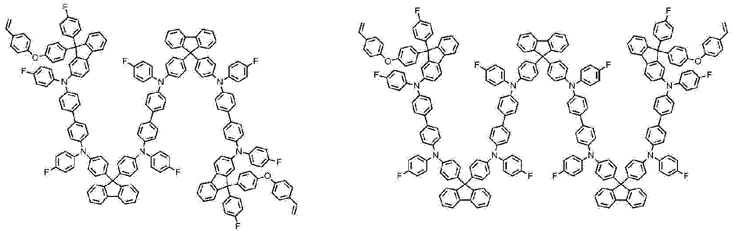

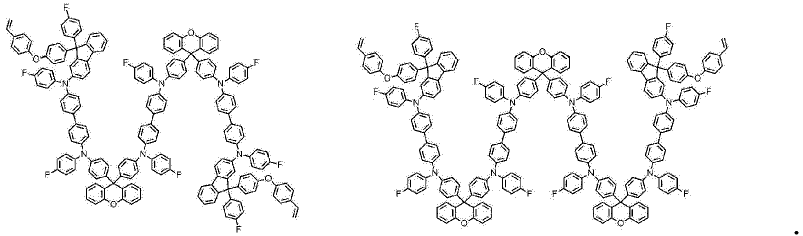

11. The compound of claim 1, wherein Chemical Formula 1 is any one of the following structures:

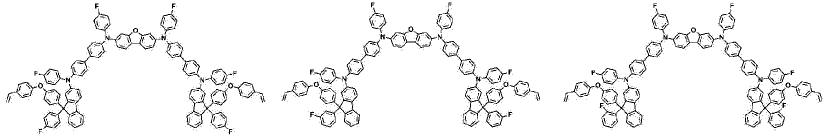

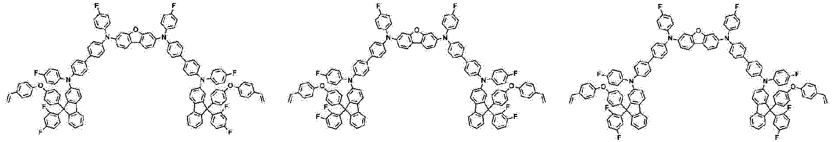

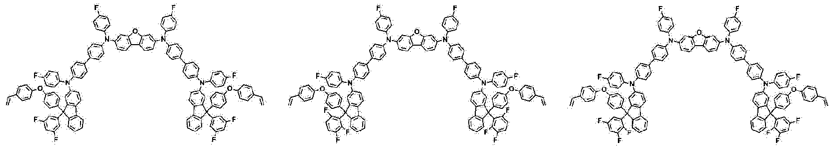

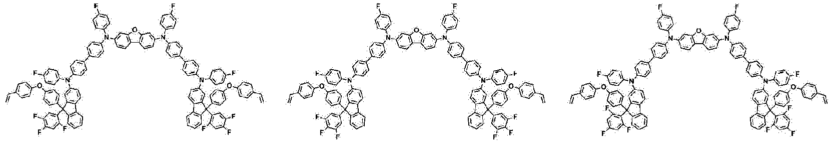

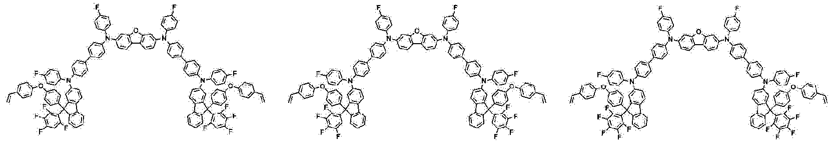

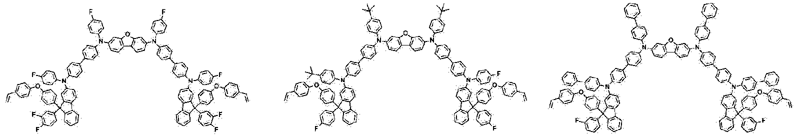

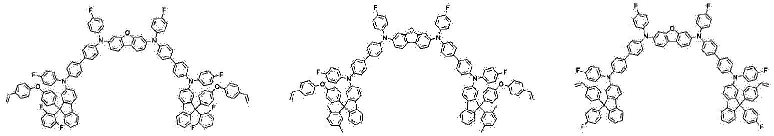

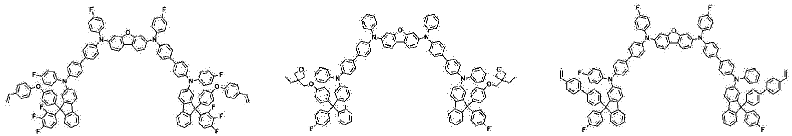

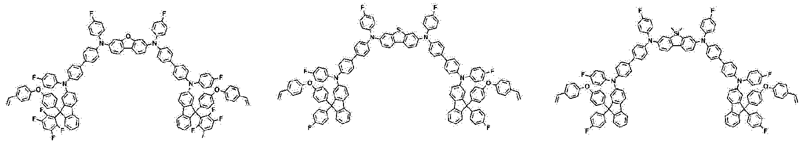

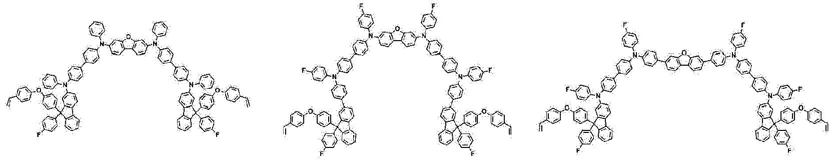

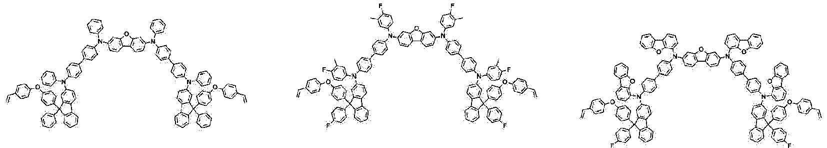

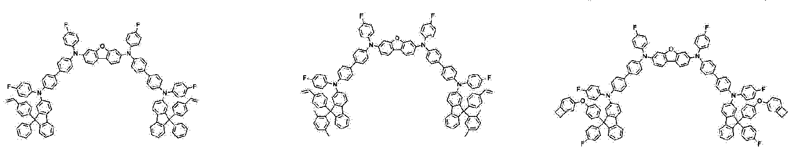

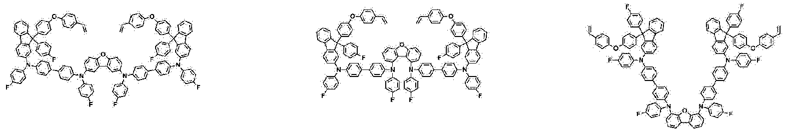

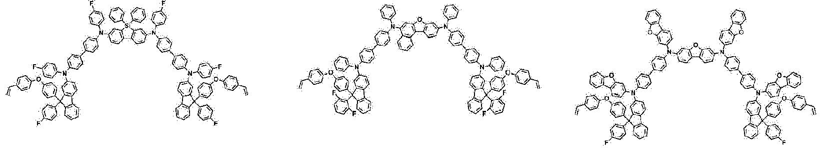

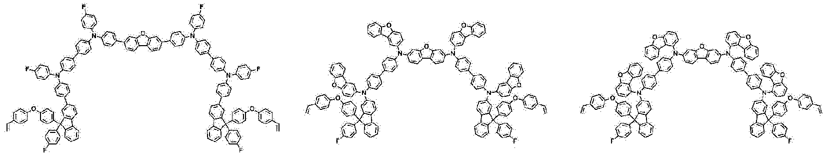

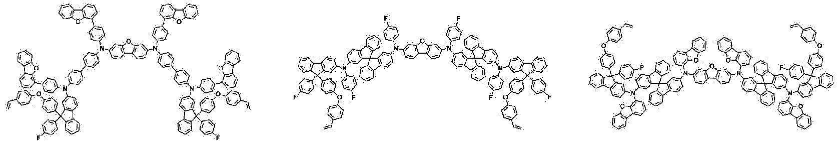

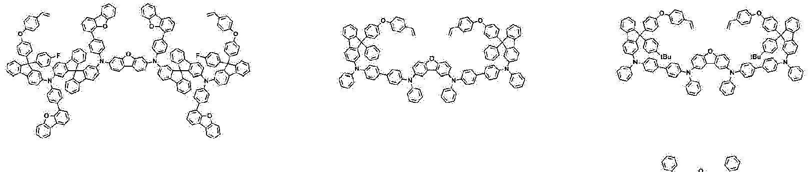

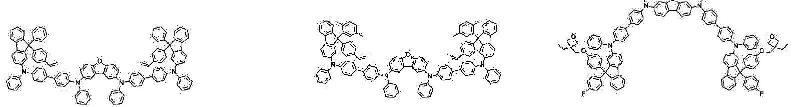

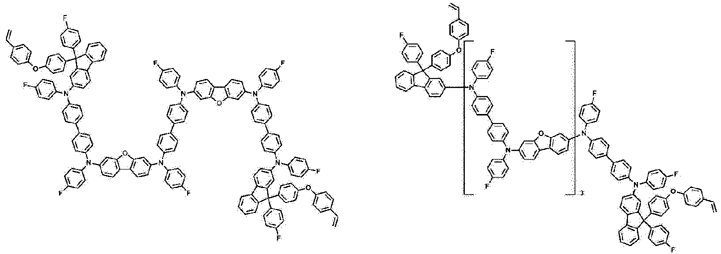

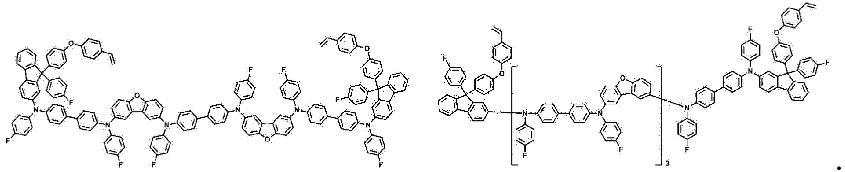

12. A coating composition comprising the compound of any one of claims 1 to 11.

13. An organic light emitting device comprising: a first electrode;
a second electrode; and
an organic material layer having one or more layers, which includes a light emitting layer provided between the first electrode and the second electrode,
wherein one or more layers of the organic material layer comprise the coating composition of claim 12 or a cured product thereof.

14. A method of manufacturing an organic light emitting device, the method comprising: preparing a substrate;
forming a first electrode on the substrate:
forming an organic material layer having one or more layers on the first electrode; and
forming a second electrode on the organic material layer,
wherein the forming of the organic material layer comprises forming an organic material layer having one or more layers by using the coating composition of claim 12.

15. The method of claim 14, wherein the forming of the organic material layer having one or more layers by using the coating composition comprises:
coating the coating composition on the first electrode; and
heat-treating or light-treating the coated coating composition.

## Patentansprüche

1. Verbindung der folgenden chemischen Formel 1: 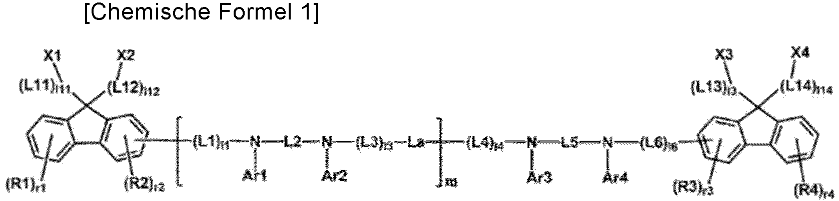 wobei in der chemischen Formel 1,
La gleich -Lx-A-Ly-; eine substituierte oder unsubstituierte zweiwertige Dihydroanthracengruppe; eine substituierte oder unsubstituierte zweiwertige heterocyclische Gruppe; eine substituierte oder unsubstituierte zweiwertige Fluorenylgruppe; oder eine substituierte oder unsubstituierte zweiwertige Spirobifluorengruppe ist,
Lx und Ly gleich oder verschieden voneinander sind und jeweils unabhängig voneinander eine substituierte oder unsubstituierte Arylengruppe sind,
A eine substituierte oder unsubstituierte zweiwertige Dihydroanthracengruppe; eine substituierte oder unsubstituierte zweiwertige heterocyclische Gruppe; eine substituierte oder unsubstituierte zweiwertige Fluorenylgruppe; oder eine substituierte oder unsubstituierte zweiwertige Spirobifluorengruppe ist,
L1 bis L6 gleich oder verschieden voneinander sind und jeweils unabhängig voneinander eine substituierte oder unsubstituierte Arylengruppe; oder eine substituierte oder unsubstituierte zweiwertige heterocyclische Gruppe sind,
L11 bis L14 gleich oder verschieden voneinander sind und jeweils unabhängig voneinander eine direkte Bindung; -O-; eine substituierte oder unsubstituierte Alkylengruppe; eine substituierte oder unsubstituierte Arylengruppe; oder eine substituierte oder unsubstituierte zweiwertige heterocyclische Gruppe sind,
Ar1 bis Ar4 gleich oder verschieden voneinander sind und jeweils unabhängig voneinander eine substituierte oder unsubstituierte Alkylgruppe; eine substituierte oder unsubstituierte Arylgruppe; oder eine substituierte oder unsubstituierte heterocyclische Gruppe sind,
X1 bis X4 gleich oder verschieden voneinander sind und jeweils unabhängig voneinander Wasserstoff; Deuterium; eine Halogengruppe; eine substituierte oder unsubstituierte Alkylgruppe; eine substituierte oder unsubstituierte Alkoxygruppe; eine substituierte oder unsubstituierte Arylgruppe; eine substituierte oder unsubstituierte heterocyclische Gruppe; eine photohärtbare Gruppe oder eine wärmehärtende Gruppe, und zwei oder mehr von X1 bis X4 sind eine photohärtbare Gruppe oder eine wärmehärtende Gruppe sind,
R1 bis R4 gleich oder verschieden voneinander sind und jeweils unabhängig voneinander Wasserstoff; Deuterium; eine Halogengruppe; eine substituierte oder unsubstituierte Alkylgruppe; eine substituierte oder unsubstituierte Alkoxygruppe; eine substituierte oder unsubstituierte Arylgruppe; oder eine substituierte oder unsubstituierte heterocyclische Gruppe sind,
l1, l3, l4 und l6 jeweils eine ganze Zahl von 0 bis 3 sind, und wenn l1, l3, l4 und l6 jeweils 2 oder höher sind, sind die Strukturen in der Klammer gleich oder verschieden voneinander,
l11 bis l14 jeweils eine ganze Zahl von 1 bis 3 sind, und wenn l11 bis l14 jeweils 2 oder höher sind, sind die Strukturen in der Klammer gleich oder verschieden voneinander,
r1 und r4 jeweils eine ganze Zahl von 1 bis 4 sind, r2 und r3 jeweils eine ganze Zahl von 1 bis 3 sind, und wenn r1 bis r4 jeweils 2 oder höher sind, sind die Substituenten in der Klammer gleich oder verschieden voneinander, und
m eine ganze Zahl von 1 bis 10 ist, und wenn m gleich 2 oder höher ist, sind die Strukturen in der Klammer gleich oder verschieden voneinander.

2. Verbindung nach Anspruch 1, wobei die photohärtbare Gruppe oder die wärmehärtende Gruppe eine der folgenden Strukturen ist: 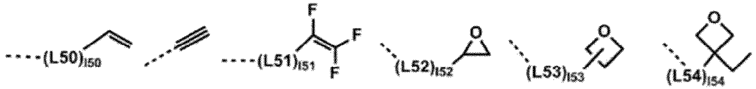 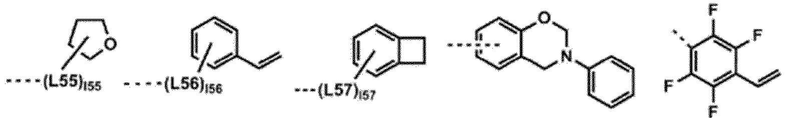 in den Strukturen,
L50 bis L57 sind gleich oder verschieden voneinander und sind jeweils unabhängig voneinander eine direkte Bindung; -O-; oder eine substituierte oder unsubstituierte Alkylengruppe,
l50 bis l57 sind jeweils eine ganze Zahl von 1 bis 5, und wenn l50 bis l57 jeweils 2 oder höher sind, sind die Strukturen in der Klammer gleich oder verschieden voneinander, und
------ ist eine Einheit, die an die chemische Formel 1 gebunden ist.

3. Verbindung nach Anspruch 1, wobei La eine der folgenden Strukturen ist: 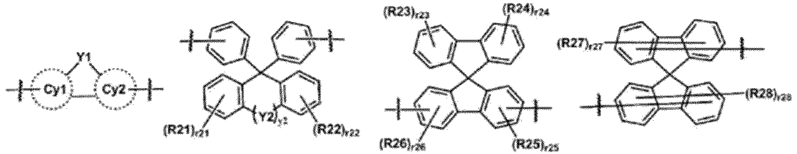 in den Strukturen,
Cy1 und Cy2 sind gleich oder verschieden voneinander und sind jeweils unabhängig voneinander ein substituierter oder unsubstituierter aromatischer Ring,
Y1 und Y2 sind gleich oder verschieden voneinander und sind jeweils unabhängig voneinander O; S; CRaRb; oder SiRcRd,
Ra bis Rd sind gleich oder verschieden voneinander und sind jeweils unabhängig voneinander Wasserstoff; Deuterium; eine substituierte oder unsubstituierte Alkylgruppe; oder eine substituierte oder unsubstituierte Arylgruppe,
R21 bis R28 sind gleich oder verschieden voneinander und sind jeweils unabhängig voneinander Wasserstoff; Deuterium; eine Halogengruppe; eine substituierte oder unsubstituierte Alkylgruppe; eine substituierte oder unsubstituierte Alkoxygruppe; eine substituierte oder unsubstituierte Arylgruppe; oder eine substituierte oder unsubstituierte heterocyclische Gruppe, oder sind an eine benachbarte Gruppe gebunden, um einen substituierten oder unsubstituierten Ring zu bilden,
y2 ist 0 oder 1,
r21 bis r24 sind jeweils eine ganze Zahl von 1 bis 4, r25 und r26 sind jeweils eine ganze Zahl von 1 bis 3, r27 und r28 sind jeweils eine ganze Zahl von 1 bis 7, und wenn r21 bis r28 gleich 2 oder höher sind, sind die Substituenten in der Klammer gleich oder verschieden voneinander, und 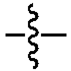
ist eine Einheit, die an die chemische Formel 1 gebunden ist.

4. Verbindung nach Anspruch 1, wobei La eine der folgenden Strukturen ist: 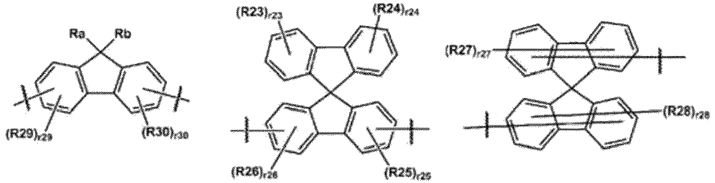 in den Strukturen,
Ra und Rb sind gleich oder verschieden voneinander und sind jeweils unabhängig voneinander Wasserstoff; Deuterium; eine substituierte oder unsubstituierte Alkylgruppe; oder eine substituierte oder unsubstituierte Arylgruppe,
R23 bis R30 sind gleich oder verschieden voneinander und jeweils unabhängig voneinander Wasserstoff; Deuterium; eine Halogengruppe; eine substituierte oder unsubstituierte Alkylgruppe; eine substituierte oder unsubstituierte Alkoxygruppe; eine substituierte oder unsubstituierte Arylgruppe; oder eine substituierte oder unsubstituierte heterocyclische Gruppe,
r25, r26, r29 und r30 sind jeweils eine ganze Zahl von 1 bis 3, r23 und r24 sind jeweils eine ganze Zahl von 1 bis 4, r27 und r28 sind jeweils eine ganze Zahl von 1 bis 7, und wenn r23 bis r30 2 oder höher sind, sind die Substituenten in der Klammer gleich oder verschieden voneinander, und 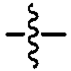
ist eine Einheit, die an die chemische Formel 1 gebunden ist.

5. Verbindung nach Anspruch 1, wobei La die folgende Struktur ist: 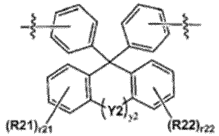 in der Struktur,
Y2 ist gleich O; S; CRaRb; oder SiRcRd,
Ra bis Rd sind gleich oder verschieden voneinander und sind jeweils unabhängig voneinander Wasserstoff; Deuterium; eine substituierte oder unsubstituierte Alkylgruppe; oder eine substituierte oder unsubstituierte Arylgruppe,
R21 und R22 sind gleich oder verschieden voneinander und sind jeweils unabhängig voneinander Wasserstoff; Deuterium; eine Halogengruppe; eine substituierte oder unsubstituierte Alkylgruppe; eine substituierte oder unsubstituierte Alkoxygruppe; eine substituierte oder unsubstituierte Arylgruppe; oder eine substituierte oder unsubstituierte heterocyclische Gruppe, oder sind an eine benachbarte Gruppe gebunden, um einen substituierten oder unsubstituierten Ring zu bilden,
y2 ist 0 oder 1,
r21 und r22 sind jeweils eine ganze Zahl von 1 bis 4, und wenn r21 und r22 jeweils 2 oder höher sind, sind die Substituenten in der Klammer gleich oder verschieden voneinander, und 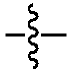
ist eine Einheit, die an die chemische Formel 1 gebunden ist.

6. Verbindung nach Anspruch 1, wobei La die folgende Struktur ist: 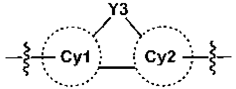 in der Struktur,
Cy1 und Cy2 sind gleich oder verschieden voneinander und sind jeweils unabhängig voneinander ein substituierter oder unsubstituierter aromatischer Ring,
Y3 ist O; S; oder SiRcRd,
Rc und Rd sind gleich oder verschieden voneinander und sind jeweils unabhängig voneinander Wasserstoff; Deuterium; eine substituierte oder unsubstituierte Alkylgruppe; oder eine substituierte oder unsubstituierte Arylgruppe, und 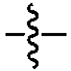
ist eine Einheit, die an die chemische Formel 1 gebunden ist.

7. Verbindung nach Anspruch 1, wobei La eine der folgenden Strukturen ist, und die folgenden Strukturen unsubstituiert oder mit einem zusätzlichen Substituenten substituiert sind: 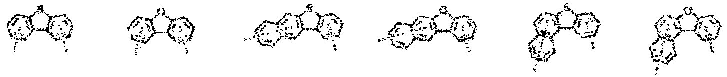 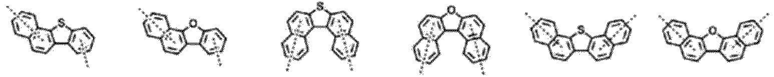 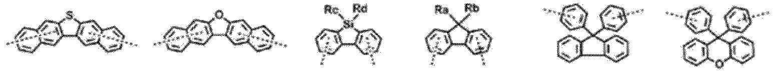 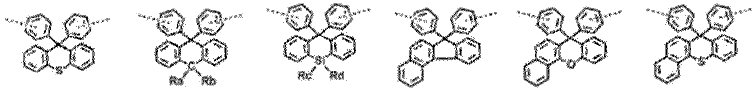 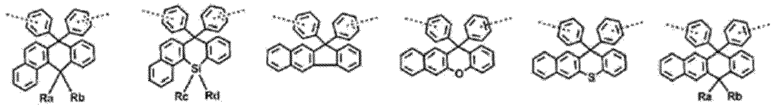 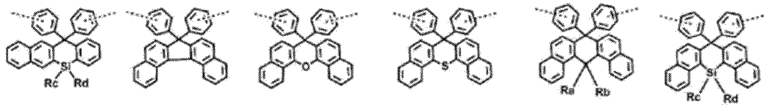 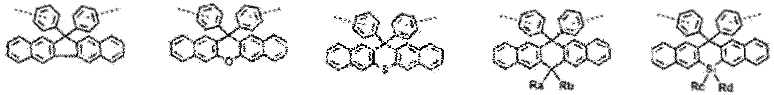 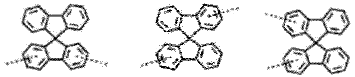 in den Strukturen,
Ra bis Rd sind gleich oder verschieden voneinander und sind jeweils unabhängig voneinander Wasserstoff; Deuterium; eine substituierte oder unsubstituierte Alkylgruppe; oder eine substituierte oder unsubstituierte Arylgruppe, und
------ ist eine mit der chemischen Formel 1 verbundene Einheit.

8. Verbindung nach Anspruch 1, wobei die chemische Formel 1 die folgende chemische Formel 1-1 ist: 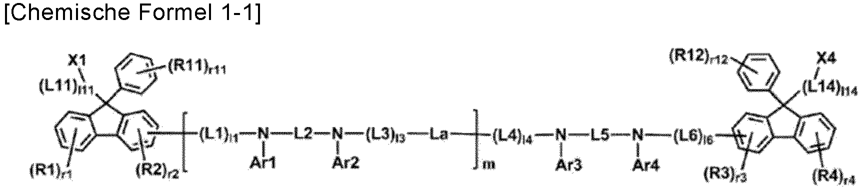 in der chemischen Formel 1-1,
La, L1 bis L6, L11, L14, Ar1 bis Ar4, R1 bis R4, l1, l3, l4, l6, l11, l14, r1 bis r4 und m sind die gleichen wie die in der chemischen Formel 1 definierten,
X1 und X4 sind gleich oder verschieden voneinander und sind jeweils unabhängig voneinander eine photohärtbare Gruppe oder eine wärmehärtende Gruppe,
R11 und R12 sind gleich oder verschieden voneinander und sind jeweils unabhängig voneinander Wasserstoff; Deuterium; eine Halogengruppe; eine substituierte oder unsubstituierte Alkylgruppe; eine substituierte oder unsubstituierte Alkoxygruppe; eine substituierte oder unsubstituierte Arylgruppe; oder eine substituierte oder unsubstituierte heterocyclische Gruppe, und
r11 und r12 sind jeweils eine ganze Zahl von 1 bis 5, und wenn r11 und r12 jeweils 2 oder höher sind, sind die Substituenten in der Klammer gleich oder verschieden voneinander.

9. Verbindung nach Anspruch 1, wobei die chemische Formel 1 eine der folgenden Strukturen ist:

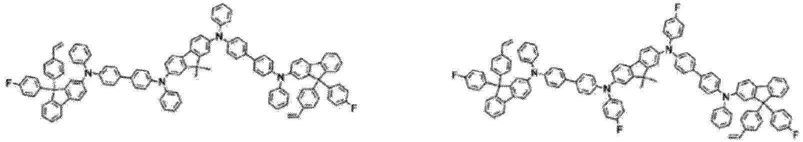

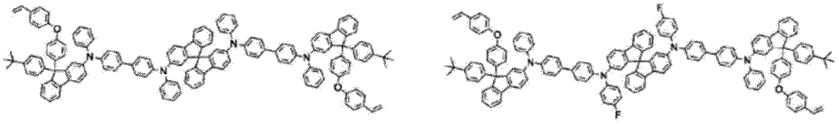

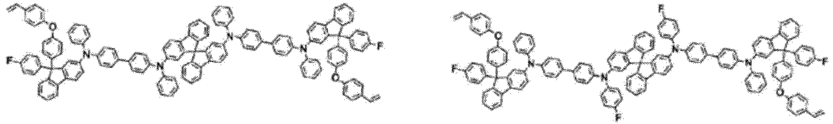

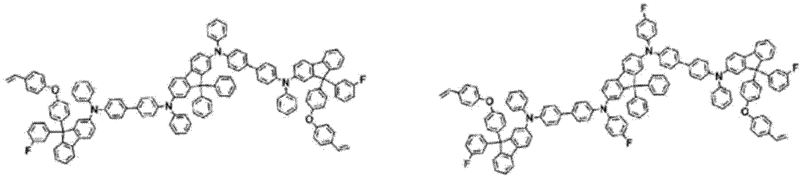

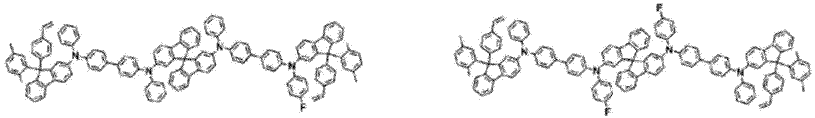

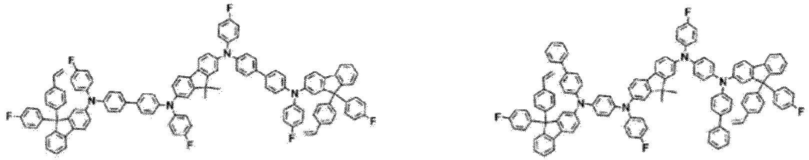

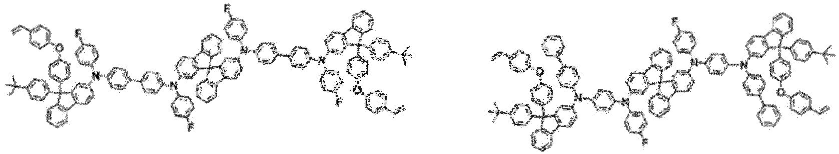

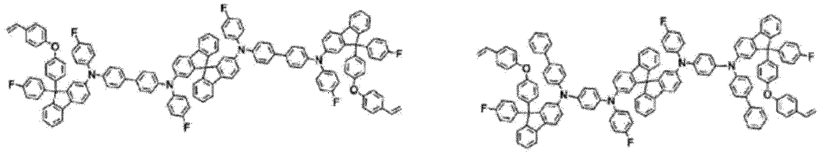

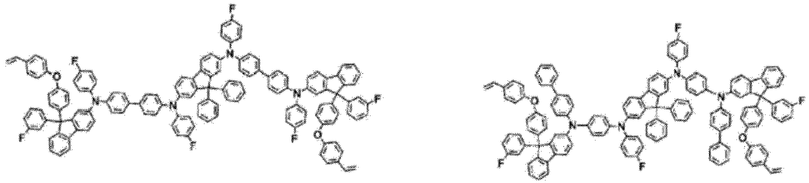

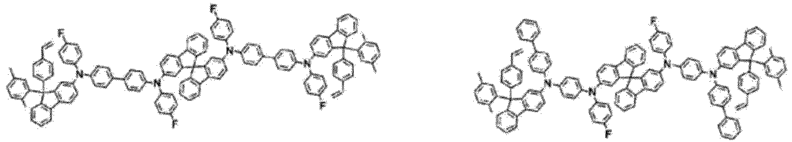

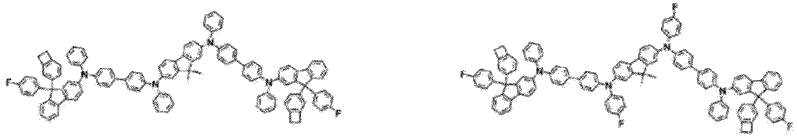

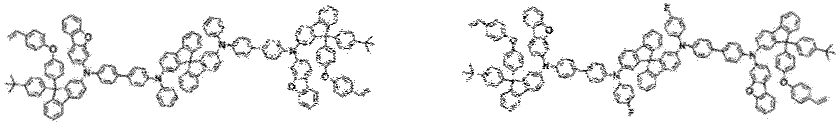

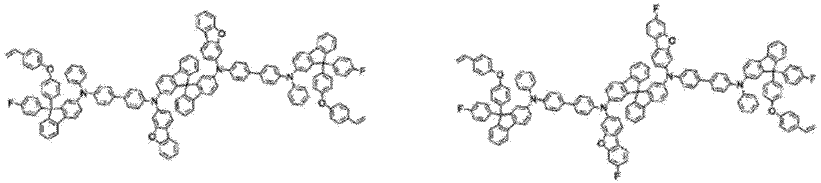

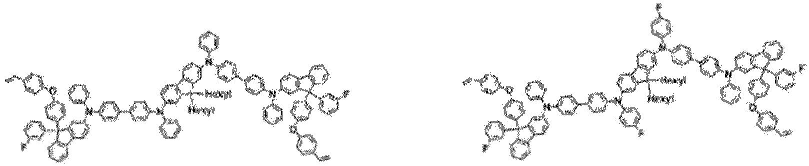

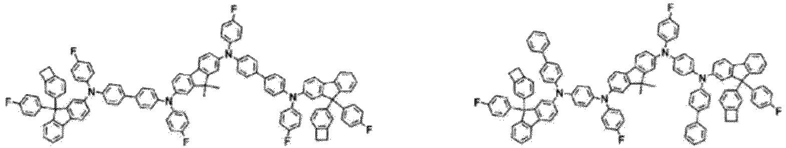

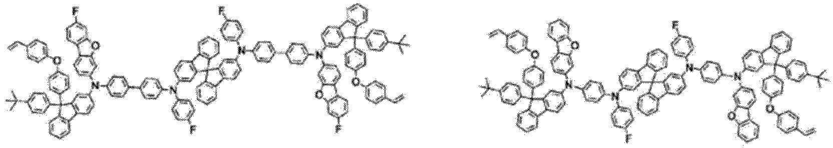

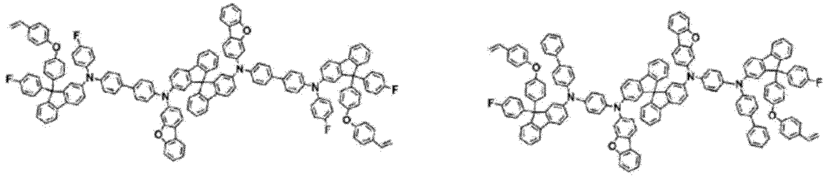

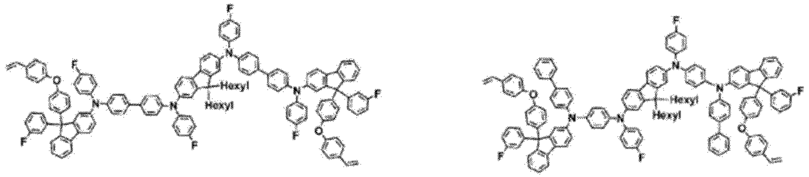

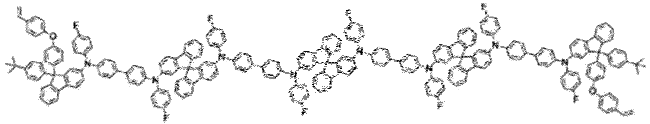

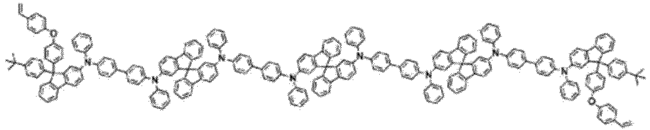

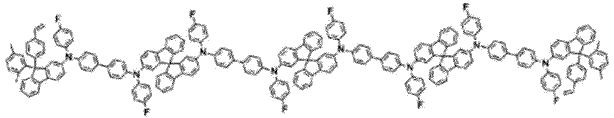

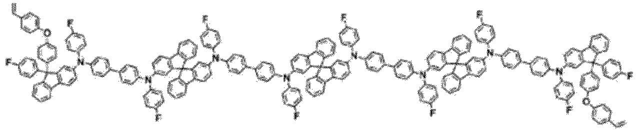

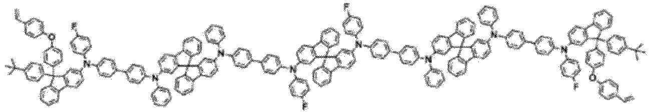

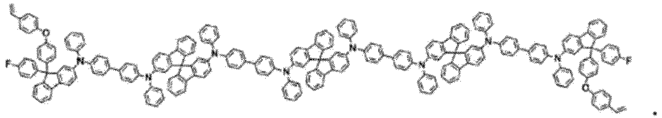

10. Verbindung nach Anspruch 1, wobei die chemische Formel 1 eine der folgenden Strukturen ist:

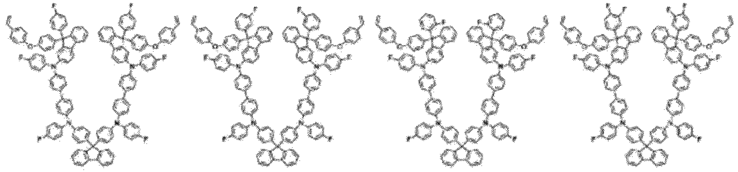

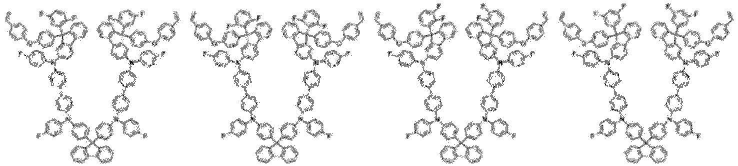

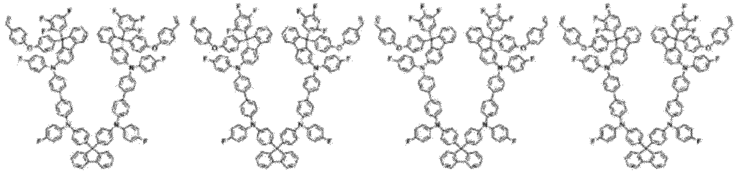

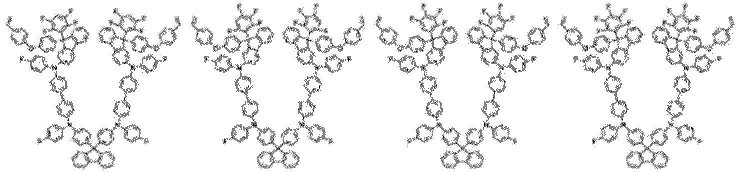

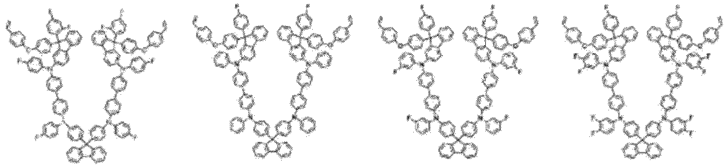

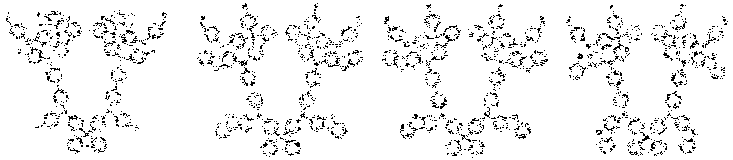

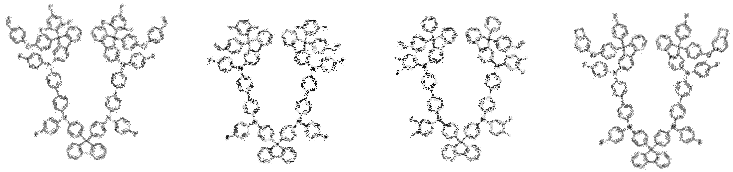

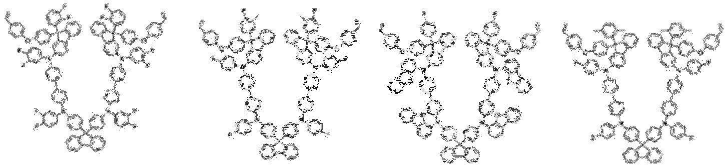

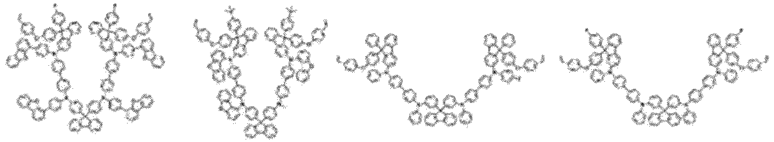

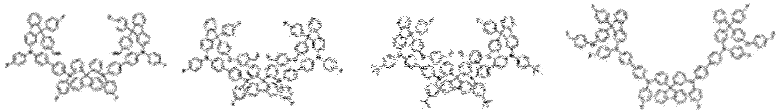

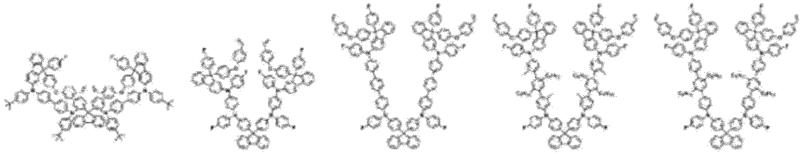

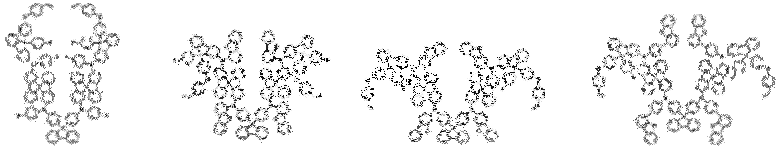

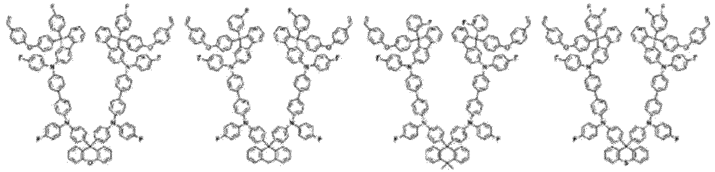

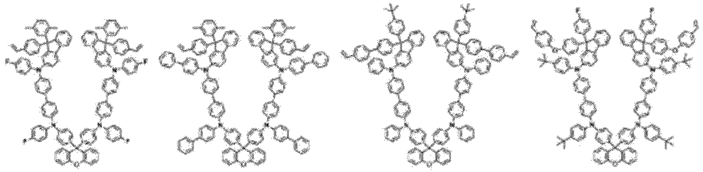

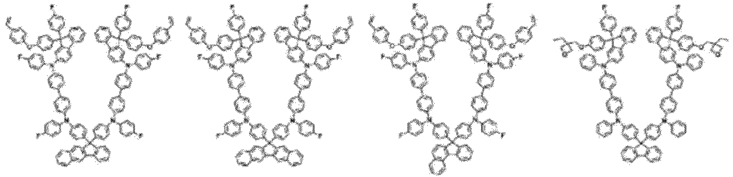

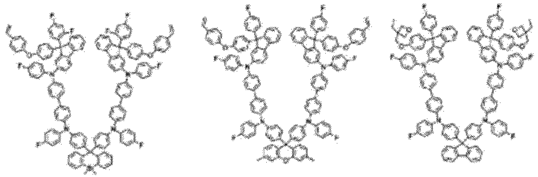

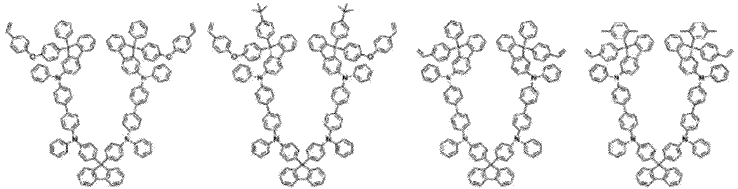

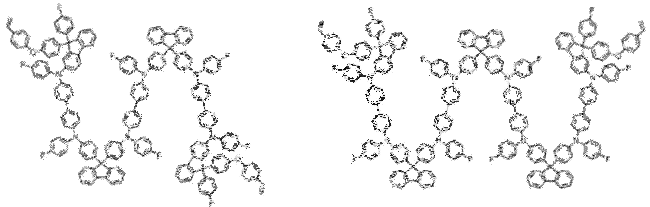

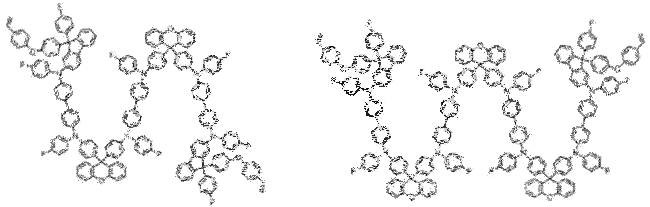

11. Verbindung nach Anspruch 1, wobei die chemische Formel 1 eine der folgenden Strukturen ist:

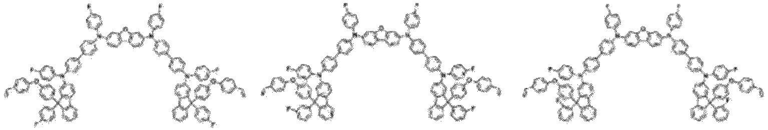

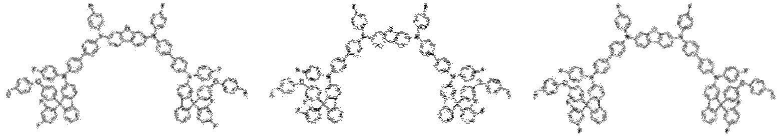

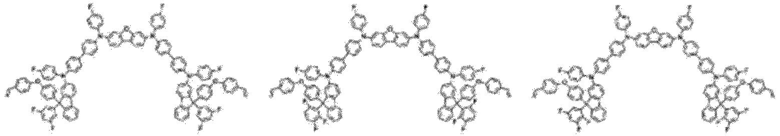

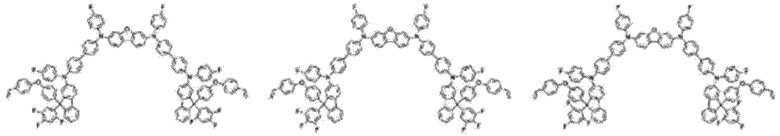

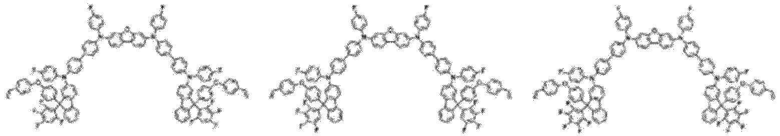

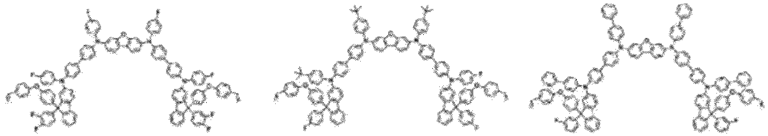

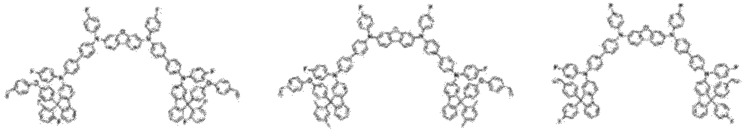

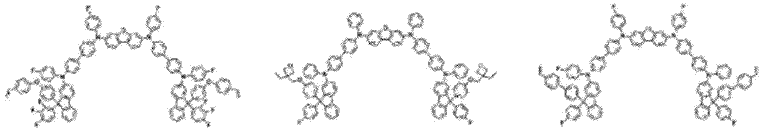

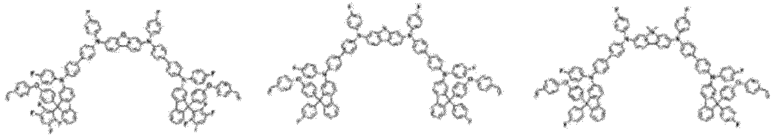

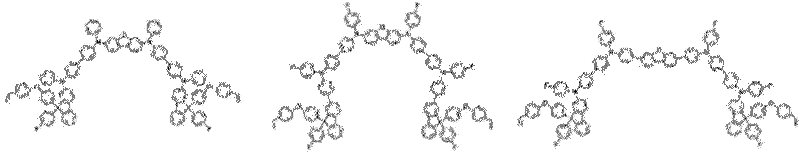

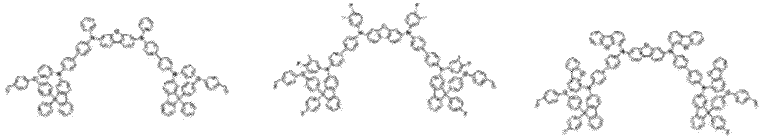

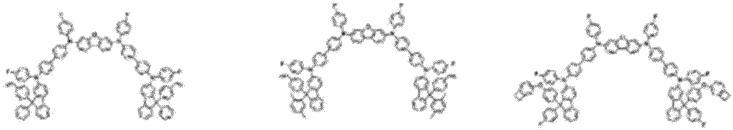

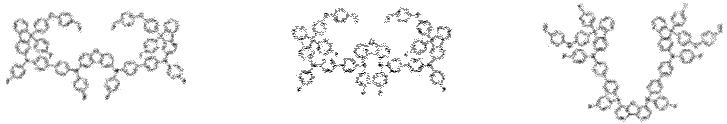

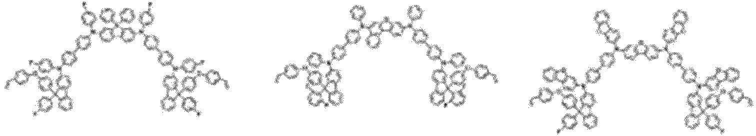

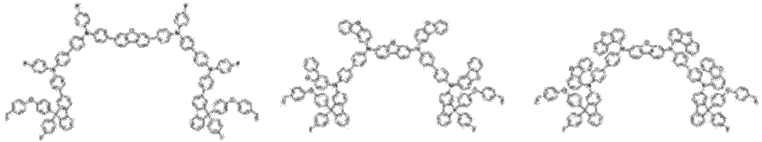

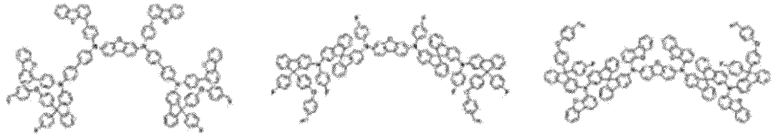

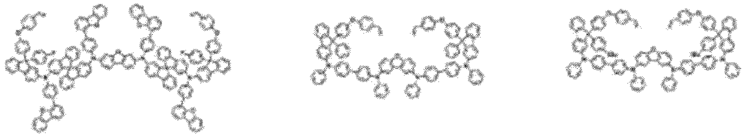

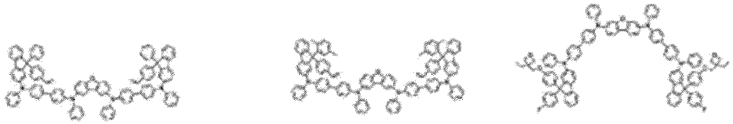

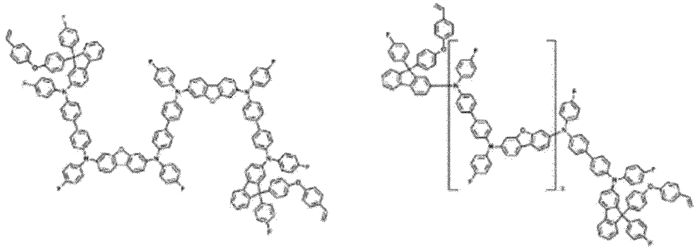

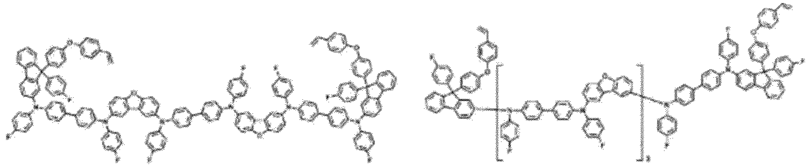

12. Beschichtungszusammensetzung, umfassend die Verbindung nach einem der Ansprüche 1 bis 11.

13. Organische lichtemittierende Vorrichtung, umfassend: eine erste Elektrode;
eine zweite Elektrode; und
eine Schicht aus organischem Material mit einer oder mehreren Schichten, die eine zwischen der ersten Elektrode und der zweiten Elektrode angeordnete lichtemittierende Schicht einschließt,
wobei eine oder mehrere Schichten der organischen Materialschicht die Beschichtungszusammensetzung nach Anspruch 12 oder ein gehärtetes Produkt davon umfassen.

14. Verfahren zur Herstellung einer organischen lichtemittierenden Vorrichtung, wobei das Verfahren Folgendes umfasst: Herstellen eines Substrats;
Bilden einer ersten Elektrode auf dem Substrat:
Bilden einer Schicht aus organischem Material mit einer oder mehreren Schichten auf der ersten Elektrode; und
Bilden einer zweiten Elektrode auf der Schicht aus organischem Material,
wobei das Bilden der organischen Materialschicht die Bildung einer organischen Materialschicht mit einer oder mehreren Schichten unter Verwendung der Beschichtungszusammensetzung nach Anspruch 12 umfasst.

15. Verfahren nach Anspruch 14, wobei das Bilden der organischen Materialschicht mit einer oder mehreren Schichten durch Verwendung der Beschichtungszusammensetzung Folgendes umfasst:
Beschichten der Beschichtungszusammensetzung auf die erste Elektrode; und
Wärmebehandlung oder Lichtbehandlung der beschichteten Beschichtungszusammensetzung.

## Revendications

1. Composé de la Formule chimique 1 suivante : 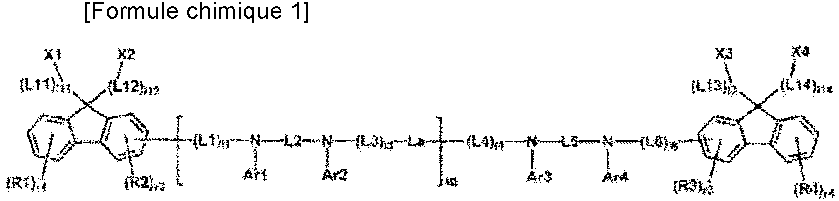 dans lequel, dans la Formule chimique 1,
La est -Lx-A-Ly- ; un groupe dihydroanthracène divalent substitué ou non substitué ; un groupe hétérocyclique divalent substitué ou non substitué ; un groupe fluorényle divalent substitué ou non substitué ; ou un groupe spirobifluorène divalent substitué ou non substitué,
Lx et Ly sont identiques ou différents l'un de l'autre, et sont chacun indépendamment un groupe arylène substitué ou non substitué,
A est un groupe dihydroanthracène divalent substitué ou non substitué ; un groupe hétérocyclique divalent substitué ou non substitué ; un groupe fluorényle divalent substitué ou non substitué ; ou un groupe spirobifluorène divalent substitué ou non substitué,
L1 à L6 sont identiques ou différents les uns des autres, et sont chacun indépendamment un groupe arylène substitué ou non substitué ; ou un groupe hétérocyclique divalent substitué ou non substitué,
L11 à L14 sont identiques ou différents les uns des autres, et sont chacun indépendamment une liaison directe ; -O- ; un groupe alkylène substitué ou non substitué ; un groupe arylène substitué ou non substitué ; ou un groupe hétérocyclique divalent substitué ou non substitué,
Ar1 à Ar4 sont identiques ou différents les uns des autres, et sont chacun indépendamment un groupe alkyle substitué ou non substitué ; un groupe aryle substitué ou non substitué ; ou un groupe hétérocyclique substitué ou non substitué,
X1 à X4 sont identiques ou différents les uns des autres, et sont chacun indépendamment un hydrogène ; un deutérium ; un groupe halogène ; un groupe alkyle substitué ou non substitué ; un groupe alcoxy substitué ou non substitué ; un groupe aryle substitué ou non substitué ; un groupe hétérocyclique substitué ou non substitué ; un groupe photodurcissable ou un groupe thermodurcissable, et deux ou plus de X1 à X4 sont un groupe photodurcissable ou un groupe thermodurcissable,
R1 à R4 sont identiques ou différents les uns des autres, et sont chacun indépendamment un hydrogène ; un deutérium ; un groupe halogène ; un groupe alkyle substitué ou non substitué ; un groupe alcoxy substitué ou non substitué ; un groupe aryle substitué ou non substitué ; ou un groupe hétérocyclique substitué ou non substitué,
l1, l3, l4 et l6 sont chacun un entier de 0 à 3, et lorsque l1, l3, l4 et l6 sont chacun 2 ou plus, des structures entre parenthèses sont identiques ou différentes les unes des autres,
l11 à 114 sont chacun un entier de 1 à 3, et lorsque l11 à 114 sont chacun 2 ou plus, des structures entre parenthèses sont identiques ou différentes les unes des autres,
r1 et r4 sont chacun un entier de 1 à 4, r2 et r3 sont chacun un entier de 1 à 3, et lorsque r1 à r4 sont chacun 2 ou plus, des substituants entre parenthèses sont identiques ou différents les uns des autres, et
m est un entier de 1 à 10, et lorsque m est 2 ou plus, des structures entre parenthèses sont identiques ou différentes les unes des autres.

2. Composé selon la revendication 1, dans lequel le groupe photodurcissable ou groupe thermodurcissable est l'une quelconque des structures suivantes : 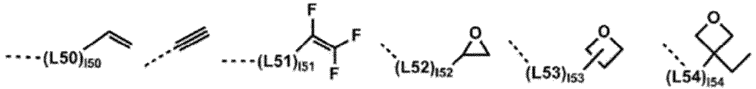 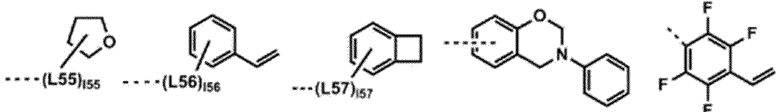 dans les structures,
L50 à L57 sont identiques ou différents les uns des autres, et sont chacun indépendamment une liaison directe ; -O- ; ou un groupe alkylène substitué ou non substitué,
l50 à l57 sont chacun un entier de 1 à 5, et lorsque l50 à l57 sont chacun 2 ou plus, des structures entre parenthèses sont identiques ou différentes les unes des autres, et
------ est un fragment lié à la Formule chimique 1.

3. Composé selon la revendication 1, dans lequel La est l'une quelconque des structures suivantes : 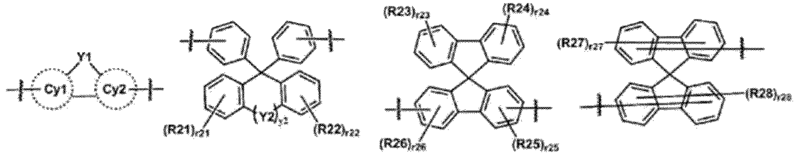 dans les structures,
Cy1 et Cy2 sont identiques ou différents l'un de l'autre, et sont chacun indépendamment un cycle aromatique substitué ou non substitué,
Y1 et Y2 sont identiques ou différents l'un de l'autre, et sont chacun indépendamment O ; S ; CRaRb ; ou SiRcRd,
Ra à Rd sont identiques ou différents les uns des autres, et sont chacun indépendamment un hydrogène ; un deutérium ; un groupe alkyle substitué ou non substitué ; ou un groupe aryle substitué ou non substitué,
R21 à R28 sont identiques ou différents les uns des autres, et sont chacun indépendamment un hydrogène ; un deutérium ; un groupe halogène ; un groupe alkyle substitué ou non substitué ; un groupe alcoxy substitué ou non substitué ; un groupe aryle substitué ou non substitué ; ou un groupe hétérocyclique substitué ou non substitué, ou sont liés à un groupe adjacent pour former un cycle substitué ou non substitué,
y2 est 0 ou 1,
r21 à r24 sont chacun un entier de 1 à 4, r25 et r26 sont chacun un entier de 1 à 3, r27 et r28 sont chacun un entier de 1 à 7, et lorsque r21 à r28 sont 2 ou plus, des substituants entre parenthèses sont identiques ou différents les uns des autres, et 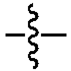
est un fragment lié à la Formule chimique 1.

4. Composé selon la revendication 1, dans lequel La est l'une quelconque des structures suivantes : 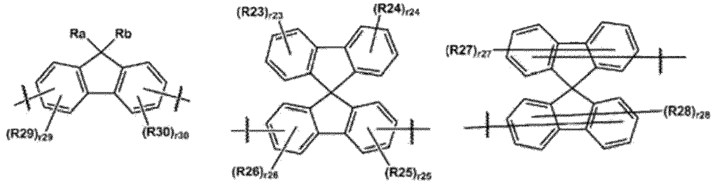 dans les structures,
Ra et Rb sont identiques ou différents l'un de l'autre, et sont chacun indépendamment un hydrogène ; un deutérium ; un groupe alkyle substitué ou non substitué ; ou un groupe aryle substitué ou non substitué,
R23 à R30 sont identiques ou différents les uns des autres, et sont chacun indépendamment un hydrogène ; un deutérium ; un groupe halogène ; un groupe alkyle substitué ou non substitué ; un groupe alcoxy substitué ou non substitué ; un groupe aryle substitué ou non substitué ; ou un groupe hétérocyclique substitué ou non substitué,
r25, r26, r29 et r30 sont chacun un entier de 1 à 3, r23 et r24 sont chacun un entier de 1 à 4, r27 et r28 sont chacun un entier de 1 à 7, et lorsque r23 à r30 sont 2 ou plus, des substituants entre parenthèses sont identiques ou différents les uns des autres, et 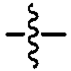
est un fragment lié à la Formule chimique 1.

5. Composé selon la revendication 1, dans lequel La est la structure suivante : 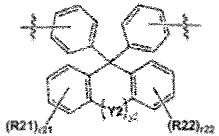 dans la structure,
Y2 est O ; S ; CRaRb ; ou SiRcRd,
Ra à Rd sont identiques ou différents les uns des autres, et sont chacun indépendamment un hydrogène ; un deutérium ; un groupe alkyle substitué ou non substitué ; ou un groupe aryle substitué ou non substitué,
R21 et R22 sont identiques ou différents l'un de l'autre, et sont chacun indépendamment un hydrogène ; un deutérium ; un groupe halogène ; un groupe alkyle substitué ou non substitué ; un groupe alcoxy substitué ou non substitué ; un groupe aryle substitué ou non substitué ; ou un groupe hétérocyclique substitué ou non substitué, ou sont liés à un groupe adjacent pour former un cycle substitué ou non substitué,
y2 est 0 ou 1,
r21 et r22 sont chacun un entier de 1 à 4, et lorsque r21 et r22 sont chacun 2 ou plus, des substituants entre parenthèses sont identiques ou différents les uns des autres, et 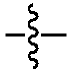
est un fragment lié à la Formule chimique 1.

6. Composé selon la revendication 1, dans lequel La est la structure suivante : 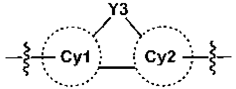 dans la structure,
Cy1 et Cy2 sont identiques ou différents l'un de l'autre, et sont chacun indépendamment un cycle aromatique substitué ou non substitué,
Y3 est O ; S ; ou SiRcRd,
Rc et Rd sont identiques ou différents l'un de l'autre, et sont chacun indépendamment un hydrogène ; un deutérium ; un groupe alkyle substitué ou non substitué ; ou un groupe aryle substitué ou non substitué, et 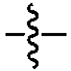
est un fragment lié à la Formule chimique 1.

7. Composé selon la revendication 1, dans lequel La est l'une quelconque des structures suivantes, et les structures suivantes sont non substituées ou substituées par un substituant supplémentaire : 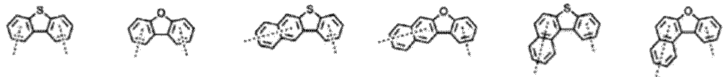 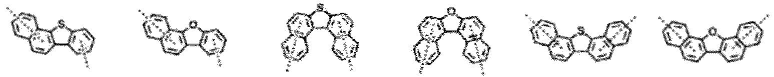 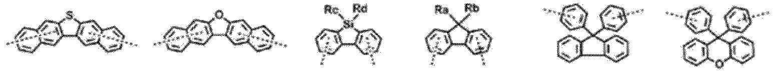 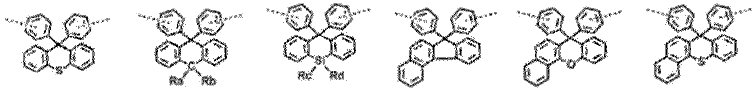 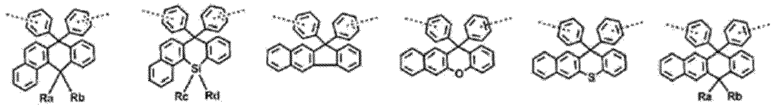 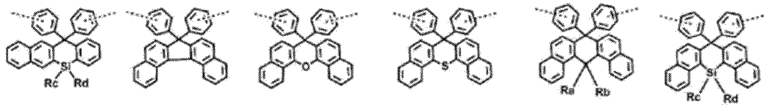 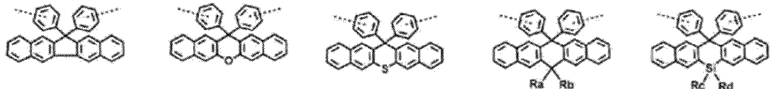 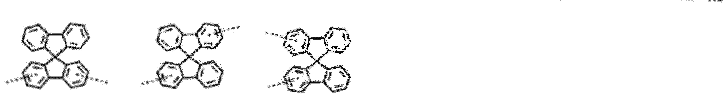 dans les structures,
Ra à Rd sont identiques ou différents les uns des autres, et sont chacun indépendamment un hydrogène ; un deutérium ; un groupe alkyle substitué ou non substitué ; ou un groupe aryle substitué ou non substitué, et
----- est un fragment lié à la Formule chimique 1.

8. Composé selon la revendication 1, dans lequel la Formule chimique 1 est la Formule chimique 1-1 suivante : 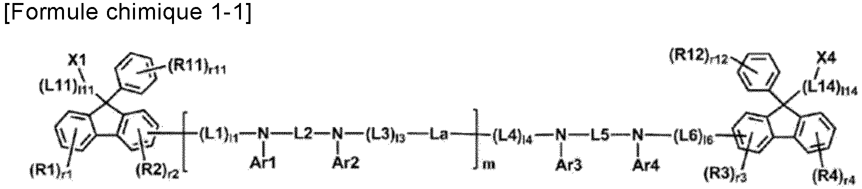 dans la Formule chimique 1-1,
La, L1 à L6, L11, L14, Ar1 à Ar4, R1 à R4, 11, 13, 14, 16, 111, 114, r1 à r4 et m sont identiques à ceux définis dans la Formule chimique 1,
X1 et X4 sont identiques ou différents l'un de l'autre, et sont chacun indépendamment un groupe photodurcissable ou un groupe thermodurcissable,
R11 et R12 sont identiques ou différents l'un de l'autre, et sont chacun indépendamment un hydrogène ; un deutérium ; un groupe halogène ; un groupe alkyle substitué ou non substitué ; un groupe alcoxy substitué ou non substitué ; un groupe aryle substitué ou non substitué ; ou un groupe hétérocyclique substitué ou non substitué, et
r11 et r12 sont chacun un entier de 1 à 5, et lorsque r11 et r12 sont chacun 2 ou plus, des substituants entre parenthèses sont identiques ou différents les uns des autres.

9. Composé selon la revendication 1, dans lequel la Formule chimique 1 est l'une quelconque des structures suivantes :

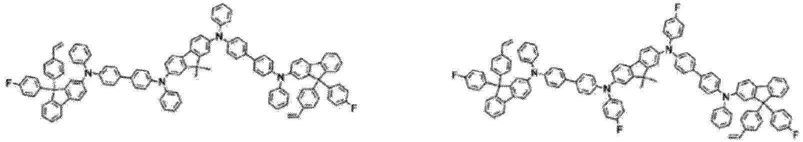

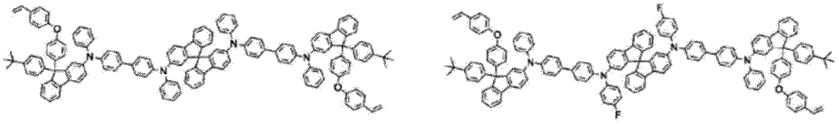

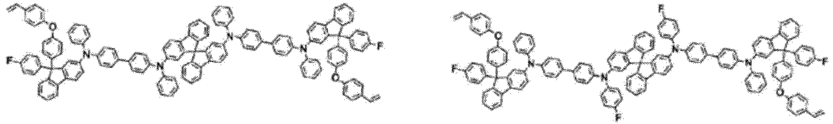

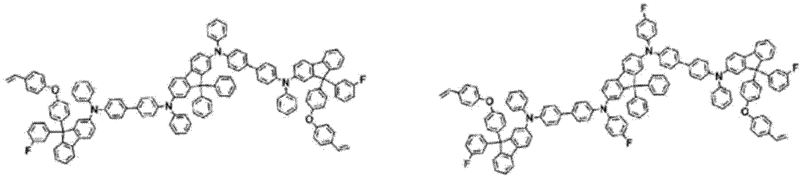

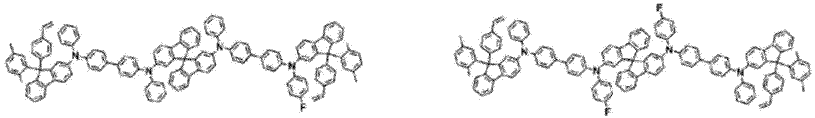

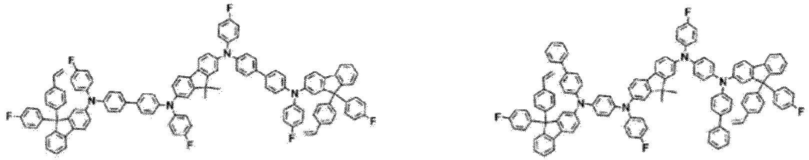

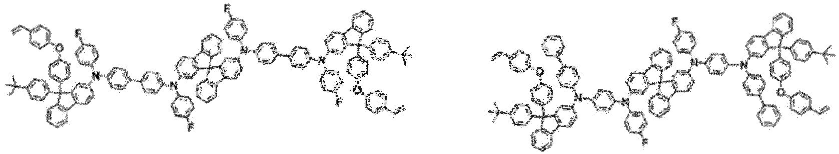

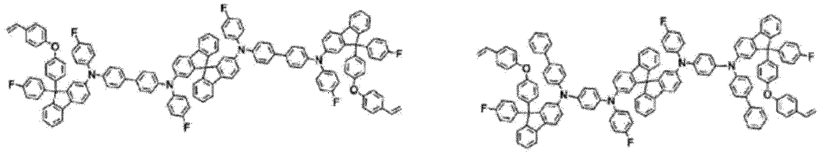

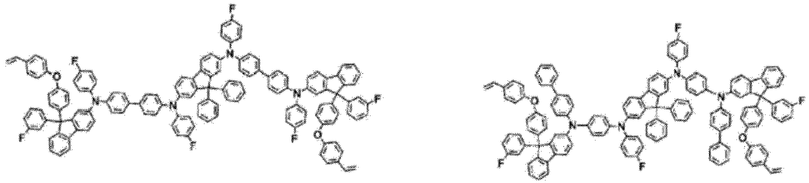

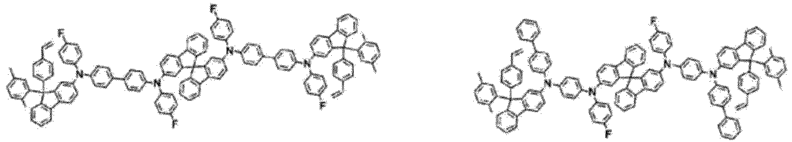

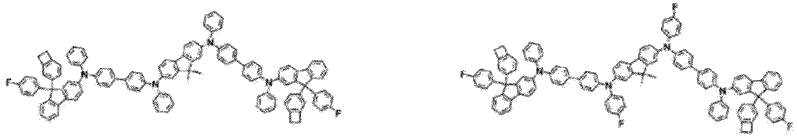

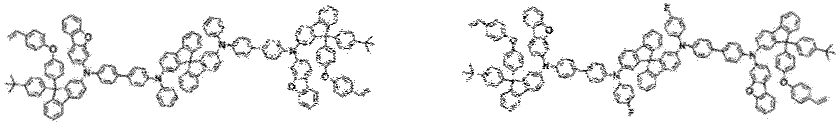

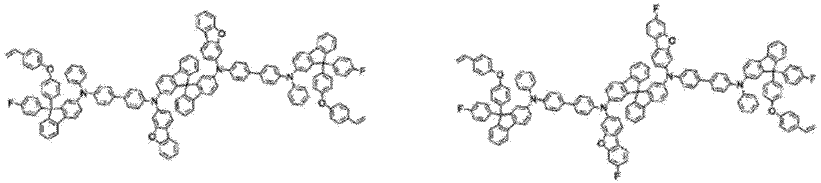

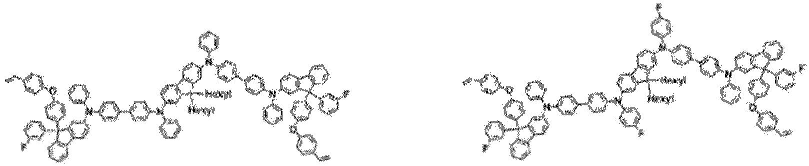

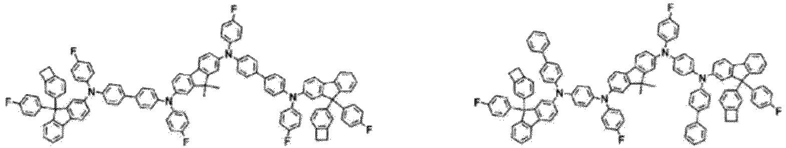

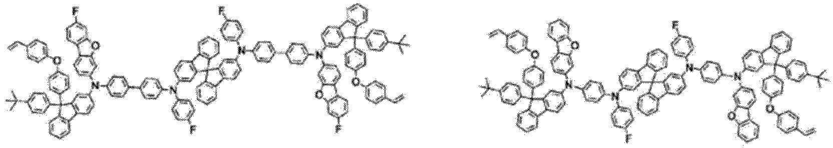

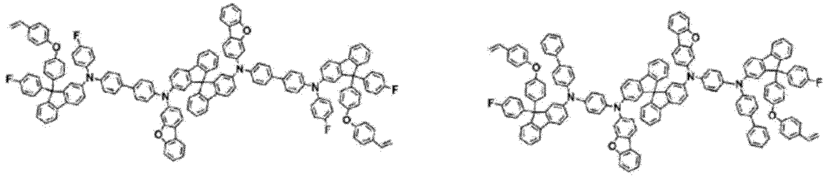

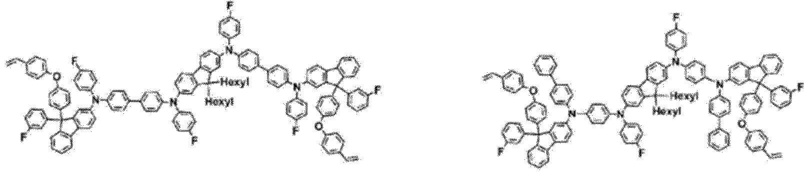

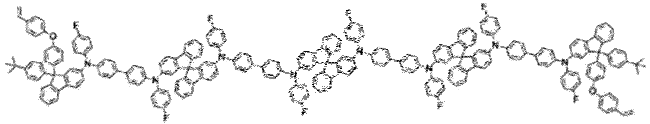

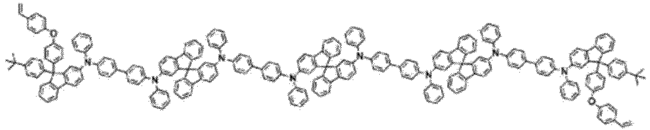

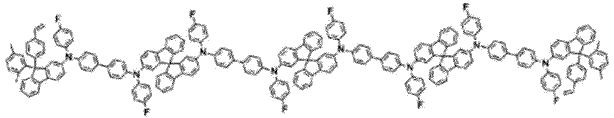

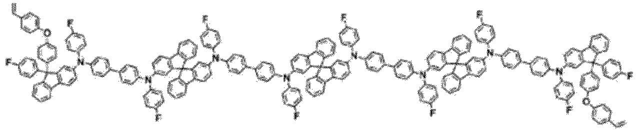

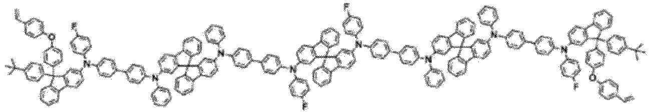

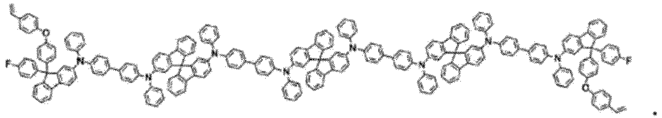

10. Composé selon la revendication 1, dans lequel la Formule chimique 1 est l'une quelconque des structures suivantes :

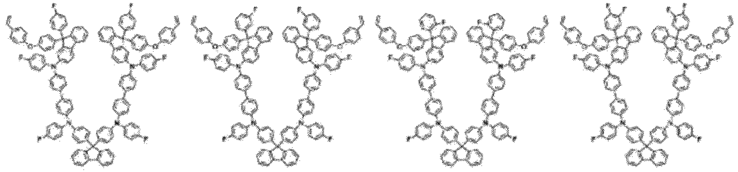

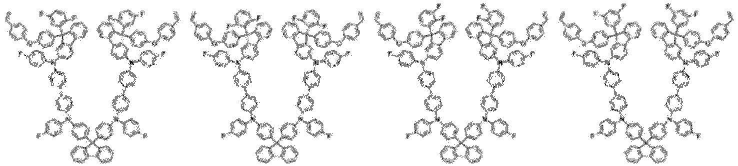

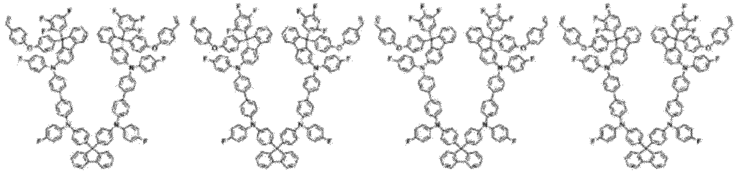

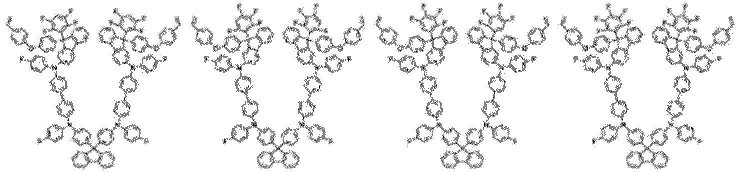

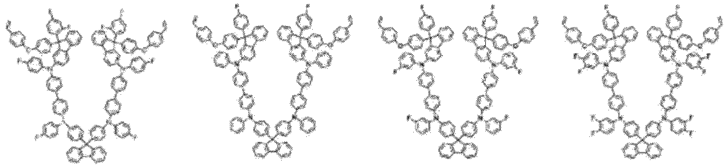

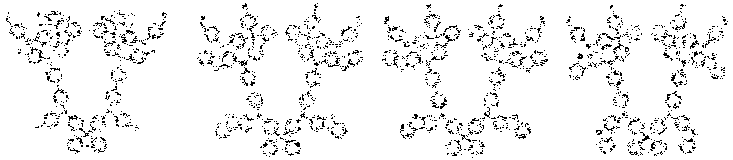

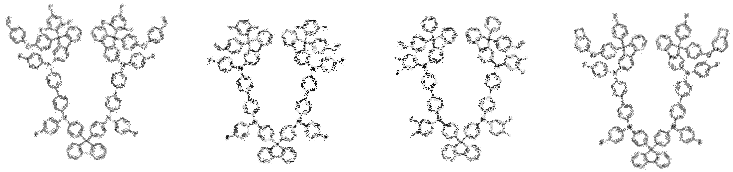

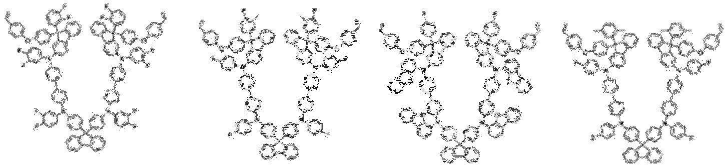

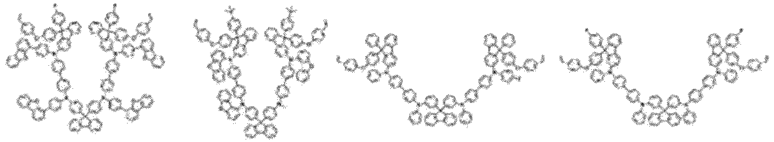

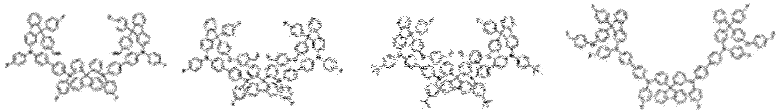

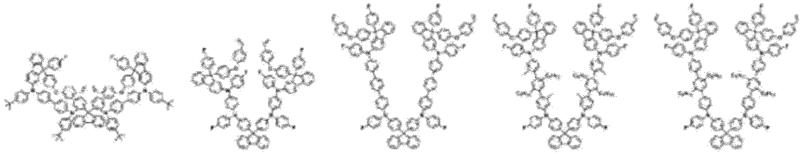

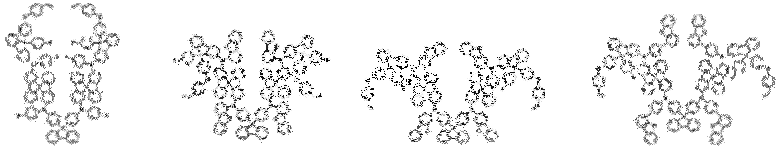

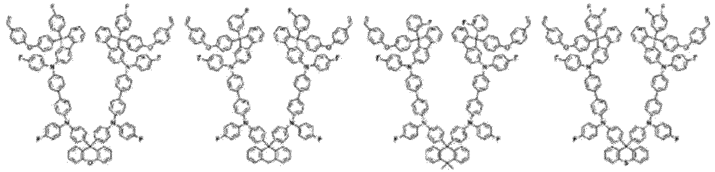

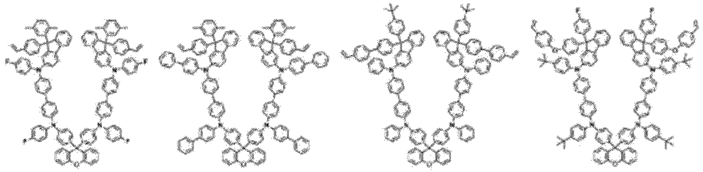

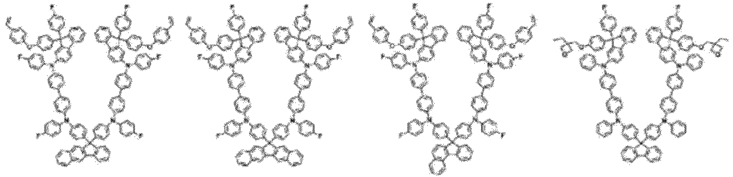

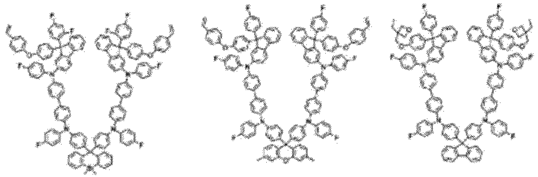

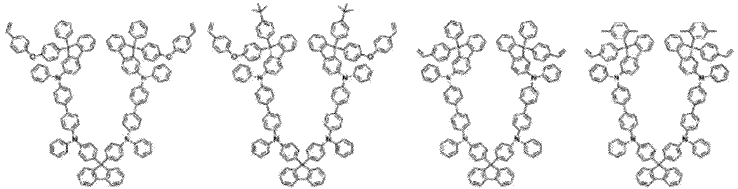

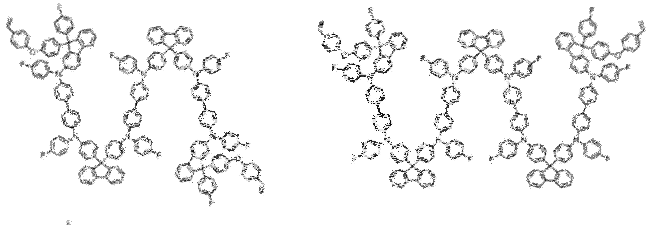

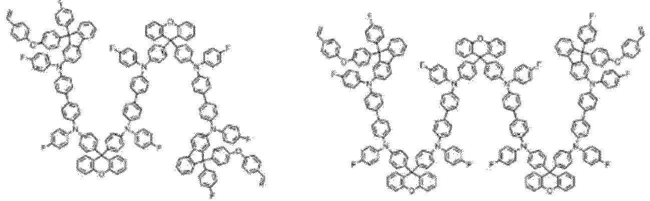

11. Composé selon la revendication 1, dans lequel la Formule chimique 1 est l'une quelconque des structures suivantes :

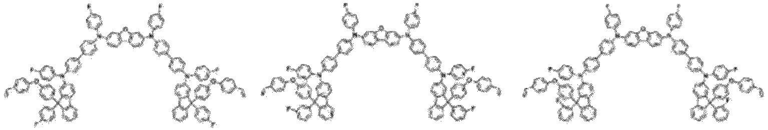

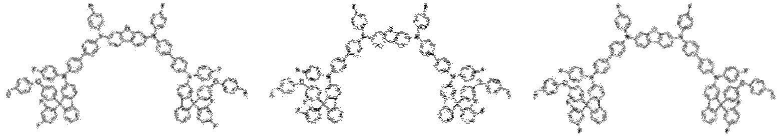

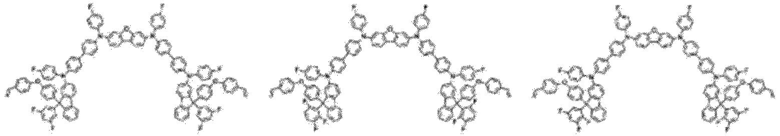

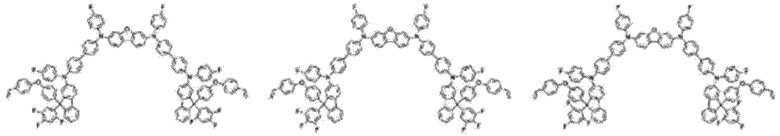

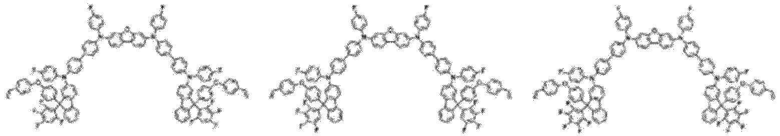

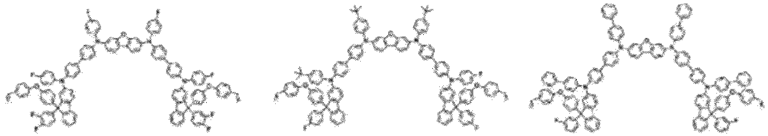

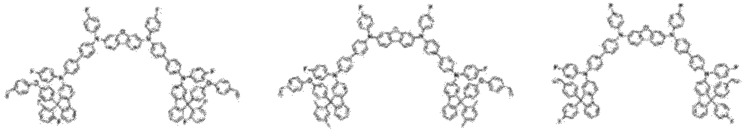

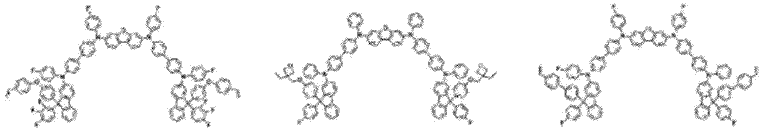

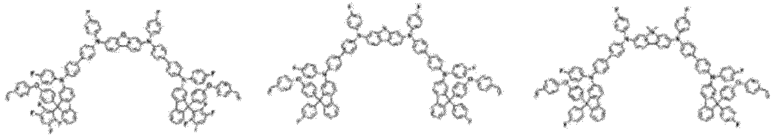

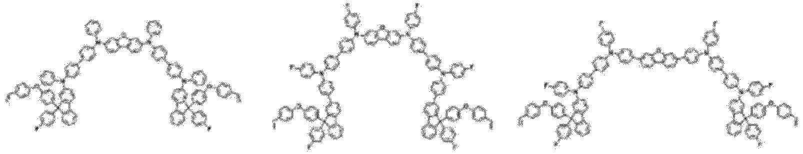

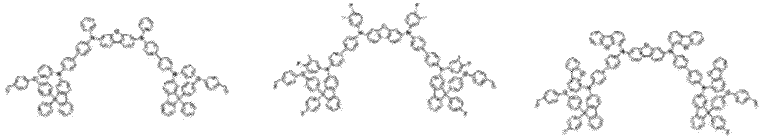

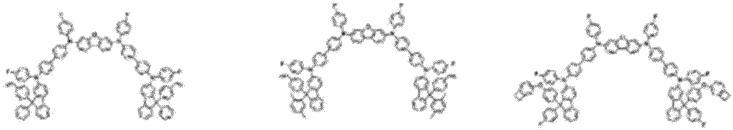

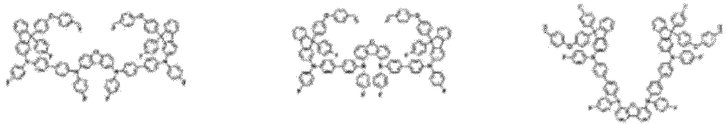

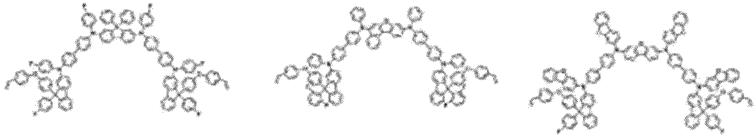

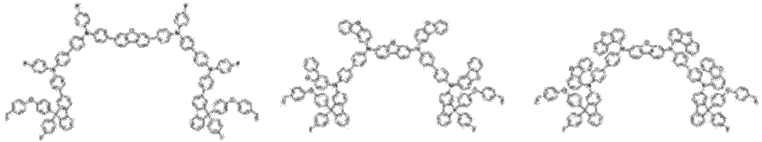

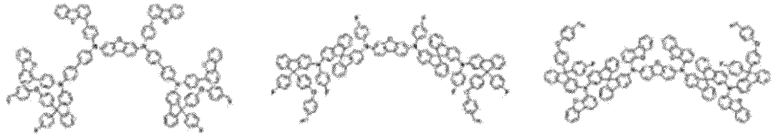

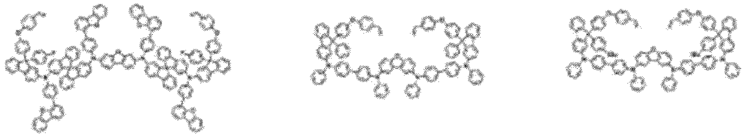

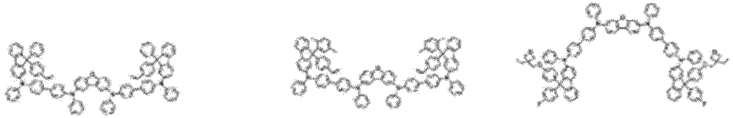

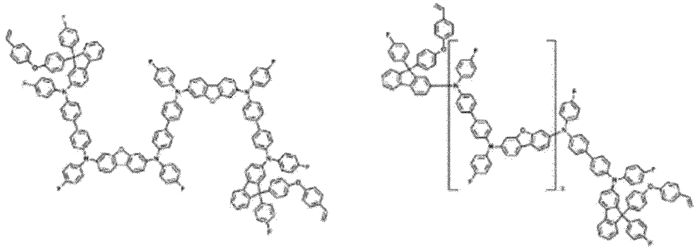

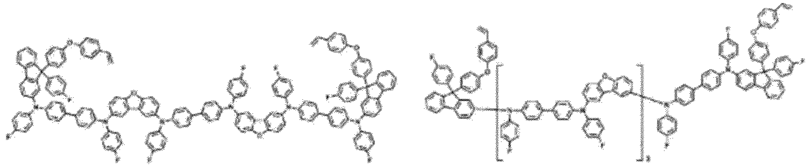

12. Composition de revêtement comprenant le composé selon l'une quelconque des revendications 1 à 11.

13. Dispositif électroluminescent organique comprenant : une première électrode ;
une seconde électrode ; et
une couche de matériau organique présentant une ou plusieurs couches, qui inclut une couche électroluminescente fournie entre la première électrode et la seconde électrode,
dans lequel une ou plusieurs couches de la couche de matériau organique comprennent la composition de revêtement selon la revendication 12 ou un produit durci de celle-ci.

14. Procédé de fabrication d'un dispositif électroluminescent organique, le procédé comprenant : la préparation d'un substrat ;
la formation d'une première électrode sur le substrat :
la formation d'une couche de matériau organique présentant une ou plusieurs couches sur la première électrode ; et
la formation d'une seconde électrode sur la couche de matériau organique,
dans lequel la formation de la couche de matériau organique comprend la formation d'une couche de matériau organique présentant une ou plusieurs couches en utilisant la composition de revêtement selon la revendication 12.

15. Procédé selon la revendication 14, dans lequel la formation de la couche de matériau organique présentant une ou plusieurs couches en utilisant la composition de revêtement comprend :
le revêtement de la composition de revêtement sur la première électrode ; et
le traitement thermique ou traitement lumineux de la composition de revêtement revêtue.
